(19) Europäisches Patentamt
European Patent Office
Office européen des brevets

(11) EP 4 538 291 A1

(12) EUROPEAN PATENT APPLICATION
published in accordance with Art. 153(4) EPC

(43) Date of publication:
**16.04.2025 Bulletin 2025/16**

(21) Application number: **23839026.4**

(22) Date of filing: **13.07.2023**

(51) International Patent Classification (IPC):
***C07K 16/28*** *(2006.01)* ***C12N 15/13*** *(2006.01)*
***A61K 47/68*** *(2017.01)* ***A61K 39/395*** *(2006.01)*
***A61P 35/00*** *(2006.01)*

(52) Cooperative Patent Classification (CPC):
**A61K 39/395; A61K 47/68; A61P 35/00;
C07K 16/00; C07K 16/28; C07K 16/30**

(86) International application number:
**PCT/CN2023/107275**

(87) International publication number:
**WO 2024/012536 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.07.2022 CN 202210833355**

(71) Applicant: **Bio-Thera Solutions, Ltd.**
**Guangzhou, Guangdong 510530 (CN)**

(72) Inventors:
- **MEI, Xingxing**
  **Guangzhou, Guangdong 510530 (CN)**
- **FENG, Cuiying**
  **Guangzhou, Guangdong 510530 (CN)**
- **TANG, Weijia**
  **Guangzhou, Guangdong 510530 (CN)**
- **QI, Yao**
  **Guangzhou, Guangdong 510530 (CN)**
- **ZHANG, Hui**
  **Guangzhou, Guangdong 510530 (CN)**
- **CHEN, Junyou**
  **Guangzhou, Guangdong 510530 (CN)**
- **WANG, Zhiwei**
  **Guangzhou, Guangdong 510530 (CN)**
- **HUANG, Xianming**
  **Guangzhou, Guangdong 510530 (CN)**
- **YU, Jin-Chen**
  **Guangzhou, Guangdong 510530 (CN)**
- **LI, Shengfeng**
  **Guangzhou, Guangdong 510530 (CN)**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

# (54) ANTI-NECTIN-4 ANTIBODY, AND ANTIBODY-DRUG CONJUGATE AND USE THEREOF

(57) Disclosed herein are an anti-Nectin-4 antibody, an antibody-drug conjugate thereof, and use thereof. The anti-Nectin-4 antibody and the antibody-drug conjugate thereof can be used for treating a disease.

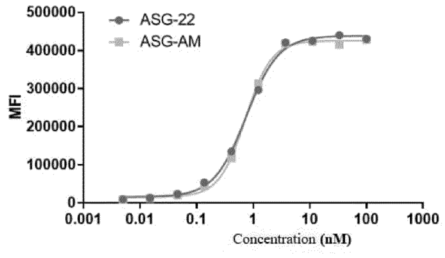


| | ASG-22 | ASG-AM |
|---|---|---|
| EC50 (nM) | 0.7522 | 0.7260 |

FIG. 1

EP 4 538 291 A1

## Description

## TECHNICAL FIELD

The present disclosure relates to the field of biopharmaceuticals, and particularly, to an anti-Nectin-4 antibody, an antibody-drug conjugate thereof, and use thereof.

## BACKGROUND

Nectin-4 is one of the cell adhesion molecules (CAMs) in the immunoglobulin superfamily (IgSF). It is a calcium-independent cell adhesion molecule, and is also known as poliovirus receptor-like protein 4 (PVRL4) or poliovirus receptor-related 4 (PRR4). Nectin-4 is a single-pass transmembrane protein comprising an extracellular region, a transmembrane region, and an intracellular region, where the extracellular region contains three highly glycosylated domains, namely two C2 domains proximal to the cell membrane and one V domain distal to the cell membrane. The Nectin-4 molecule binds to H protein through the V domain at the N-terminal of the Nectin-4 molecule, thereby achieving the infection of a virus to cells. Nectin-4 is involved in the establishment and maintenance of adhering junctions by interacting with cadherin. Nectin-4 mediates $Ca^{2+}$-independent adhesion, and promotes anchorage-independent growth by driving intercellular adhesion and activating matrix-independent integrin β4/SHP-2/c-Src.

Nectin-4 is expressed during fetal development, but unlike the widespread expression of other Nectins in adult tissues, its expression in adult tissues is significantly reduced. Several institutions have confirmed that Nectin-4 is overexpressed in many tumors, including breast cancer, bladder cancer, and the like. Nectin-4 has low or moderate expression in the stratum corneum of skin, skin appendages (sweat glands and hair follicles), transitional epithelium of bladder, salivary glands, esophagus, mammary glands, and stomach, and low expression in the larynx, pituitary, placenta, testis, ureter, and uterus among normal adult tissues. The target has good specificity and can mediate the endocytosis of antibodies, thus becoming a potential therapeutic target for ADC drugs.

## SUMMARY

The present application provides an anti-Nectin-4 antibody and an antibody-drug conjugate thereof.

One or more embodiments of the present application provide an antibody or an antigen-binding unit, wherein the antibody or the antigen-binding unit specifically binds to Nectin-4 and comprises one or more of (a)-(f):

(a) a VH CDR1 comprising the amino acid sequence set forth in SEQ ID NO: 2;

(b) a VH CDR2 comprising the amino acid sequence set forth in SEQ ID NO: 3;

(c) a VH CDR3 comprising the amino acid sequence set forth in SEQ ID NO: 4;

(d) a VL CDR1 comprising the amino acid sequence set forth in SEQ ID NO: 5;

(e) a VL CDR2 comprising the amino acid sequence set forth in SEQ ID NO: 6; and

(f) a VL CDR3 comprising the amino acid sequence set forth in SEQ ID NO: 7.

In one or more embodiments, the antibody or the antigen-binding unit comprises a VL CDR3 set forth in SEQ ID NO: 7, and optionally, one or more of a VH CDR1 set forth in SEQ ID NO: 2, a VH CDR2 set forth in SEQ ID NO: 3, a VH CDR3 set forth in SEQ ID NO: 4, a VL CDR1 set forth in SEQ ID NO: 5, and a VL CDR2 set forth in SEQ ID NO: 6.

In one or more embodiments, the antibody or the antigen-binding unit comprises a VL CDR1 set forth in SEQ ID NO: 5, a VL CDR2 set forth in SEQ ID NO: 6, and a VL CDR3 set forth in SEQ ID NO: 7.

In one or more embodiments, the antibody or the antigen-binding unit comprises a VH CDR1 set forth in SEQ ID NO: 2, a VH CDR2 set forth in SEQ ID NO: 3, a VH CDR3 set forth in SEQ ID NO: 4, a VL CDR1 set forth in SEQ ID NO: 5, a VL CDR2 set forth in SEQ ID NO: 6, and a VL CDR3 set forth in SEQ ID NO: 7.

In one or more embodiments, the antibody or the antigen-binding unit comprises a heavy chain variable region and/or a light chain variable region. In one or more embodiments, the heavy chain variable region comprises the amino acid sequence set forth in SEQ ID NO: 8, an amino acid sequence having at least 80%, at least 90%, at least 95%, at least 96%, at least 97%, at least 98%, or at least 99% sequence identity to the amino acid sequence set forth in SEQ ID NO: 8, or an amino acid sequence having one or more conservative amino acid substitutions compared with the amino acid sequence set forth in SEQ ID NO: 8. In one or more embodiments, the light chain variable region comprises the amino acid

sequence set forth in SEQ ID NO: 10, an amino acid sequence having at least 80%, at least 90%, at least 95%, at least 96%, at least 97%, at least 98%, or at least 99% sequence identity to the amino acid sequence set forth in SEQ ID NO: 10, or an amino acid sequence having one or more conservative amino acid substitutions compared with the amino acid sequence set forth in SEQ ID NO: 10.

In one or more embodiments, the antibody or the antigen-binding unit further comprises a heavy chain constant region and/or a light chain constant region. In one or more embodiments, the heavy chain constant region is selected from IgG1, IgG2, IgG3, and IgG4 types. In one or more embodiments, the light chain constant region is a λ chain or κ chain constant region.

In one or more embodiments, the heavy chain constant region comprises the amino acid sequence set forth in SEQ ID NO: 9, an amino acid sequence having at least 80%, at least 90%, at least 95%, at least 96%, at least 97%, at least 98%, or at least 99% sequence identity to the amino acid sequence set forth in SEQ ID NO: 9, or an amino acid sequence having one or more conservative amino acid substitutions compared with the amino acid sequence set forth in SEQ ID NO: 9. In one or more embodiments, the light chain constant region comprises the amino acid sequence set forth in SEQ ID NO: 11, an amino acid sequence having at least 80%, at least 90%, at least 95%, at least 96%, at least 97%, at least 98%, or at least 99% sequence identity to the amino acid sequence set forth in SEQ ID NO: 11, or an amino acid sequence having one or more conservative amino acid substitutions compared with the amino acid sequence set forth in SEQ ID NO: 11. In one or more embodiments, the heavy chain constant region comprises the amino acid sequence set forth in SEQ ID NO: 9, and the light chain constant region comprises the amino acid sequence set forth in SEQ ID NO: 11.

In one or more embodiments, the antibody comprises a heavy chain and a light chain. In one or more embodiments, the antibody comprises two heavy chains with identical sequences and two light chains with identical sequences.

In one or more embodiments, the heavy chain of the antibody comprises the amino acid sequence set forth in SEQ ID NO: 12, an amino acid sequence having at least 80%, at least 90%, at least 95%, at least 96%, at least 97%, at least 98%, or at least 99% sequence identity to the amino acid sequence set forth in SEQ ID NO: 12, or an amino acid sequence having one or more conservative amino acid substitutions compared with the amino acid sequence set forth in SEQ ID NO: 12. In one or more embodiments, the light chain of the antibody comprises the amino acid sequence set forth in SEQ ID NO: 14, an amino acid sequence having at least 80%, at least 90%, at least 95%, at least 96%, at least 97%, at least 98%, or at least 99% sequence identity to the amino acid sequence set forth in SEQ ID NO: 14, or an amino acid sequence having one or more conservative amino acid substitutions compared with the amino acid sequence set forth in SEQ ID NO: 14. In one or more embodiments, the heavy chain of the antibody comprises the amino acid sequence set forth in SEQ ID NO: 12, an amino acid sequence having at least 80%, at least 90%, at least 95%, at least 96%, at least 97%, at least 98%, or at least 99% sequence identity to the amino acid sequence set forth in SEQ ID NO: 12, or an amino acid sequence having one or more conservative amino acid substitutions compared with the amino acid sequence set forth in SEQ ID NO: 12; and the light chain of the antibody comprises the amino acid sequence set forth in SEQ ID NO: 14, an amino acid sequence having at least 80%, at least 90%, at least 95%, at least 96%, at least 97%, at least 98%, or at least 99% sequence identity to the amino acid sequence set forth in SEQ ID NO: 14, or an amino acid sequence having one or more conservative amino acid substitutions compared with the amino acid sequence set forth in SEQ ID NO: 14. In one or more embodiments, the heavy chain of the antibody comprises the amino acid sequence set forth in SEQ ID NO: 12, and the light chain of the antibody comprises the amino acid sequence set forth in SEQ ID NO: 14.

One or more embodiments of the present application provide a biomaterial selected from:

(1) a nucleic acid encoding the antibody or the antigen-binding unit or a portion thereof;

(2) a vector, a host cell, or a microorganism comprising (1); and

(3) an expression product, a suspension, or a supernatant of (2) described above.

In one or more embodiments, the nucleic acid sequence encoding the amino acid sequence set forth in SEQ ID NO: 12 is shown in SEQ ID NO: 13. In one or more embodiments, the nucleic acid sequence encoding the amino acid sequence set forth in SEQ ID NO: 14 is shown in SEQ ID NO: 15.

**Table 1 Sequences**

| SEQ ID NO | Sequence |
|---|---|
| 2 | GFTFSSYN |
| 3 | YISSSSSTIY |
| 4 | AYYYGMDV |

(continued)

| SEQ ID NO | Sequence |
|---|---|
| 5 | RASQGISGWLA |
| 6 | AASTLQS |
| 7 | GPGWVFGQG |
| 8 | EVQLVESGGGLVQPGGSLRLSCAASGFTFSSYNMNWVRQAPGKGLEW VSYISSSSSTIYYADSVKGRFTISRDNAKNSLSLQMNSLRDEDTAVYYCA RAYYYGMDVWGQGTLVTVSS |
| 9 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQD WLNGKEYKCKVSNKALPAPIEKTISKAKGQPREPQVYTLPPSRDELTKN QVSLTCLVKGFYPSDIAVEWESNGQPENNYKTTPPVLDSDGSFFLYSKL TVDKSRWQQGNVFSCSVMHEALHNHYTQKSLSLSPGK |
| 10 | DIQMTQSPSSVSASVGDRVTITCRASQGISGWLAWYQQKPGKAPKFLIY AASTLQSGVPSRFSGSGSGTDFTLTISSLQPEDFATYYCQQAGPGWVFG QGTKVEIKR |
| 11 | TVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVT KSFNRGEC |
| 12 | EVQLVESGGGLVQPGGSLRLSCAASGFTFSSYNMNWVRQAPGKGLEW VSYISSSSSTIYYADSVKGRFTISRDNAKNSLSLQMNSLRDEDTAVYYCA RAYYYGMDVWGQGTLVTVSSASTKGPSVFPLAPSSKSTSGGTAALGCL VKDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKG QPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQ KSLSLSPGK |

(continued)

| SEQ ID NO | Sequence |
|---|---|
| 13 | gaggtgcagctggtggagtccggcggcggactggtgcagcctggaggaagcctgaggctgtcctgtgccgcct ccggcttcacattctccagctacaatatgaactgggtgagacaggcccccggcaagggcctggagtgggttagct acatcagctccagctccagcacaatctactacgccgacagcgtgaagggcagattcactatctccagggacaatg ccaagaatagcctgtccctgcagatgaattccctgagagatgaggacacagccgtgtactactgtgccagggcct actactacggcatggacgtgtggggccagggtaccctggttaccgttagcagcgcctctaccaagggcccctctg tgtttcctctggctccctccagcaagtctacctctggtggaacagctgccctgggctgcctggtcaaggattactttc ctgagcctgtcaccgtgtcctggaactctggcgctctgacatctggcgtgcacacctttccagctgtgctccagtcct ccggcctgtactctctgtcctctgtcgtgaccgtgccttctagctctctgggcacccagacctacatctgcaatgtga accacaagccttccaacaccaaggtggacaagaaggtggaacccaagtcctgcgacaagacccacacctgtcct ccatgtcctgctccagaactgctcggcggaccttccgtgttcctgtttcctccaaagcctaaggacaccctgatgatc tctcggacccctgaagtgacctgcgtggtggtggatgtgtctcacgaagatcccgaagtgaagttcaactggtacg tggacggcgtggaagtgcacaacgccaagaccaagcctagagaggaacagtacaactccacctacagagtggt gtccgtgctgaccgtgctgcaccaggattggctgaacggcaaagagtacaagtgcaaggtgtccaacaaggccc tgcctgctcctatcgaaaagaccatctccaaggccaagggccagcctagggaaccccaggtttacaccttgcctc catctcgggacgagctgaccaagaaccaggtgtccctgacctgtctcgtgaagggcttctacccctccgacatcg ccgtggaatgggagtctaatggccagcctgagaacaactacaagacaacccctcctgtgctggactccgacggct cattcttcctgtactccaagctgacagtggacaagtccagatggcagcagggcaacgtgttctcctgctccgtgatg cacgaggccctgcacaatcactacacccagaagtccctgtctctgtcccctggcaaataa |
| 14 | DIQMTQSPSSVSASVGDRVTITCRASQGISGWLAWYQQKPGKAPKFLIY AASTLQSGVPSRFSGSGSGTDFTLTISSLQPEDFATYYCQQAGPGWVFG QGTKVEIKRTVAAPSVFIFPPSDEQLKSGTASVVCLLNNFYPREAKVQW KVDNALQSGNSQESVTEQDSKDSTYSLSSTLTLSKADYEKHKVYACEV THQGLSSPVTKSFNRGEC |
| 15 | gacatccagatgacacagagccccagcagcgtgtccgcctccgtgggagatagagtgacaatcacatgtagagc cagccagggcatcagcggctggctggcttggtaccagcagaagcccggcaaggcccccaagttcctgatctac gccgccagcacactgcagtccggcgtgccttccaggttttccggcagcggctccggcacagactttaccctgaca atcagcagcctgcagcctgaggatttcgccacatactactgccagcaggccggccccggctgggttttcggacag ggaacaaaggtggagatcaagcgtacggtggctgcaccatctgtcttcatcttcccgccatctgatgagcagttga aatctggaactgcctctgttgtgtgcctgctgaataacttctatcccagagaggccaaagtacagtggaaggtggat aacgccctccaatcgggtaactcccaggagagtgtcacagagcaggacagcaaggacagcacctacagcctca gcagcaccctgacgctgagcaaagcagactacgagaaacacaaagtctacgcctgcgaagtcacccatcaggg cctgagctcgcccgtcacaaagagcttcaacaggggagagtgttga |

One or more embodiments of the present application provide a method for preparing the antibody or the antigen-binding unit described in the present application, comprising: culturing the host cells described in the present application to express the antibody or the antigen-binding unit described in the present application, and isolating the antibody or the antigen-binding unit from the host cells.

One or more embodiments of the present application provide an antibody-drug conjugate comprising the antibody or the antigen-binding unit conjugated to a drug (such as the drug described herein) via a linker, or a pharmaceutically acceptable salt or solvate thereof.

In one or more embodiments, the linker is a cleavable linker.

One or more embodiments of the present application provide an antibody-drug conjugate having a structure shown in Formula I-A, Formula I-B or Formula I-C or a stereoisomer thereof, or a pharmaceutically acceptable salt or solvate thereof:

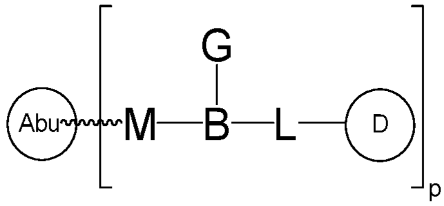

Formula I-A

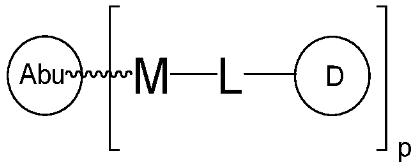

Formula I-B

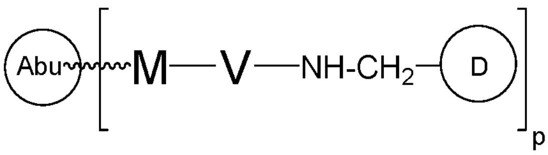

Formula I-C

Abu is the anti-Nectin-4 antibody or the antigen-binding unit of the present application;

D is a drug (such as the drug described herein);

One or more embodiments of the present application provide an antibody-drug conjugate having a structure shown in Formula I-A, or a stereoisomer thereof, or a pharmaceutically acceptable salt or solvate thereof:

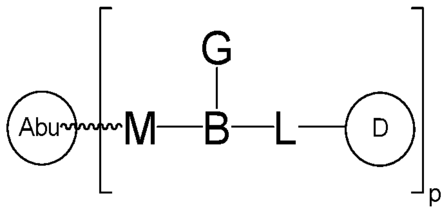

Formula I-A

Wherein

Abu is the anti-Nectin-4 antibody or the antigen-binding unit of the present application;

D is a drug (such as the drug described herein);

M is

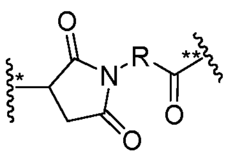

,

wherein * links to Abu, and ** links to B, R is selected from $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$, and $-(CH_2CH_2O)_rC(O)NR^m(CH_2)_r-$; wherein each $R^m$ is independently H, C1-C6 alkyl, C3-C8 carbocyclyl, phenyl, or benzyl; and each r is independently 1, 2, 3, 4, 5, 6, 7, 8, 9, or 10;

B is

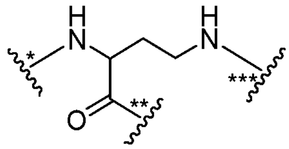

,

or is

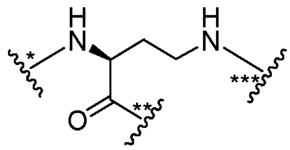

;

wherein * links to M, ** links to L, and *** links to G;

L is $-(AA)_i-(FF)_f-$, wherein AA is an amino acid or polypeptide, and i is 1, 2, 3, 4, 5, 6, 7, 8, 9, 10, 11, 12, 13, 14, 15, 16, 17, 18, 19, or 20; or each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly; each FF is independently

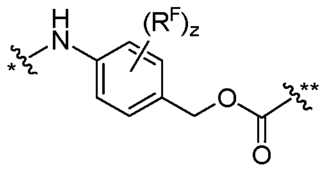

,

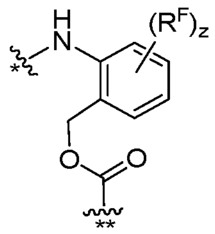

,

or

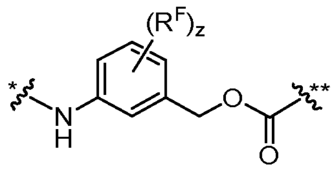

,

wherein each $R^F$ is independently C1-C6 alkyl, C1-C6 alkoxy, $-NO_2$, or halogen, wherein * links to AA, and ** links to D, and z is 0, 1, 2, 3, or 4; f is 1, 2, 3, 4, 5, 6, 7, 8, 9, or 10;

G is

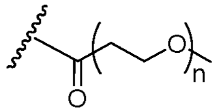,

wherein n is 1-24; such as 1, 2, 3, 4, 5, 6, 7, 8, 9, 10, 11, 12, 13, 14, 15, 16, 17, 18, 19, 20, 21, 22, 23 or 24;

p is 1-10.

In one or more embodiments, R is $-(CH_2)_r-$, or r is 1 or 5.

In one or more embodiments, AA is Val-Cit, and i is 1.

In one or more embodiments, each FF is independently

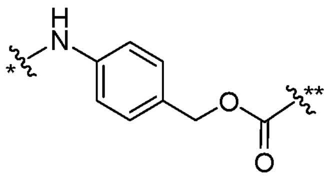,

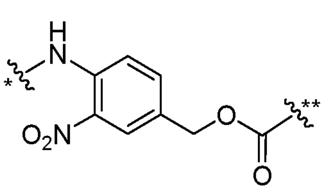, 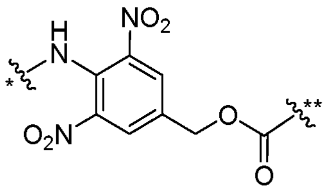, 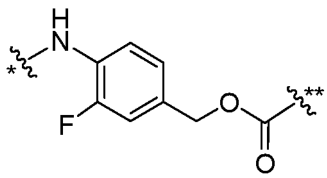,

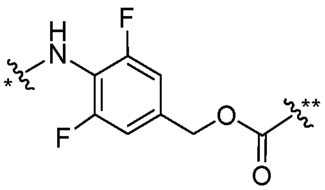, 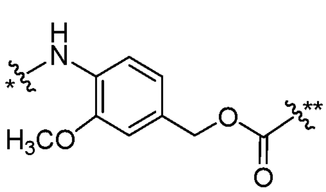, 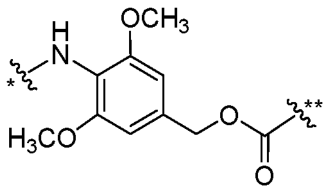,

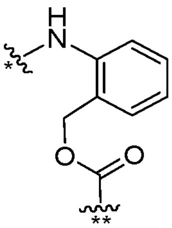, 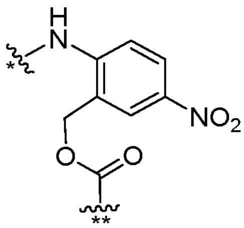, 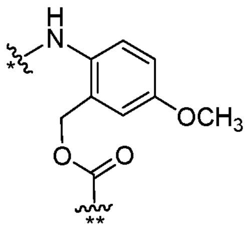, 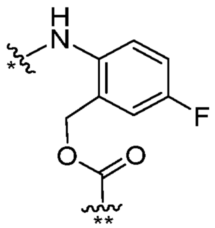, 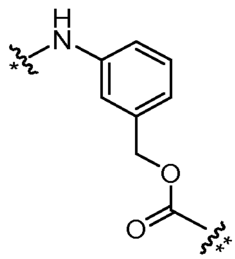,

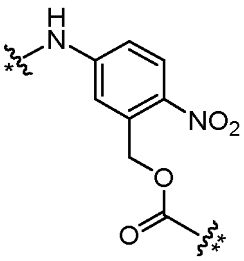, 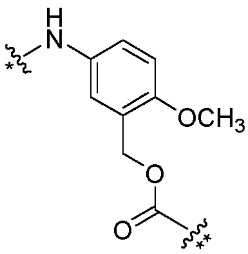, or 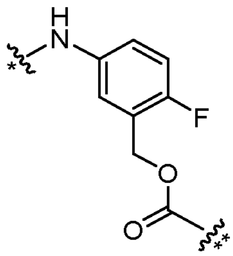;

wherein * links to AA, ** links to D.

In one or more embodiments, FF is

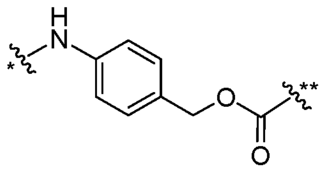

,

and f is 1; wherein * links to AA, and ** links to D.

In one or more embodiments, L is

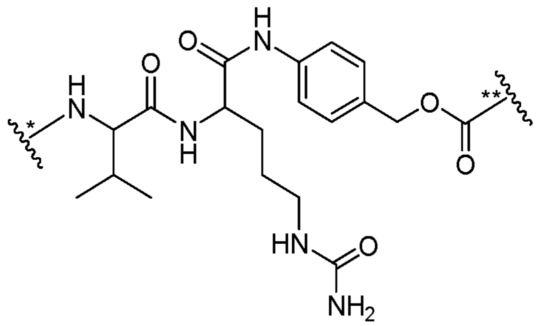

,

wherein * links to B, and ** links to D.

In one or more embodiments, L is

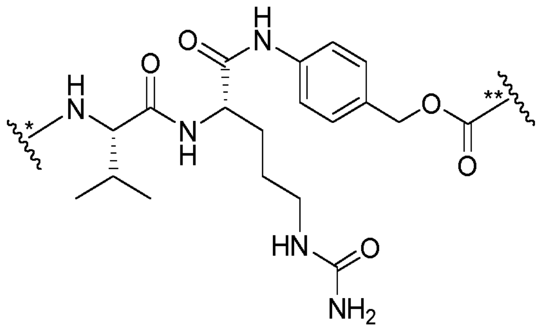

,

wherein * links to B, and ** links to D.

In one or more embodiments, G is

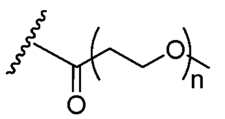

,

and n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

One or more embodiments of the present application provide an antibody-drug conjugate having a structure shown in Formula I-B, or a stereoisomer thereof, or a pharmaceutically acceptable salt or solvate thereof:

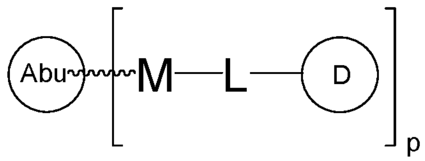

Formula I-B

Wherein

Abu is the anti-Nectin-4 antibody or the antigen-binding unit of the present application;

D is a drug (such as the drug described herein);

M is

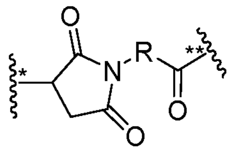

,

wherein * links to Abu, and ** links to L, R is selected from $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$, and $-(CH_2CH_2O)_rC(O)NR^m(CH_2)_r-$; wherein each $R^m$ is independently H, C1-C6 alkyl, C3-C8 carbocyclyl, phenyl, or benzyl; and each r is independently 1, 2, 3, 4, 5, 6, 7, 8, 9, or 10;

L is $-(AA)_i-(FF)_f-$, wherein AA is an amino acid or polypeptide, and i is 1, 2, 3, 4, 5, 6, 7, 8, 9, 10, 11, 12, 13, 14, 15, 16, 17, 18, 19, or 20; or each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly; each FF is independently

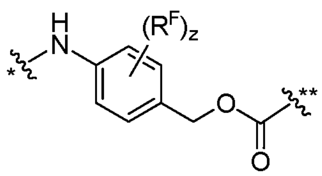

,

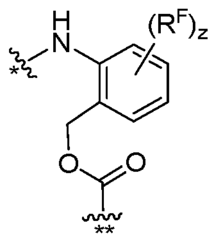

, or

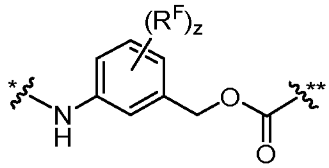

,

wherein each $R^F$ is independently C1-C6 alkyl, C1-C6 alkoxy, $-NO_2$, or halogen, wherein * links to AA, and ** links to D, and z is 0, 1, 2, 3, or 4; f is 1, 2, 3, 4, 5, 6, 7, 8, 9, or 10;

p is 1-10.

In one or more embodiments, R is $-(CH_2)_r-$, or r is 1 or 5.

In one or more embodiments, AA is Val-Cit, and i is 1.

In one or more embodiments, each FF is independently

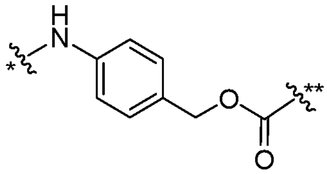,

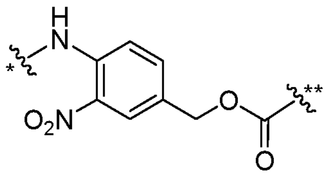, 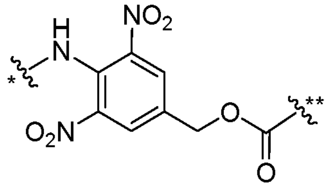, 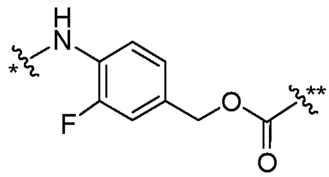,

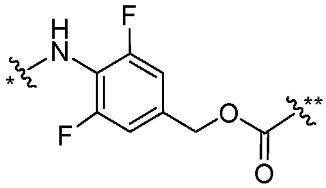, 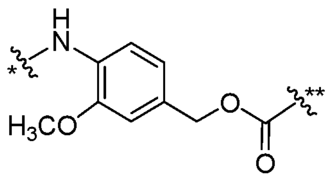, 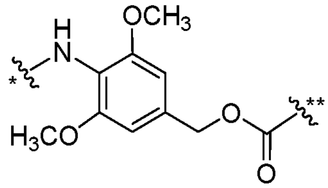,

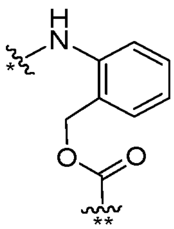, 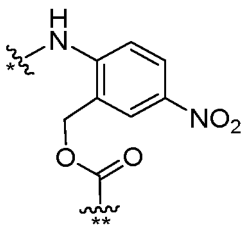, 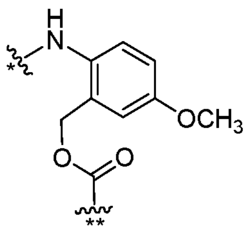, 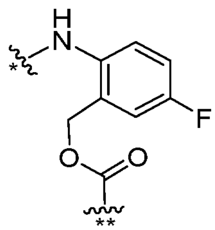, 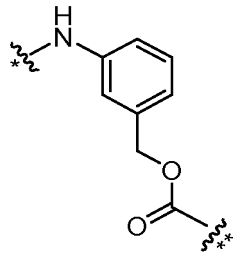,

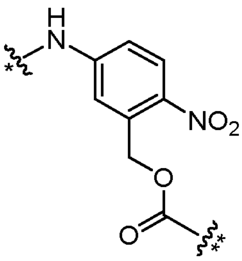, 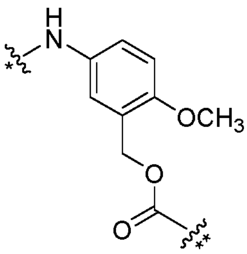, or 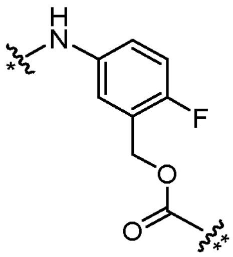;

wherein * links to AA, ** links to D.

In one or more embodiments, FF is

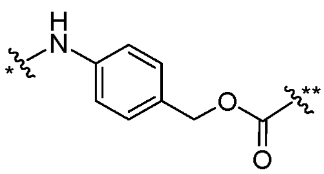,

and f is 1; wherein * links to AA, and ** links to D.

In one or more embodiments, L is

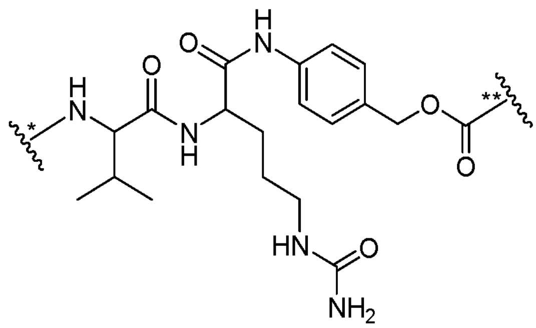

,

wherein * links to M, and ** links to D.

In one or more embodiments, L is

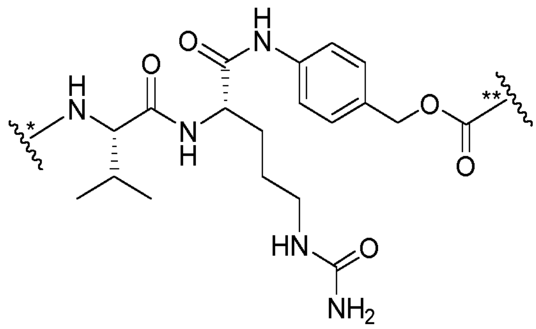

,

wherein * links to M, and ** links to D.

In one or more embodiments, p is 2-8.

In one or more embodiments, p is 4-8.

In one or more embodiments, p is 6-8.

In one or more embodiments, p is 7-8.

One or more embodiments of the present application provide an antibody-drug conjugate having a structure shown in Formula I-C, or a stereoisomer thereof, or a pharmaceutically acceptable salt or solvate thereof:

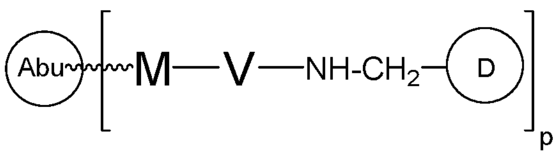

Formula I-C

Wherein

Abu is the anti-Nectin-4 antibody or the antigen-binding unit of the present application;

D is a drug (such as the drug described herein);

M is

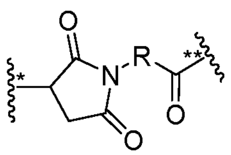

,

wherein * links to Abu, and ** links to V, R is selected from $-(CH_2)_r-$, $-(CHR^m)_r-$, C3-C8 carbocyclyl, $-O-(CH_2)_r-$, arylene,

$-(CH_2)_r$-arylene-, -arylene-$(CH_2)_r$-, $-(CH_2)_r$-(C3-C8 carbocyclyl)-, -(C3-C8 carbocyclyl)-$(CH_2)_r$-, C3-C8 heterocyclyl, $-(CH_2)_r$-(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$, and $-(CH_2CH_2O)_rC(O)NR^m(CH_2)_r-$; wherein each $R^m$ is independently H, C1-C6 alkyl, C3-C8 carbocyclyl, phenyl, or benzyl; and each r is independently 1, 2, 3, 4, 5, 6, 7, 8, 9, or 10;

V is

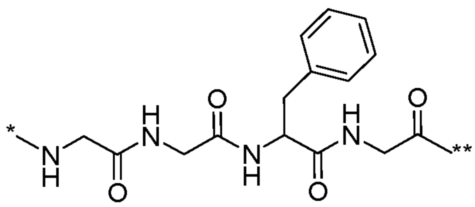

;

wherein * links to M, ** links to $-NH-CH_2-$;

p is 1-10.

In one or more embodiments, R is $-(CH_2)_r-$, or r is 1 or 5.

In one or more embodiments, V is

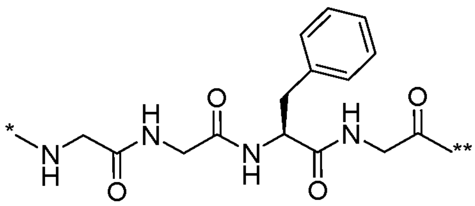

;

wherein * links to M, ** links to $-NH-CH_2-$.

In one or more embodiments, p is 2-8.

In one or more embodiments, p is 4-8.

In one or more embodiments, p is 6-8.

In one or more embodiments, p is 7-8.

One or more embodiments of the present application provide an antibody-drug conjugate having a structure shown in Formula I-A-1 or Formula I-A-2, or a stereoisomer thereof, or a pharmaceutically acceptable salt or solvate thereof, wherein the Formula I-A-1 and Formula I-A-2 are:

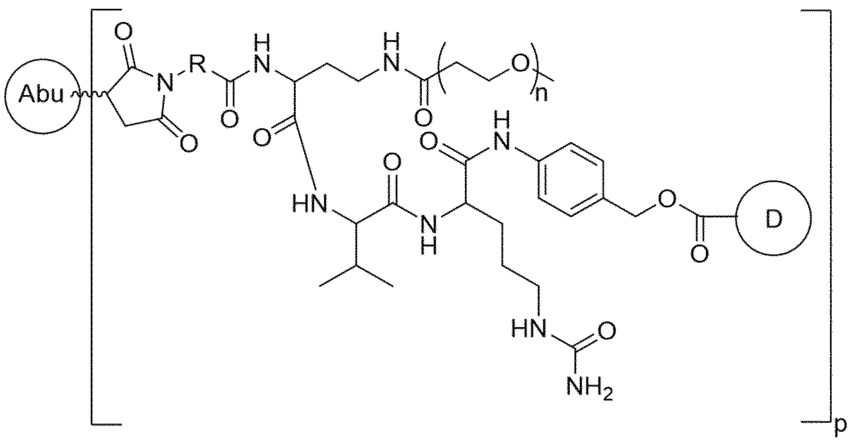

Formula I-A-1

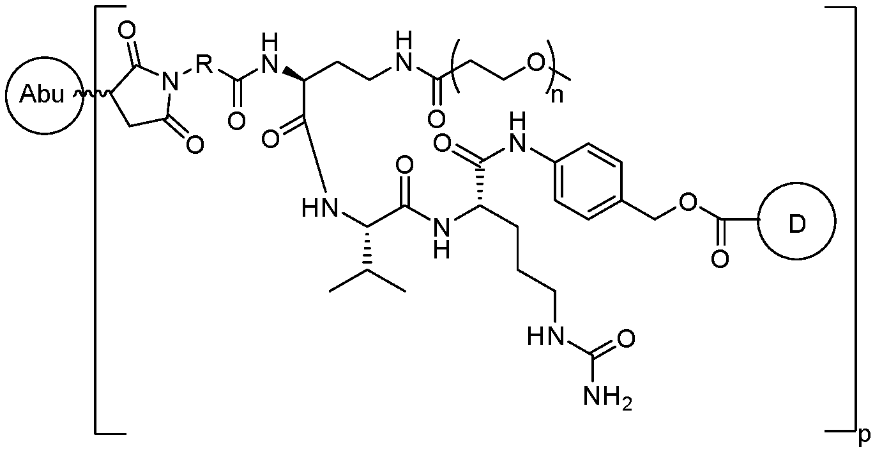

Formula I-A-2

Wherein

Abu is the anti-Nectin-4 antibody or the antigen-binding unit of the present application;

R is selected from $-(CH_2)_r-$, $-(CHR^m)_r-$, C3-C8 carbocyclyl, $-O-(CH_2)_r-$, arylene, $-(CH_2)_r$-arylene-, - arylene-$(CH_2)_r-$, $-(CH_2)_r$-(C3-C8 carbocyclyl)-, -(C3-C8 carbocyclyl)-$(CH_2)_r-$, C3-C8 heterocyclyl, - $(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, - $(CH_2CH_2O)_rC(O)NR^m(CH_2CH_2O)_r-$, $-(CH_2CH_2O)_rC(O)NR^m(CH_2CH_2O)_r-CH_2-$, and - $(CH_2CH_2O)_rC(O)NR^m(CH_2)_r-$; wherein each $R^m$ is independently H, C1-C6 alkyl, C3-C8 carbocyclyl, phenyl, or benzyl; and each r is independently 1, 2, 3, 4, 5, 6, 7, 8, 9, or 10;

D is a drug (such as the drug described herein);

n is an integer of 1-24; such as 1, 2, 3, 4, 5, 6, 7, 8, 9, 10, 11, 12, 13, 14, 15, 16, 17, 18, 19, 20, 21, 22, 23 or 24;

p is 1-10.

In one or more embodiments, R is $-(CH_2)_r-$, or r is 1 or 5.
In one or more embodiments, n is 4-12.
In one or more embodiments, n is 4-8.
In one or more embodiments, n is 4.
In one or more embodiments, n is 8.
In one or more embodiments, p is 2-8.
In one or more embodiments, p is 4-8.
In one or more embodiments, p is 6-8.
In one or more embodiments, p is 7-8.
One or more embodiments of the present application provide an antibody-drug conjugate having a structure shown in Formula I-A-3 or Formula I-A-4, or a stereoisomer thereof, or a pharmaceutically acceptable salt or solvate thereof, wherein the Formula I-A-3 and Formula I-A-4 are:

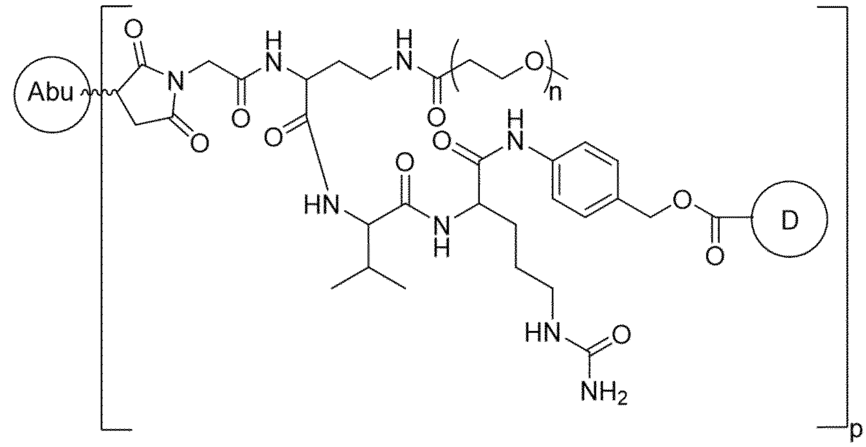

Formula I-A-3

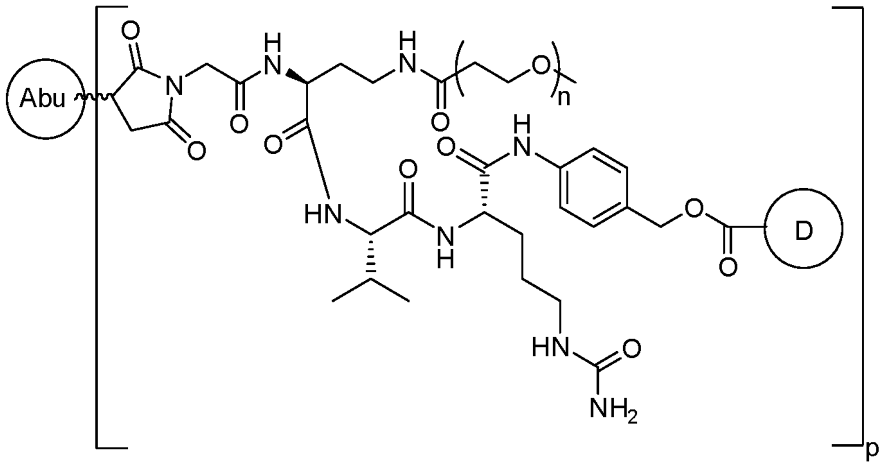

Formula I-A-4

Wherein

Abu is the anti-Nectin-4 antibody or the antigen-binding unit of the present application;

D is a drug (such as the drug described herein);

n is an integer of 1-24; such as 1, 2, 3, 4, 5, 6, 7, 8, 9, 10, 11, 12, 13, 14, 15, 16, 17, 18, 19, 20, 21, 22, 23 or 24;

p is 1-10.

In one or more embodiments, n is 4-12.
In one or more embodiments, n is 4-8.
In one or more embodiments, n is 4.
In one or more embodiments, n is 8.
In one or more embodiments, p is 2-8.
In one or more embodiments, p is 4-8.
In one or more embodiments, p is 6-8.
In one or more embodiments, p is 7-8.
One or more embodiments of the present application provide an antibody-drug conjugate having a structure shown in Formula I-A-5, I-A-6, I-A-7, I-A-8, I-A-9, I-A-10 or I-A-11, or a stereoisomer thereof, or a pharmaceutically acceptable salt or solvate thereof, wherein the Formulas I-A-5, I-A-6, I-A-7, I-A-8, I-A-9, I-A-10 and I-A-11 are:

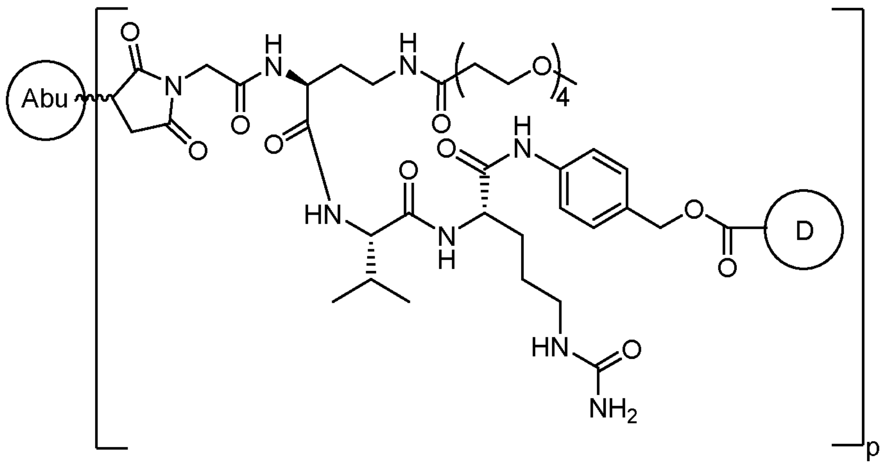

Formula I-A-5

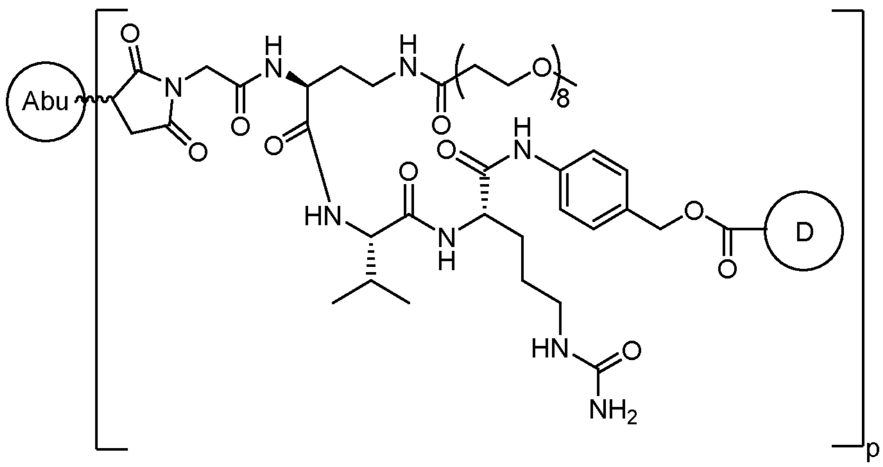

Formula I-A-6

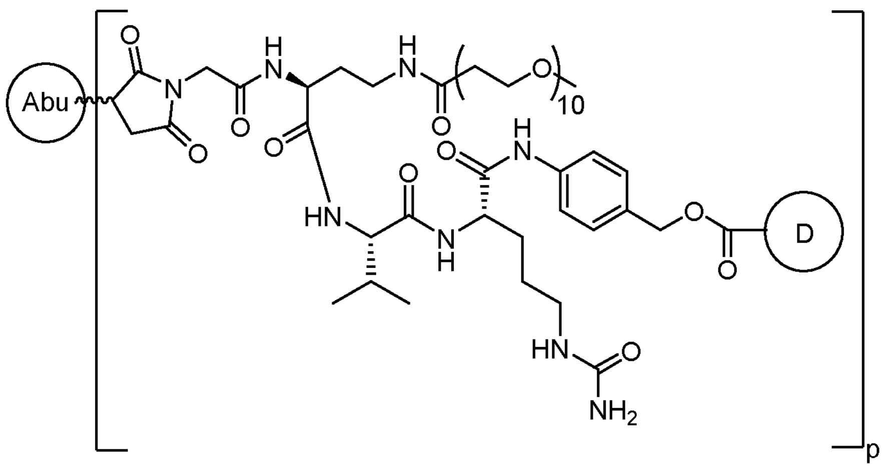

Formula I-A-7

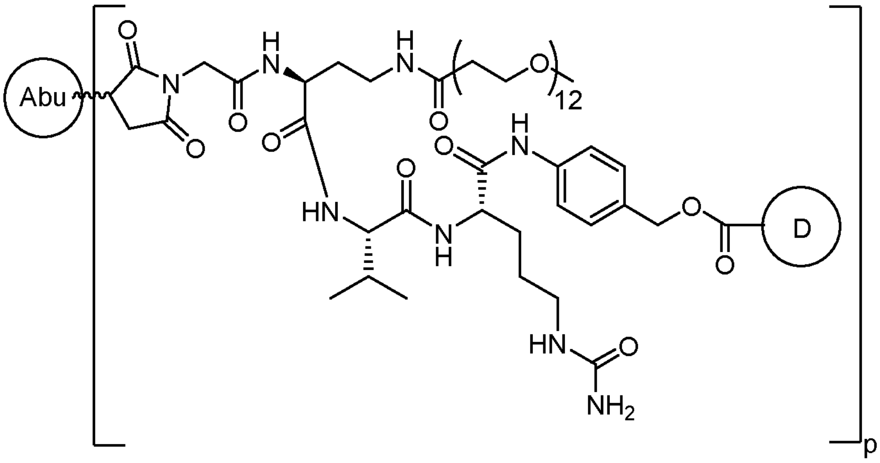

Formula I-A-8

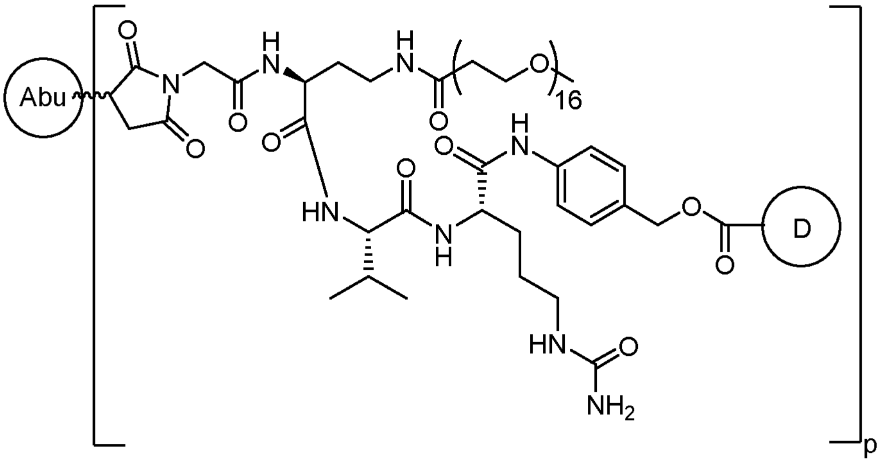

Formula I-A-9

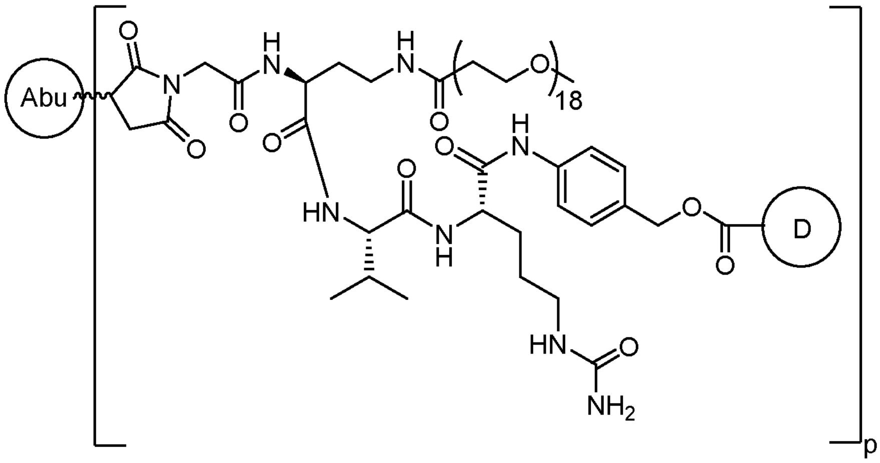

or

Formula I-A-10

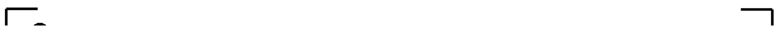

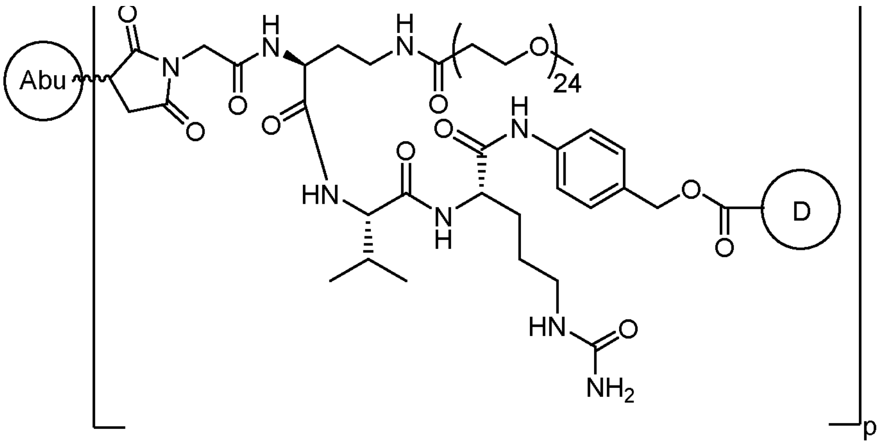

Formula I-A-11

Wherein

Abu is the anti-Nectin-4 antibody or the antigen-binding unit of the present application;
D is a drug (such as the drug described herein);
p is 1-10.

In one or more embodiments, p is 2-8.
In one or more embodiments, p is 4-8.
In one or more embodiments, p is 6-8.
In one or more embodiments, p is 7-8.
In one or more embodiments, the drug in the antibody-drug conjugate herein is a cytotoxic drug, a cell differentiation factor, a stem cell trophic factor, a steroid drug, a drug for treating autoimmune diseases, an anti-inflammatory drug, or a drug for treating an infectious disease.
In one or more embodiments, the drug is an anti-cancer drug.
In one or more embodiments, the drug is a tubulin inhibitor, a DNA damaging agent, or a DNA topoisomerase inhibitor.
In one or more embodiments, the drug is a tubulin inhibitor, and the tubulin inhibitor is selected from dolastatin, an auristatin, and a maytansine;
In one or more embodiments, the drug is an auristatin selected from monomethyl auristatin E (MMAE), monomethyl auristatin F (MMAF), and auristatin F (AF).
In one or more embodiments, the drug is a DNA damaging agent selected from a calicheamicins, a duocarmycin, and anthramycin derivative PBD (pyrrolobenzodiazepine).
In one or more embodiments, the drug is a DNA topoisomerase inhibitor or a salt thereof selected from irinotecan, irinotecan hydrochloride, camptothecin, 9-aminocamptothecin, 9-nitrocamptothecin, 10-hydroxycamptothecin, 9-chloro-10-hydroxycamptothecin, camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride, and *N*-[2-(dimethylamino)ethyl]-4-acridinecarboxamide.
In one or more embodiments, the DNA topoisomerase inhibitor is camptothecin, 10-hydroxycamptothecin, topotecan, belotecan, irinotecan, 22-hydroxyacuminatine, or exatecan.
In one or more embodiments, the drug is

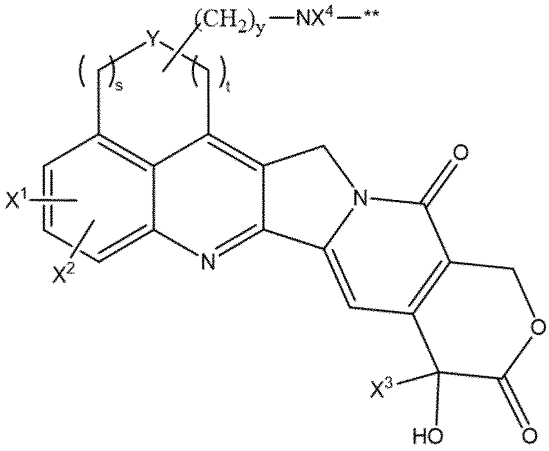

,

wherein

$X^1$ and $X^2$ are each independently:

H,

hydroxy,

C1-C6 alkyl,

C1-C6 alkyl substituted with one or more hydroxy, halogen, nitro, or cyano groups,

C2-C6 alkenyl,

C2-C6 alkynyl,

C1-C6 alkoxy,

C1-C6 aminoalkoxy,

halogen,

nitro,

cyano,

sulfhydryl,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclyl, wherein the heterocyclyl is optionally substituted with one or more C1-C6 alkyl, C1-C6 alkoxy, amino, halogen, nitro, or cyano groups,

C1-C6 alkylamino linking to a heterocyclyl, wherein the heterocyclyl is optionally substituted with C1-C6 alkyl or C1-C6 alkoxy, and the amino is optionally substituted with an amino-protecting group, halogen, nitro, cyano, or a protecting group,

amino-substituted heterocyclyl optionally substituted at a nitrogen atom of the heterocyclic moiety or at the amino

moiety with a protecting group or one or more C1-C6 alkyl groups,

heterocyclylamino optionally substituted at a nitrogen atom of the heterocyclyl moiety or at the amino moiety with a protecting group or C1-C6 alkyl,

carbamoyl optionally substituted with a carbamoyl-protecting group or C1-C6 alkyl,

morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, - $(CH_2)_qC(O)NR^n(CH_2)_q-CH_3$, $-(CH_2CH_2O)_q-CH_3$, $-(CH_2CH_2O)_q-CH_2-CH_3$, - $(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_20)_q-CH_3$, $-(CH_2CH_2O)_qC(O)NR^n(CH_2CH_2O)_q-CH_2-CH_3$, or - $(CH_2CH_2O)_qC(O)NR^n(CH_2)_q-CH_3$, wherein each $R^n$ is independently H, C1-C6 alkyl, C3-C8 carbocyclyl, phenyl, or benzyl, and each q is independently 1, 2, 3, 4, 5, 6, 7, 8, 9, or 10;

** is a linking point;

y is 0, 1, or 2;

Y is O, S, or $CR^{1D}R^{2D}$, wherein $R^{1D}$ and $R^{2D}$ are each independently H or C1-C6 alkyl;

s and t are each independently 0, 1, or 2, but not both 0.

In one or more embodiments, $X^4$ is H or C1-C6 alkyl.

In one or more embodiments, the drug is

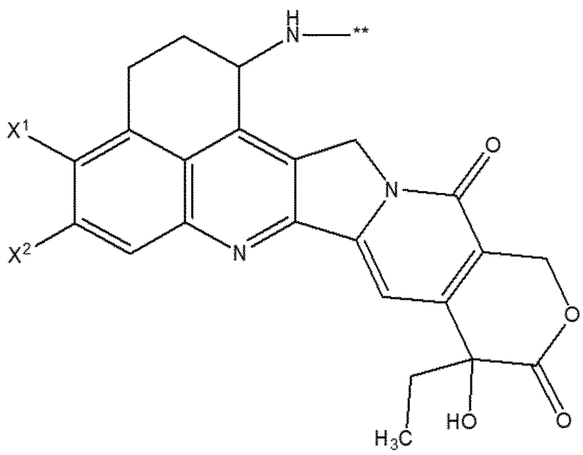

,

wherein $X^1$ and $X^2$ are each independently C1-C6 alkyl, halogen, or -OH, ** is a linking point.

In one or more embodiments, the C1-C6 alkyl is $-CH_3$

In one or more embodiments, the halogen is F.

In one or more embodiments, the drug is

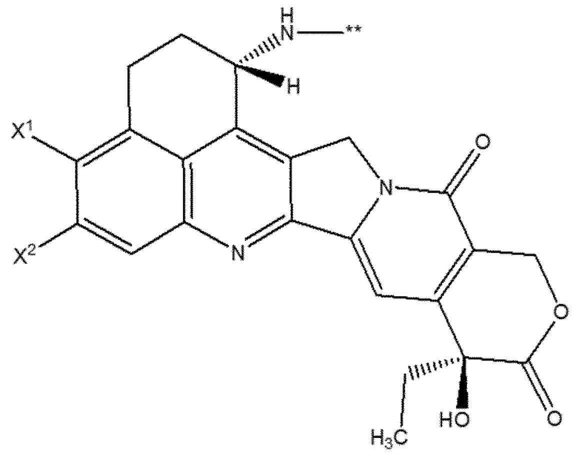

,

wherein $X^1$ and $X^2$ are each independently C1-C6 alkyl, halogen, or -OH, ** is a linking point.

In one or more embodiments, the C1-C6 alkyl is $-CH_3$.

In one or more embodiments, the halogen is F.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F, or $-CH_3$.

In one or more embodiments, $X^1$ is $-CH_3$ and $X^2$ is F.

In one or more embodiments, the drug is

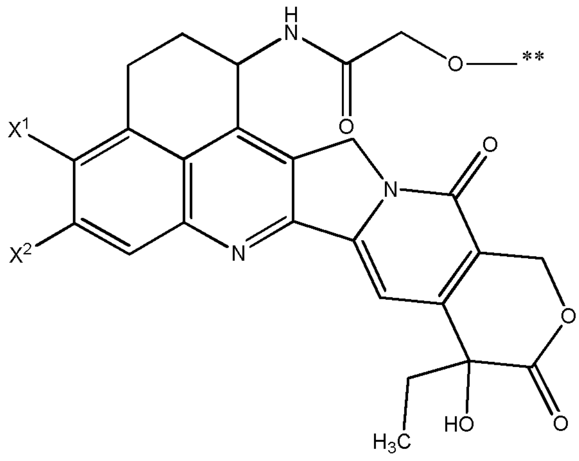

,

wherein $X^1$ and $X^2$ are each independently C1-C6 alkyl, halogen, or -OH, ** is a linking point.

In one or more embodiments, the C1-C6 alkyl is $-CH_3$.

In one or more embodiments, the halogen is F.

In one or more embodiments, the drug is

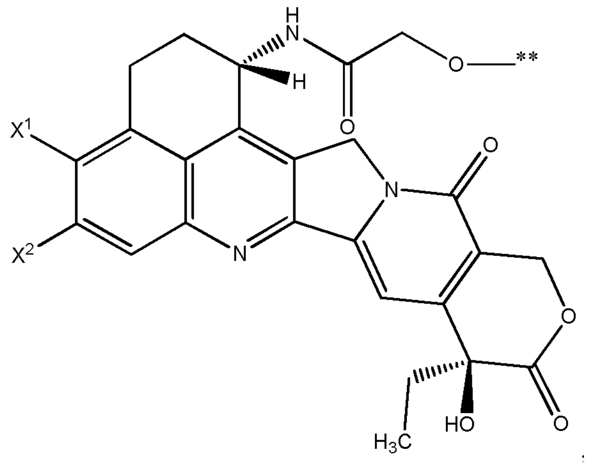

wherein $X^1$ and $X^2$ are each independently C1-C6 alkyl, halogen, or -OH, ** is a linking point.

In one or more embodiments, the C1-C6 alkyl is $-CH_3$.

In one or more embodiments, the halogen is F.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F, or $-CH_3$.

In one or more embodiments, $X^1$ is $-CH_3$ and $X^2$ is F.

In one or more embodiments, the drug is

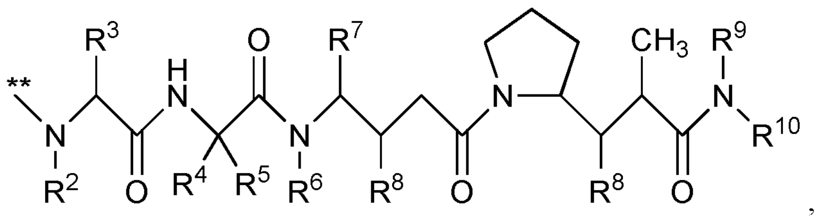

wherein ** is a linking point;

$R^2$ is H or C1-C8 alkyl;

$R^3$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or $C_1$-$C_8$ alkyl-(C3-C8 heterocyclyl);

$R^4$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);

$R^5$ is H or methyl;

or $R^4$ and $R^5$ are linked to form a carbocyclyl having a formula of $-(CR^aR^b)_j-$, wherein $R^a$ and $R^b$ are each independently H, C1-C8 alkyl, or C3-C8 carbocyclyl, and j is 2, 3, 4, 5, or 6;

$R^6$ is H or C1-C8 alkyl;

$R^7$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);

each $R^8$ is independently H, OH, $C_1$-$C_8$ alkyl, C3-C8 carbocyclyl, or O-(C1-C8 alkyl);

$R^9$ is H or C1-C8 alkyl; and

$R^{10}$ is $-C(R^8)_2-C(R^8)_2$-aryl, $-C(R^8)_2-C(R^8)_2$-(C3-C8 heterocyclyl), or $-C(R^8)_2-C(R^8)_2$-(C3-C8 carbocyclyl).

In one or more embodiments, the drug is

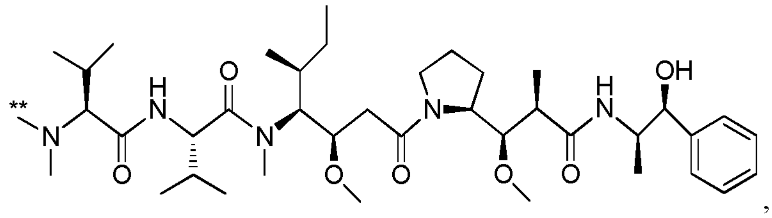

wherein ** is a linking point.

One or more embodiments of the present application provide an antibody-drug conjugate having a structure shown in Formula I-A-12, I-A-13, I-A-14, I-A-15, I-A-16, I-A-17, I-A-18, I-A-19, I-A-20, I-A-21, I-A-22, I-A-23, I-A-24, or I-A-25 or a stereoisomer thereof, or a pharmaceutically acceptable salt or solvate thereof, wherein the Formulas I-A-12, I-A-13, I-A-14, I-A-15, I-A-16, I-A-17, I-A-18, I-A-19, I-A-20, I-A-21, I-A-22, I-A-23, I-A-24, and I-A-25 are:

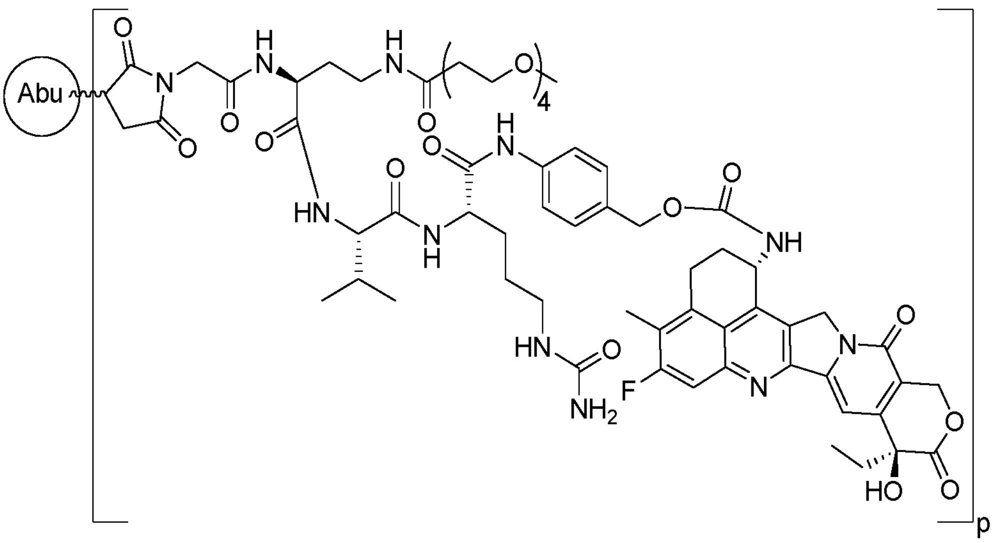

Formula I-A-12

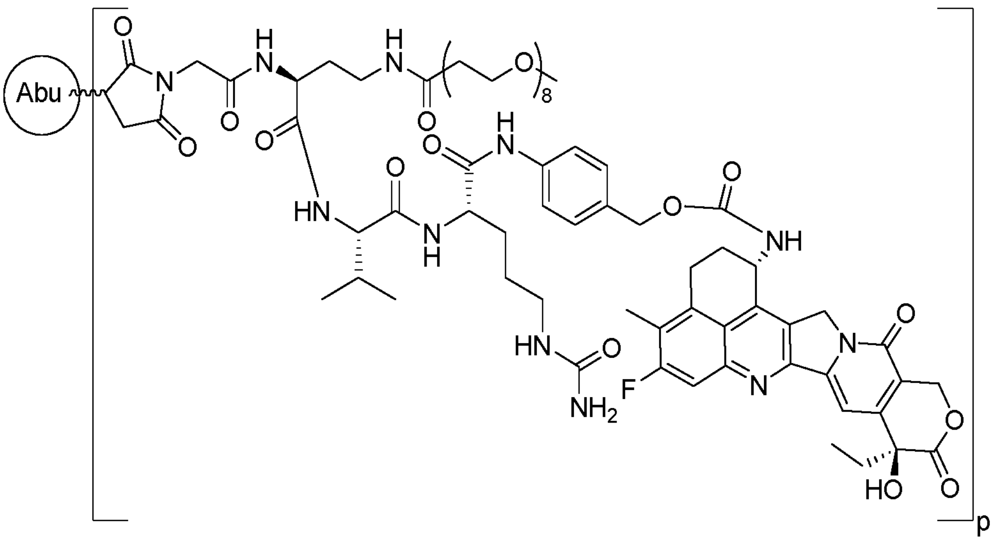

Formula I-A-13

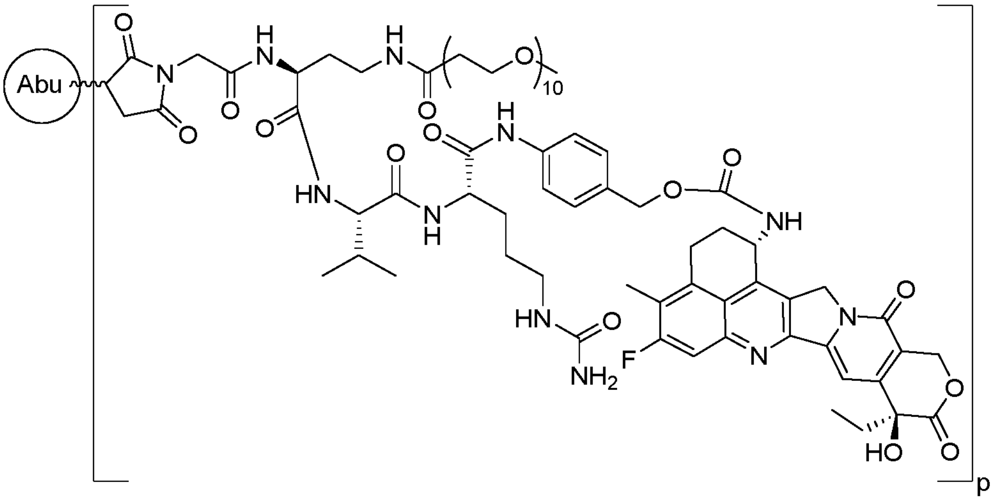

Formula I-A-14

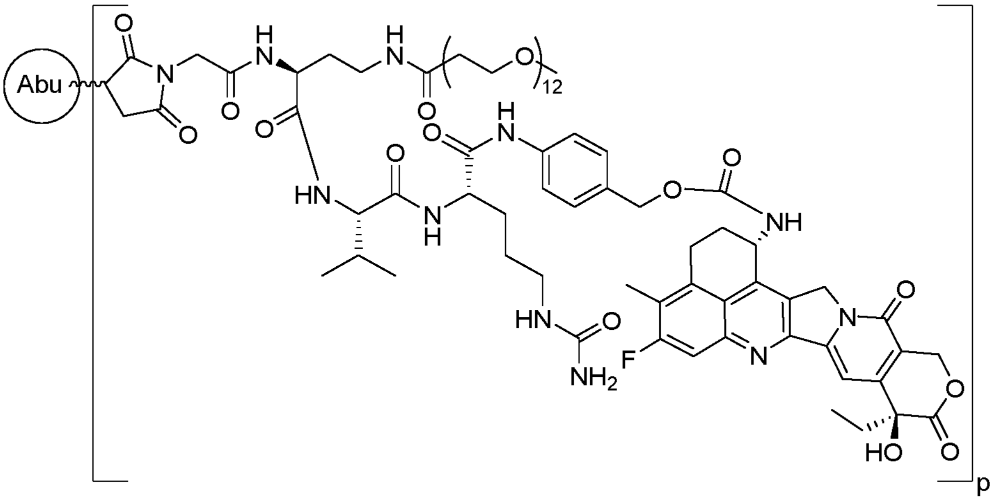

Formula I-A-15

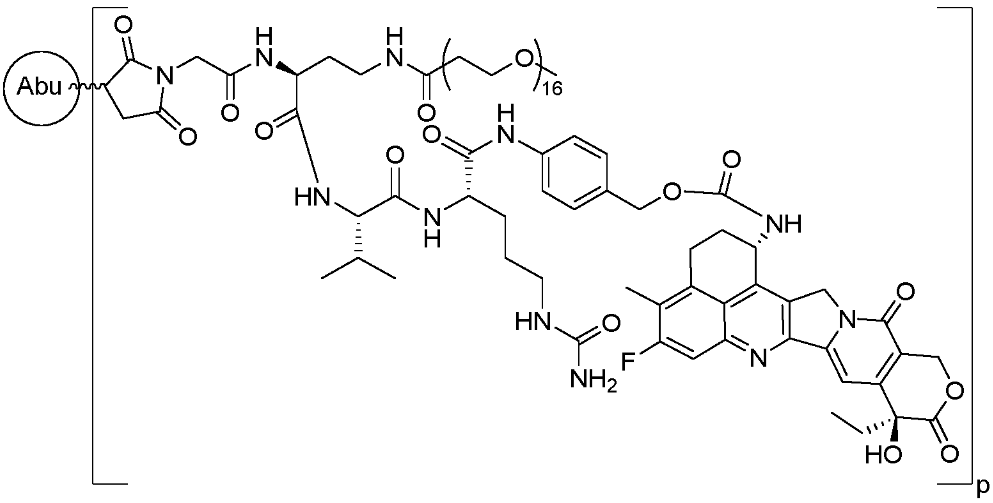

Formula I-A-16

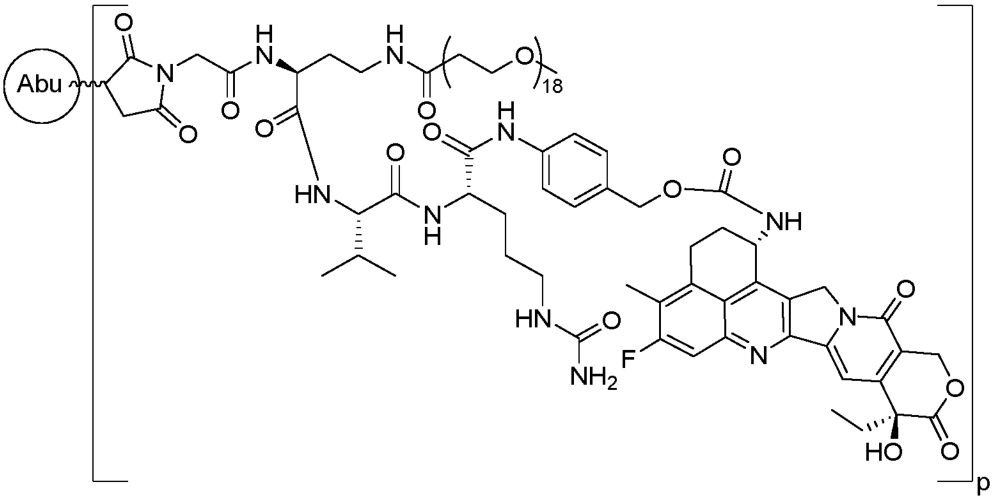

Formula I-A-17

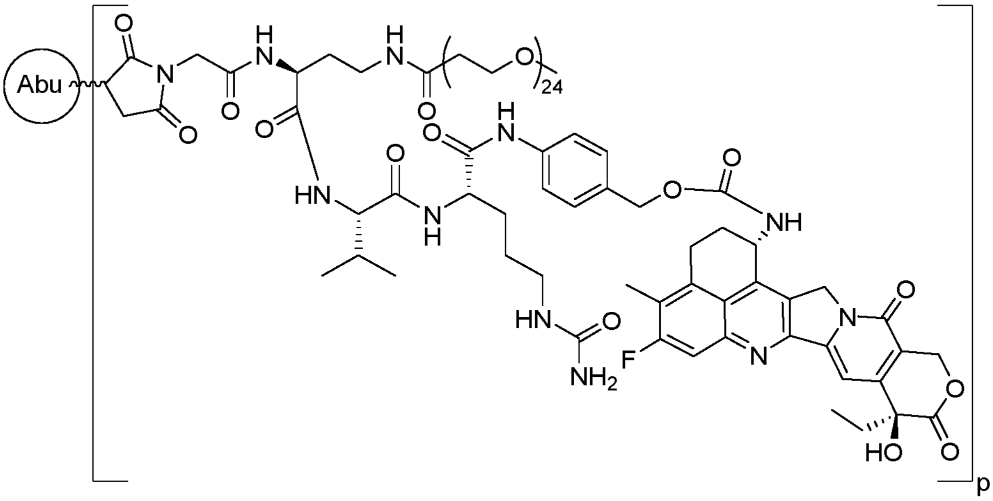

Formula I-A-18

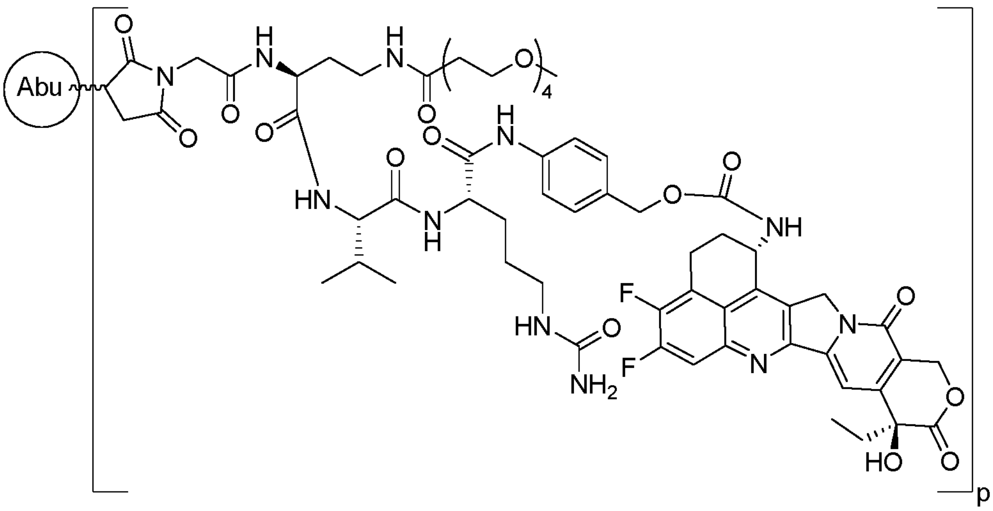

Formula I-A-19

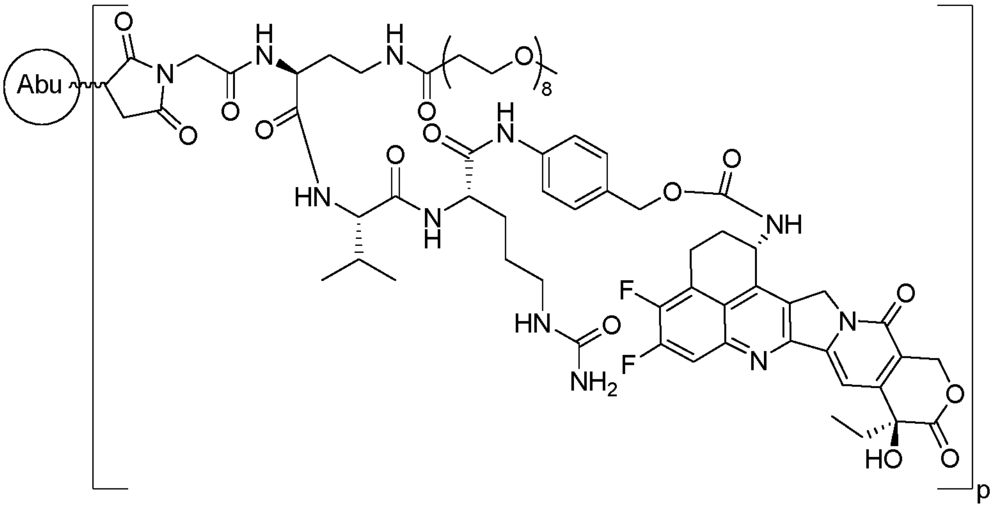

Formula I-A-20

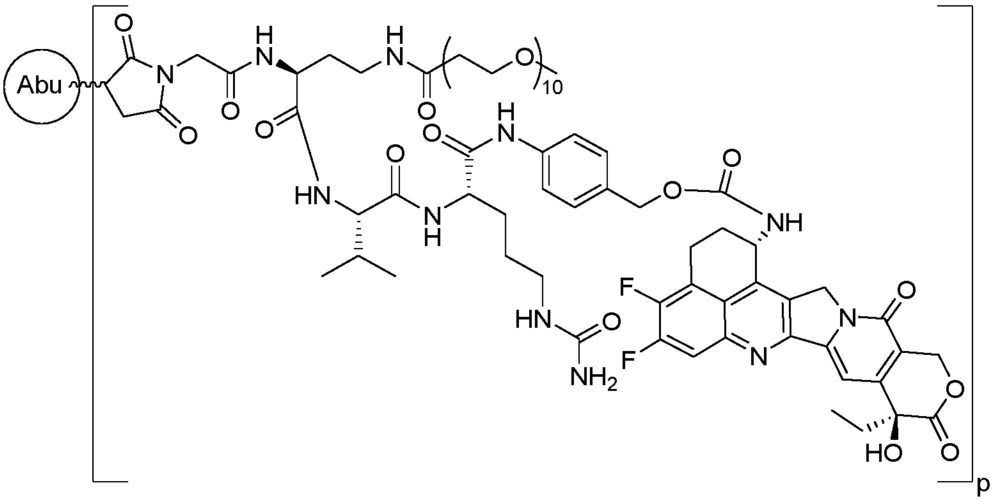

Formula I-A-21

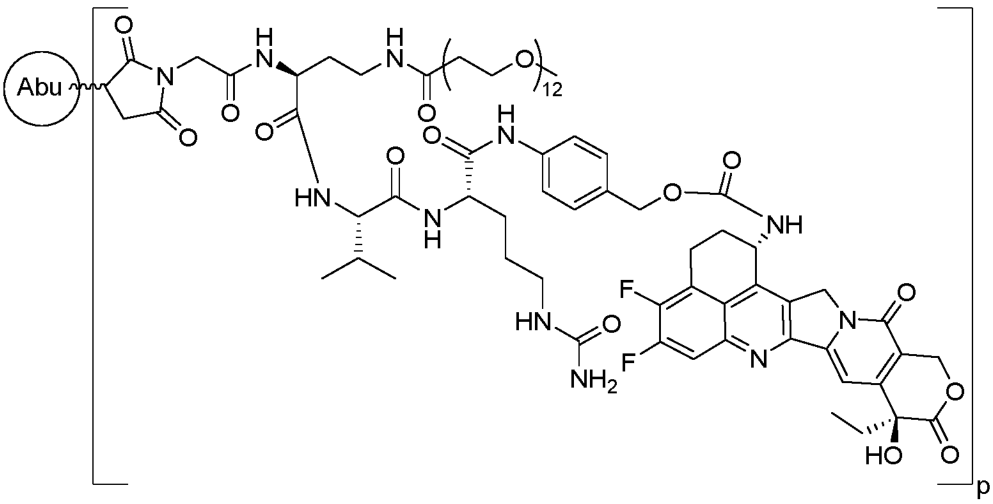

Formula I-A-22

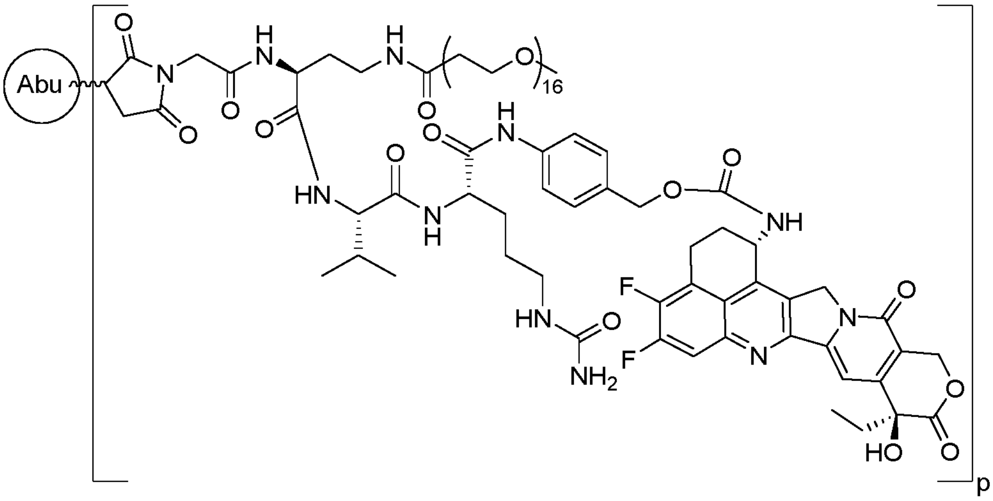

Formula I-A-23

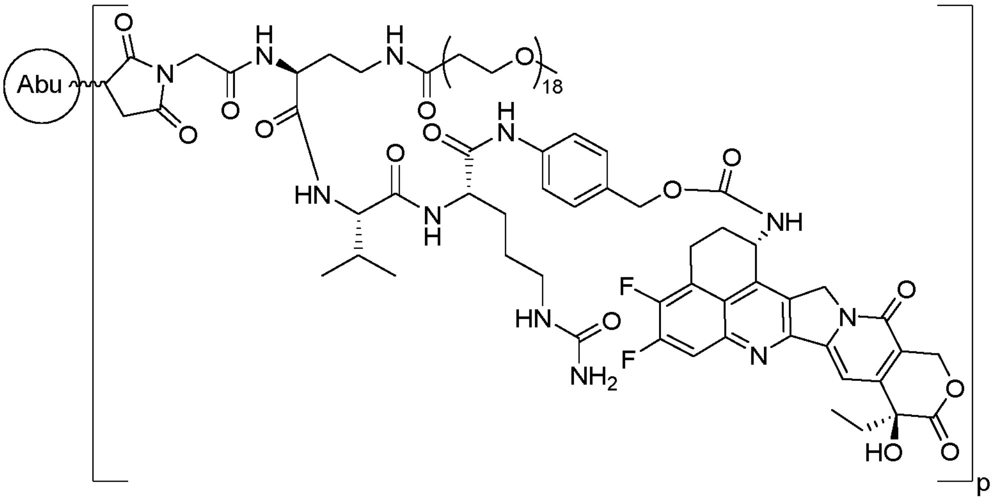

Formula I-A-24

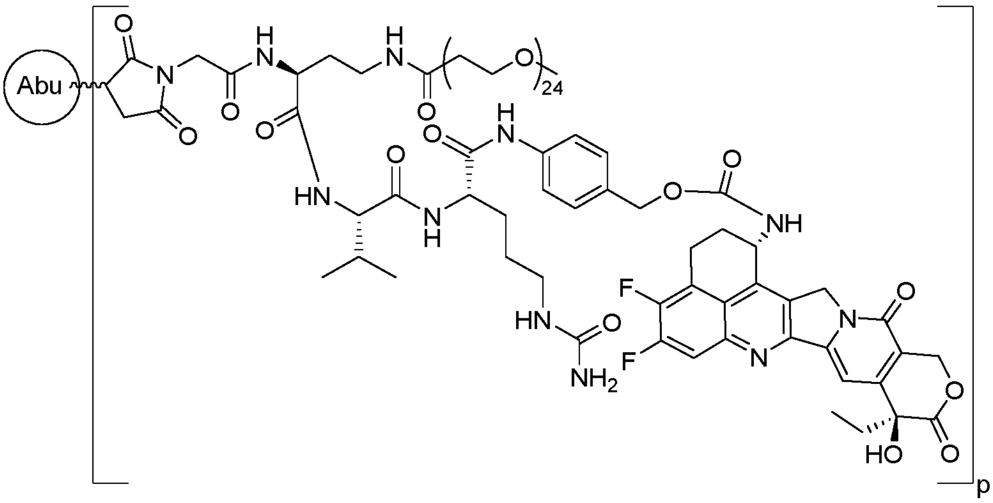

Formula I-A-25

Wherein

Abu is the anti-Nectin-4 antibody or the antigen-binding unit of the present application;

p is 1-10.

In one or more embodiments, p is 2-8.
In one or more embodiments, p is 4-8.
In one or more embodiments, p is 6-8.
In one or more embodiments, p is 7-8.
One or more embodiments of the present application provide an antibody-drug conjugate having a structure shown in Formula I-B-1 or a stereoisomer thereof, or a pharmaceutically acceptable salt or solvate thereof, wherein the Formulas I-B-1 is:

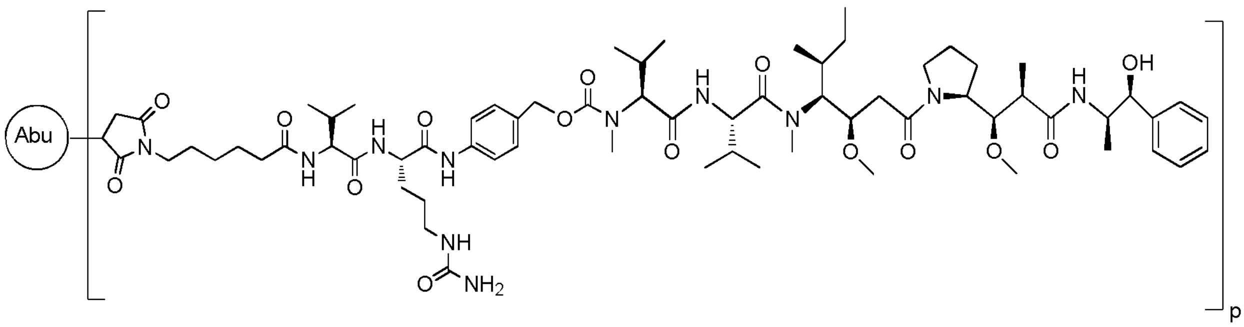

Formula I-B-1

Wherein

Abu is the anti-Nectin-4 antibody or the antigen-binding unit of the present application;

p is 1-10.

In one or more embodiments, p is 2-8.
In one or more embodiments, p is 4-8.
In one or more embodiments, p is 6-8.
In one or more embodiments, p is 7-8.
One or more embodiments of the present application provide an antibody-drug conjugate having a structure shown in Formula I-C-1 or a stereoisomer thereof, or a pharmaceutically acceptable salt or solvate thereof, wherein the Formulas I-C-1 is:

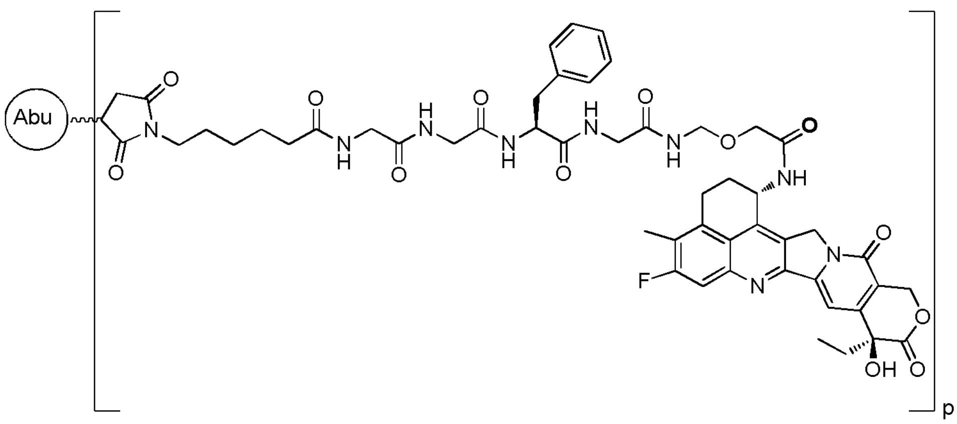

Formula I-C-1

Wherein

Abu is the anti-Nectin-4 antibody or the antigen-binding unit of the present application;

p is 1-10.

In one or more embodiments, p is 2-8.

In one or more embodiments, p is 4-8.

In one or more embodiments, p is 6-8.

In one or more embodiments, p is 7-8.

In one or more embodiments, a pharmaceutical composition is provided comprising the anti-Nectin-4 antibody or the antigen-binding unit of the present application, the biomaterial of the present application, or the antibody-drug conjugate of the present application or the pharmaceutically acceptable salt or solvate thereof , and a pharmaceutically acceptable carrier, excipient, and/or adjuvant material. In one or more embodiments, the pharmaceutical composition further comprises an additional drug.

In one or more embodiments, a pharmaceutical composition is provided comprising an antibody or an antigen-binding unit, an antibody-drug conjugate or a pharmaceutically acceptable salt or solvate thereof , and a pharmaceutically acceptable carrier, excipient, and/or adjuvant material. In one or more embodiments, the pharmaceutical composition further comprises an optionally additional anti-cancer drug. The pharmaceutical composition may be administered by any convenient route, e.g., by infusion or bolus injection, by absorption through epithelial or cutaneous mucosa (e.g., oral mucosa or rectal and intestinal mucosa), and may be co-administered with other biologically active agents. Therefore, the pharmaceutical composition may be administered subcutaneously, intravenously, orally, rectally, parenterally, intracisternally, intravaginally, intraperitoneally, topically (e.g. through powder, ointment, drops or transdermal patch), buccally or by oral or nasal spray.

In some embodiments, the composition is formulated into a pharmaceutical composition suitable for intravenous injection into a human body according to conventional steps. A composition for intravenous administration is usually a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture, for example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administrated by injection, an ampoule bottle containing sterile water or saline for injection can be used, so that the active ingredients can be mixed before administration.

In one or more embodiments, provided is use of the anti-Nectin-4 antibody or the antigen-binding unit, the biomaterial, the antibody-drug conjugate or the pharmaceutically acceptable salt or solvate thereof, or the pharmaceutical composition described herein in preparing a medicament for treating a disease. In one or more embodiments, the medicament is also used in combination with an additional drug.

In one or more embodiments, provided is use of the antibody or the antigen-binding unit described herein, the biomaterial described herein, the antibody-drug conjugate or the pharmaceutically acceptable salt or solvate thereof described herein, or the pharmaceutical composition described herein in treating a disease. In one or more embodiments, the use includes combined use with an additional anti-cancer drug.

In one or more embodiments, the present invention further provides a method for treating a disease, comprising: administering to a patient in need an effective amount of the antibody or the antigen-binding unit, the antibody-drug conjugate or the pharmaceutically acceptable salt or solvate thereof, or the pharmaceutical composition described herein. The effective amount refers to an amount of an active compound or drug that results in a biological or pharmacological response in tissues, systems, animals, individuals, and humans which is being sought by researchers, vets, doctors, or other clinical physicians, including the treatment of a disease. In general, the effective amount can be about 0.1-100 mg/kg, and the administration frequency can be, for example, once a month. The administration method can be intravenous infusion, intravenous push injection, subcutaneous injection, intramuscular injection, etc. In one or more embodiments, the method further comprises administering additional anti-cancer drugs to the patient.

In one or more embodiments, the disease is a disease associated with the expression or overexpression of Nectin-4. In one or more embodiments, the disease is a disease associated with the abnormal expression of Nectin-4. In one or more embodiments, the disease is a cancer or tumor. In one or more embodiments, the disease is a tumor expressing or overexpressing Nectin-4. In one or more embodiments, the disease is a cancer expressing or overexpressing Nectin-4. In one or more embodiments, the disease is a solid tumor or a hematologic cancer. In one or more embodiments, the disease is selected from breast cancer (e.g., triple-negative breast cancer (TNBC); topical or metastatic TNBC), pancreatic cancer, bladder cancer, urothelial carcinoma, melanoma, lung cancer (e.g., non-small cell lung cancer, squamous cell cancer, or lung adenocarcinoma), head and neck cancer (e.g., head and neck squamous cell carcinoma), cervical cancer (e.g., cervical squamous cell carcinoma), ovarian cancer, choriocarcinoma, skin cancer, esophageal cancer (e.g., esophageal adenocarcinoma), gastric cancer, uterine cancer (e.g., endometrial cancer), gallbladder cancer, liver cancer, hepatocellular carcinoma, urinary tract cancer, renal pelvic carcinoma, ureteral cancer, colorectal cancer,

colon cancer, and prostate cancer.

In one or more embodiments, the antibody-drug conjugate or the pharmaceutically acceptable salt or solvate thereof is the antibody-drug conjugate of Formula I-A, Formula I-B or Formula I-C of the present application or the pharmaceutically acceptable salt or solvate thereof .

The pharmaceutically acceptable salts include the antibody-drug conjugates, with a variety of organic and inorganic counterions well known in the art, and salts as examples only include organic or inorganic salts such as sodium salt, potassium salt, calcium salt, magnesium salt, ammonium salt, isopropylamine, trimethylamine, diethylamino, triethylamine, tripropylamine, ethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine piperazine, piperidine, N-ethyl, piperidine, polyamine resin, and tetraalkylammonium salt, etc,when the molecule contains an acidic functional group; and organic or inorganic acid salts such as hydrochloride salt, hydrobromide salt, tartrate salt, mesylate salt, acetate salt, maleate salt and oxalate salt when the molecule contains a basic functional group. Other non-limiting examples of acids include sulfuric acid, nitric acid, phosphoric acid, propionic acid, glycolic acid, pyruvic acid, malonic acid, succinic acid, fumaric acid, tartaric acid, citric acid, benzoic acid cinnamic acid, mandelic acid, methanesulfonic acid, ethanesulfonic acid, p-toluenesulfonic acid salicylic acid, etc. The solvate includes hydrates.

One or more embodiments provide a product comprising the antibody or the antigen-binding unit, the antibody-drug conjugate, or the pharmaceutically acceptable salt or the solvate thereof, or the pharmaceutical composition described herein;

a container; and

a package insert, instructions or label indicating that the antibody or the antigen-binding unit, the antibody-drug conjugate, or the pharmaceutically acceptable salt or the solvate thereof, or the pharmaceutical composition is for use in treating a disease.

## BRIEF DESCRIPTION OF THE DRAWINGS

FIG. 1 illustrates the binding curves of antibodies ASG-AM and ASG-22 to T47D cells;

FIG. 2 illustrates the proliferation inhibitory effects of ADCs on OVCAR-3, T-47D, and MDA-MB-468 cells, wherein FIG. 2A illustrates the proliferation inhibitory effects of ADCs on OVCAR-3 cells, FIG. 2B illustrates the proliferation inhibitory effects of ADCs on T-47D cells; FIG. 2C illustrates the proliferation inhibitory effect of ASG-AM-MMAE on MDA-MB-468 cells;

FIG. 3 illustrates the tumor inhibitory activity of ADCs in a human breast cancer MDA-MB-468 nude mouse subcutaneous xenograft tumor model;

FIG. 4 illustrates the tumor inhibitory activity of ADCs in a human choriocarcinoma JEG-3 cell subcutaneous xenograft female NOD/SCID mouse animal model.

## DETAILED DESCRIPTION

Unless otherwise defined, scientific and technical terms used in the present invention have the meanings that are commonly understood by those skilled in the art.

### Definition

It should be noted that the term "an" entity refers to one or more of the entities. For example, "an antibody" should be interpreted as one or more antibodies. As such, the terms "a" (or "an"), "one or more" and "at least one" can be used interchangeably herein.

As used herein, the term "contain" or "comprise" means that the antibodies, compositions, methods or the like comprise the recited elements, such as components or steps and do not exclude the others. "Consisting essentially of... " means that the antibodies, compositions, methods or the like exclude other elements that have a fundamental impact on the characteristics of the combination, but do not exclude elements that do not substantially affect the characteristics of the antibodies, compositions, methods or the like. "Consisting of..." means that elements not specifically listed are excluded.

The term "antibody" as used herein refers to immunoglobulin (Ig) molecules and immunologically active portions

of immunoglobulin molecules, i.e., molecules that contain an antigen-binding site that specifically binds to (immunoreacts with) an antigen. Antibodies include, but are not limited to, monoclonal antibodies, chimeric antibodies, dAbs (domain antibodies), single-chain antibodies, Fab, Fab- and $F(ab')_2$ fragments, Fv and Fab expression libraries.

The term "antibody" includes a wide variety of polypeptides that can be biochemically distinguished.

The antibodies, antigen-binding units or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies, epitope-binding fragments (e.g., Fab, Fab' and $F(ab')_2$), and single-chain Fv (scFv).

The term "monoclonal antibody" (mAb) refers to a population of antibody molecules that contain only one molecular species of the antibody molecules consisting of a unique light chain gene product and a unique heavy chain gene product. In particular, the complementarity determining regions (CDRs) of the monoclonal antibody are identical in all the molecules of the population. MAbs contain an antigen-binding site capable of immunoreacting with a particular epitope of an antigen.

The term "single-chain antibody" (scFv) refers to an antibody in which its heavy chain variable region (VH) and light chain variable region (VL) are linked by a linker of 15-20 amino acids. The linker may be enriched with glycine to improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

Those skilled in the art will appreciate that the classes of heavy chains include gamma, mu, alpha, delta, or epsilon ($\gamma$, $\mu$, $\alpha$, $\delta$, or $\varepsilon$), and some subclasses (e.g., $\gamma$1-$\gamma$4). The nature of this chain determines the "type" of the antibody as IgG, IgM, IgA, IgD or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In one or more embodiments, the type of immunoglobulin molecule is IgG. Two heavy chains and two light chains are connected in a "Y" configuration through disulfide bonds, wherein the light chain starts at the opening of "Y" configuration and extends through the variable region to surround the heavy chain.Light chains can be classified into kappa ($\kappa$) or lambda ($\lambda$). Each heavy chain may bind to a $\kappa$ or $\lambda$ light chain. In general, when an immunoglobulin is produced by a hybridoma, a B cell or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N terminus at the forked end of the Y configuration to the C terminus at the bottom of each chain. The immunoglobulin $\kappa$ light chain variable region is $V_\kappa$; and the immunoglobulin $\lambda$ light chain variable region is $V_\lambda$.

The light chain variable region (VL) and the heavy chain variable region (VH) of the antibody determine the antigen recognition and specificity. The light chain constant region (CL) and the heavy chain constant region (CH) impart important biological properties such as secretion, transplacental movement, Fc receptor binding, complement fixation, etc. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region ("FR"), exhibit little intermolecular variability. Most of the framework regions adopt a $\beta$-sheet conformation, with the CDRs forming a loop structure connected to, or in some cases forming part of the $\beta$-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. Generally, in an antibody molecule, the heavy and light chains each have three CDRs, referred to as VH CDR1, VH CDR2, VH CDR3, VL CDR1, VL CDR2, and VL CDR3, respectively. In positional order, the heavy chain variable region typically comprises VH FR1, VH CDR1, VH FR2, VH CDR2, VH FR3, VH CDR3, and VH FR4, and the light chain variable region comprises VL FR1, VL CDR1, VL FR2, VL CDR2, VL FR3, VL CDR3, and VL FR4. For a given heavy or light chain variable region, amino acids in the CDRs and the framework regions may be identified by those of ordinary skill in the art according to known methods (see, Kabat, E., et al., U.S. Department of Health and Human Services, Sequences of Proteins of Immunological Interest, (1983) and Chothia and Lesk, J. Mol. Biol., 196: 901-917 (1987)).

The framework and CDR regions of a humanized antibody need not correspond precisely to the parental sequences, e.g., the donor antibody CDR or the consensus framework may be mutagenized by substitution, insertion and/or deletion of at least one amino acid residue so that the CDR or framework residue at that site does not correspond to

either the donor antibody or the consensus framework. Usually, at least 80%, at least 85%, at least 90% or at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (See e.g., Winnaker, From Genes to Clones (Verlagsgesellschaft, Weinheim, Germany 1987)). In a family of immunoglobulins, each position in the consensus sequence is occupied by the amino acid occurring most frequently at that position in the family. If two amino acids occur equally frequently, either can be included in the consensus sequence.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found within the variable regions of heavy and light chain polypeptides. This specific region is described in Kabat et al., U.S. Dept. of Health and Human Services, Sequences of Proteins of Immunological Interest (1983) and Chothia et al., J. Mol. Biol. 196:901-917 (1987), which are incorporated herein by reference in their entirety.

Kabat et al. also define a numbering scheme applicable to the variable region sequence of any antibody. One of ordinary skills in the art can apply the "Kabat numbering" scheme to any variable region sequence without depending on other experimental data beyond the sequence itself. "Kabat numbering" refers to the numbering system proposed by Kabat et al., U.S. Dept. of Health and Human Services in Sequence of Proteins of Immunological Interest (1983). EU or Chothia numbering scheme can also be applicable to the antibody.

The antibodies disclosed herein may be derived from any animal, including fish, birds and mammals. Preferably, the antibody is derived from a human being, a mouse, a donkey, a rabbit, a goat, a camel, a llama, a horse, or a chicken source. In another embodiment, the variable region may be derived from a condricthoid source (e.g., from a shark).

The "heavy chain constant region" comprises at least one of a CH1 domain, a hinge (e.g., upper, middle, and/or lower hinge region) domain, a CH2 domain, a CH3 domain, or a variant or a fragment. The heavy chain constant regions of the antibody may be derived from different immunoglobulin molecules. For example, the heavy chain constant regions of the antibody may comprise a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant κ domain or a constant λ domain. "Light chain-heavy chain pair" refers to a collection of light and heavy chains that can form dimers through disulfide bonds between the CL domain of the light chain and the CH1 domain of the heavy chain.

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and the constant region thereof (which may be intact, partial or modified) is derived from a second species. In certain embodiments, the variable region is derived from a non-human source (e.g., mouse or primate) and the constant region is derived from a human source.

The term "epitope" as used herein includes any protein determining region that can specifically bind to an immunoglobulin or a fragment thereof or a T cell receptor. The epitope determinant generally consists of chemically active surface groups of molecules (such as amino acids or sugar side chains), and generally has particular three-dimensional structural properties and specific charge properties.

As used herein, the term "specific bind to" or "immune response" refers to a non-covalent interaction between an immunoglobulin molecule and one or more antigenic determinants of its target antigen. The strength or affinity of an immunological binding interaction can be expressed in terms of the equilibrium dissociation constant (KD) of the interaction, where a smaller KD represents a greater affinity. The immunological binding properties of the selected polypeptides may be quantified using methods well known in the art. One such method requires the measurement of the rates of antigen-binding site/antigen complex formation and dissociation, where those rates depend on the concentration of the complex partners, the affinity of the interaction and geometric parameters that affect the rates equally in both directions. Thus, both "association rate constant" ($k_{on}$) and "dissociation rate constant" ($k_{off}$) can be determined by calculating the concentration and the actual association and dissociation rates (see Malmqvist, M.,Nature 361: 186-87 (1993)). The ratio $k_{off}/k_{on}$ is capable of eliminating all the parameters that are independent of affinity and is equal to the equilibrium dissociation constant KD (see Davies et al., (1990) Annual Rev Biochem 59:439-473). Specific binding can be measured by radioligand binding assays, surface plasmon resonance (SPR), flow cytometry binding assay or similar assays known to those skilled in the art.

The term "isolated" as used herein with respect to cells, nucleic acids, polypeptides and the like, e.g., "isolated"

DNA, RNA or polypeptides, refers to molecules that are separated from one or more other components, e.g., DNA or RNA, in the natural environment of the cell. The term "isolated" as used herein also refers to nucleic acids or peptides that are substantially free of cellular materials, viral materials or cell media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides and the like are usually prepared by at least one purification step. In one or more embodiments, the purity of the isolated nucleic acids, polypeptides and the like is at least about 50%, about 60%, about 70%, about 80%, about 90%, about 95%, about 99%, or a range between any two of these values (inclusive) or any value therein.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Amino acid" refers to an organic compound containing both an amino group and a carboxyl group, such as an α-amino acid or β-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The encoding of the same amino acid by different codons is known as "codon degeneracy". Amino acids include natural amino acids and non-natural amino acids.

As used herein, the twenty conventional amino acids and their abbreviations follow conventional usage. See Immunology-A Synthesis (second edition, Eds. E. S. Golub and D. R. Gren, Sinauer Associates, Sunderland Mass. (1991)). Stereoisomers (e.g., D-amino acids) of the twenty conventional amino acids, unnatural amino acids (such as α- or α-disubstituted amino acids), N-alkyl amino acids, lactic acid, and other unconventional amino acids can also be suitable components for the polypeptides of the present disclosure. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine and other similar amino acids and imino acids (e.g., 4-hydroxyproline). In the polypeptide notation used herein, the left-hand direction is the amino-terminal direction and the right-hand direction is the carboxy-terminal direction, in accordance with standard usage and convention. The conventional (natural) amino acids include alanine (three-letter symbol: Ala, one-letter symbol: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), valine (Val, V) etc.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a specific length of the product. Thus, included within the definition of "polypeptide" are peptides, dipeptides, tripeptides, oligopeptides, "proteins", "amino acid chains" or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage, or non-naturally occurring amino acid modification. The polypeptide may be derived from a natural biological source or produced by recombinant techniques, but it is not necessarily translated from a specified nucleic acid sequence. It may be produced in any manner, including chemical synthesis.

The term "substantially identical" when applied to polypeptides means that two peptide sequences, when optimally aligned, such as by the program GAP or BESTFIT using default GAP weights, share at least 80% sequence identity, preferably at least 90% sequence identity, more preferably at least 95% sequence identity, and most preferably at least 99% sequence identity.

A polynucleotide consists of a specific sequence of four bases: adenine (A), cytosine (C), guanine (G) and thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transfer RNA, ribosomal RNA, ribozymes, cDNA, dsRNA, siRNA, miRNA, recombinant

polynucleotides, branched polynucleotides, plasmids, vectors, DNA, RNA, and nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

A polynucleotide or a polynucleotide sequence (or a polypeptide or an antibody sequence) having a certain percentage (e.g., 90%, 95%, 98% or 99%) of "identity or sequence identity" to another sequence refers to that when the sequences are aligned, the percentage of bases (or amino acids) in the compared sequences are the same. This alignment and identity percentage or sequence identity can be determined using visual inspection or software programs known in the art, such as the software programs described in Ausubel et al. eds., (2007), Current Protocols in Molecular Biology. Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above-specified percentage identity and encoding polypeptides having identical or similar biological activity.

Minor variations in the amino acid sequences of antibodies or immunoglobulin molecules are contemplated by the present disclosure, provided that the identity of the amino acid sequences is maintained to be at least 90%, such as at least 92%, 95%, 98% or 99%. In some embodiments, the variations are conservative amino acid substitutions. Conservative amino acid substitutions are those that occur within a family of amino acids that are related in their side chains. Genetically encoded amino acids are generally classified into the following categories: (1) acidic amino acids: aspartate, and glutamate; (2) basic amino acids: lysine, arginine, and histidine; (3) non-polar amino acids: alanine, valine, leucine, isoleucine, proline, phenylalanine, methionine, and tryptophan; and (4) uncharged polar amino acids: glycine, asparagine, glutamine, cysteine, serine, threonine, and tyrosine. Amino acids of the other families include (i) serine and threonine of the aliphatic-hydroxy family; (ii) asparagine and glutamine of the amide-containing family; (iii) alanine, valine, leucine, and isoleucine of the aliphatic family; and (iv) phenylalanine, tryptophan, and tyrosine of the aromatic family. In some embodiments, conservative amino acid substitution groups are valine-leucine-isoleucine, phenylalanine-tyrosine, lysine-arginine, alanine-valine, glutamic acid-aspartic acid, and asparagine-glutamine. For example, it is reasonable to expect that the single replacement of leucine with isoleucine or valine, aspartate with glutamate, threonine with serine, or the similar replacement of an amino acid with a structurally related amino acid, will not have a major effect on the binding or properties of the resulting molecule, particularly when the replacement does not involve an amino acid within a binding site. Whether an amino acid change results in a functional peptide can be readily determined by measuring the specific activity of the polypeptide derivative. The measurement is described in detail herein. Fragments or analogs of antibodies or immunoglobulin molecules can be readily prepared by those of ordinary skill in the art.

In some embodiments, the amino acid substitutions have the following effects: (1) reducing susceptibility to proteolysis, (2) reducing susceptibility to oxidation, (3) altering the binding affinity for formation of protein complexes, (4) altering binding affinity, and (5) conferring or improving other physicochemical or functional properties of such analogs. Analogs can include various mutant proteins with sequences different from those of the naturally occurring peptide sequences. For example, single or multiple amino acid substitutions (preferably conservative amino acid substitutions) may be made in the naturally occurring sequence (preferably in a portion of the polypeptide outside the domains that form intermolecular contacts). A conservative amino acid substitution should not significantly alter the structural characteristics of the parent sequence (e.g., an amino acid for the replacement should not tend to disrupt a helix that occurs in the parent sequence, or disrupt other types of secondary structures that characterize the parent sequence). Examples of secondary and tertiary structures of artificially recognized polypeptides are described in Proteins: Structures and Molecular Principles (Ed. Creighton, W. H. Freeman and Company, New York (1984)); Introduction to Protein Structure (Eds. C. Branden and J. Tooze, Garland Publishing, New York, N.Y. (1991)); and Thornton et al., Nature 354:105 (1991).

The number of amino acids of conservative amino acid substitutions of VL or VH may be about 1, about 2, about 3, about 4, about 5, about 6, about 8, about 9, about 10, about 11, about 13, about 14, about 15, or a range between any two of these values (inclusive) or any value therein. The number of amino acids of conservative amino acid substitutions of a heavy chain constant region, a light chain constant region, a heavy chain or a light chain may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

The term "agent" as used herein means a chemical compound, a mixture of chemical compounds, a biological macromolecule, or an extract made from biomaterial.

As used herein, the term "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., $^{3}H$, $^{14}C$, $^{15}N$, $^{35}S$, $^{90}Y$, $^{99}Tc$, $^{111}In$, $^{125}I$ and $^{131}I$), fluorescent labels (e.g., FITC, rhodamine and lanthanide phosphor), enzymatic labels (e.g., horseradish peroxidase, β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In some embodiments, the labels are connected by spacer arms of various lengths to reduce potential steric hindrance. The term "medicament" or "drug" refers to a compound or composition that is capable of inducing the desired therapeutic effect when properly administered to a patient.

"About" refers to a general error range for corresponding values as readily understood by those skilled in the relevant art. In some embodiments, "about" mentioned herein refers to the values described and ranges thereof of $\pm 10\%$, $\pm 5\%$ or $\pm 1\%$.

"$EC_{50}$", i.e., concentration for 50% of maximal effect, refers to a concentration that causes 50% of maximal effect.

"$IC_{50}$" represents 50% inhibitory concentration, i.e., a concentration of drug or inhibitor required to inhibit half of a given biological process.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the pharmaceutical composition disclosed herein for detection, diagnostic procedures, and/or treatment.

The term "cancer" means or is intended to describe the physiological state of a mammal, which is typically characterized by uncontrolled cell growth. Examples of cancers include, but are not limited to, carcinoma, lymphoma, blastoma, sarcoma or leukemia. More specific examples of such cancers include, but are not limited to, colorectal cancer, lung cancer, ovarian cancer, uterine cancer, endometrial cancer, salivary gland cancer, peritoneal cancer, fallopian tube cancer, pancreatic cancer, thyroid cancer, head and neck squamous cell carcinoma, nasopharyngeal cancer, laryngeal cancer, lung adenocarcinoma, lung squamous cell carcinoma, liver cancer, hepatocellular carcinoma, gastrointestinal cancer, glioblastoma, breast cancer, brain cancer, renal cancer, renal cell carcinoma, colon cancer, rectal cancer, prostate cancer, vulval cancer, testicular cancer, squamous cell carcinoma, small cell lung cancer, cervical cancer, bladder cancer, retinoblastoma, glioblastoma, mesothelioma, oral epithelioid cancer, choriocarcinoma and head and neck cancer.

The term "overexpression" or "overexpressed" interchangeably refers to a gene that is transcribed or translated at a detectably greater level, usually in certain cells, e.g., a cancer cell, in comparison to a normal cell. Overexpression may be overexpression of a protein and RNA (due to increased transcription, post transcriptional processing, translation, post translational processing, altered stability, and altered protein degradation), as well as local overexpression due to altered protein traffic patterns (increased nuclear localization), and augmented functional activity, e.g., increased enzyme hydrolysis of substrate. Overexpression may be by 1%, 5%, 10%, 20%, 30%, 50%, 60%, 70%, 80%, 90% or more in comparison to a normal cell or control cell. In some embodiments, the anti-Nectin-4 antibody and the antibody-drug conjugate of the present invention is used for treating a solid tumor expressing or overexpressing Nectin-4.

As used herein, the term "tumor overexpressing Nectin-4" refers to a tumor that overexpresses Nectin-4 (including benign tumors and cancers). In some embodiments, Nectin-4 expression in a tumor sample above the immunohistochemical background level (e.g., as determined by immunohistochemical staining) indicates that the tumor is a tumor overexpressing Nectin-4. Methods for detecting the expression of Nectin-4 in a tumor are known in the art, such as immunohistochemical assays.

As used herein, the terms "give" and "administer" are used interchangeably and refer to the delivery of a substance (e.g., anti-Nectin-4 antibody or ADC) for therapeutic purposes (e.g., treating a disease associated with the expression or overexpression of Nectin-4). The mode of administration may be parenteral, enteral, and topical. Parenteral administration is typically performed by injection, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, and intrasternal injection and infusion.

As used herein, the term "effective amount" or "therapeutically effective amount" refers to the amount of a drug, e.g., an antibody or ADC, that is sufficient to reduce or ameliorate the severity and/or duration of a disorder (e.g., cancer) or one or more symptoms thereof, prevent the progression of a disorder, cause regression of a disorder, prevent the

recurrence, development, onset or progression of one or more symptoms associated with a disorder, detect a disorder, or enhance or improve the prophylactic or therapeutic effect of another therapy (e.g., a prophylactic or therapeutic agent). For example, the effective amount of an antibody may inhibit tumor growth (e.g., inhibit an increase in tumor volume), decrease tumor growth (e.g., decrease tumor volume), reduce the number of cancer cells, and/or relieve to some extent one or more of the symptoms associated with the cancer. For example, the effective amount may improve disease free survival (DFS), improve overall survival (OS), or decrease likelihood of recurrence.

The terms "patient" and "subject" are used interchangeably and refer to any mammal in need of diagnosis, prognosis or treatment, including, but not limited to, humans, dogs, cats, guinea pigs, rabbits, rats, mice, horses, cattle, and the like.

As used herein, the term "in need" means that the patient has been identified as in need of a particular method or treatment. In some embodiments, identification may be made by any diagnostic mode. The patient may be in need of any methods and treatments described herein.

The term "drug for treating a tumor" as used herein refers to an agent having the functional property of inhibiting in a human the development or progression of a tumor, particularly a malignant (cancerous) lesion, such as cancer, sarcoma, lymphoma or leukemia. Inhibition of metastasis is a property of antineoplastic drugs in many cases.

The term "antibody-drug conjugate" or "ADC" refers to a binding protein (e.g., an antibody or an antigen-binding unit) that is linked to one or more chemical drugs, which may optionally be a therapeutic agent or a cytotoxic agent. In a preferred embodiment, the ADC comprises an antibody, a drug (e.g., a cytotoxic drug), and a linker capable of attaching or conjugating the drug to the antibody. Non-limiting examples of drugs that can be included in the ADC are a mitotic inhibitor, an anti-tumor antibiotic, an immunomodulator, a vector for gene therapy, an alkylating agent, an anti-angiogenic agent, an antimetabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor (e.g., a TEC-family kinase inhibitor and a serine/threonine kinase inhibitor) and a radiosensitizer.

The terms "antibody-drug conjugate" and "ADC" are used interchangeably. The terms "anti-Nectin-4 antibody-drug conjugate" and "anti-Nectin-4 ADC" are used interchangeably to refer to an ADC comprising an antibody that specifically binds to Nectin-4, wherein the antibody is conjugated to one or more drugs. In one or more embodiments, the anti-Nectin-4 ADC comprises an antibody conjugated to exatecan. In one or more embodiments, the anti-Nectin-4 antibody or ADC binds to Nectin-4 (e.g., human Nectin-4).

The term "drug to antibody ratio" or "DAR" refers to the number of drugs (e.g., exatecan) that are attached to the antibody in the ADC. The DAR of an ADC may be in the range of 1 to 10, but a higher load (e.g., 20) is also possible depending on the number of connection sites on the antibody. The term DAR may be used when referring to the number of drugs loaded onto a single antibody, or alternatively, when referring to an average or mean DAR for a set of ADCs. In some embodiments, the value is selected from 1, 2, 3, 4, 5, 6, 7, 8, 9, or 10. In considering the mean binding number of small molecule drugs, i.e., the mean drug binding number of an antibody, or the mean drug to antibody ratio, the value is selected from about 0 to about 10, or about 2 to about 8. In some embodiments, the drug to antibody ratio is about 3 to about 6. In other embodiments, the drug to antibody ratio is about 6 to about 8, or about 7 to about 8. DAR values may be denoted herein by p. The DAR value of ADC can be measured by ultraviolet-visible absorption spectroscopy (UV-Vis), high performance liquid chromatography-hydrophobic interaction chromatography (HPLC-HIC), reversed-phase high performance liquid chromatography (RP-HPLC), liquid chromatography-mass spectrometry (LC-MS), etc. These techniques are described in Ouyang, J. Methods Mol Biol, 2013, 1045: p. 275-83.

Various substituents are defined below. In some cases, the number of carbon atoms in a substituent (e.g., alkyl, alkenyl, alkynyl, alkoxy, aminoalkoxy, aminoalkyl, aminoalkylamino, alkylamino, heterocyclyl, heterocyclylamino and aryl) is indicated by the prefix "Cx-Cy" or "Cx-y", wherein x is the minimum number and y is the maximum number of carbon atoms. Thus, for example, "C1-C6 alkyl" refers to an alkyl containing 1 to 6 carbon atoms. If a substituent is described as "substituted with... ", a hydrogen atom on a carbon or nitrogen is substituted with a non-hydrogen group. For example, a substituted alkyl substituent is an alkyl substituent in which at least one hydrogen atom on the alkyl is substituted with a non-hydrogen group. To illustrate, monofluoroalkyl is an alkyl substituted with a fluoro group, and difluoroalkyl is an alkyl substituted with two fluoro groups. It should be recognized that if there is more than one substitution on a substituent, each substitution may be identical or different (unless otherwise stated). If a substituent is described as "optionally substituted with...", the substituent may be (1) unsubstituted or (2) substituted. Possible substituents include, but are not limited to, hydroxy, C1-C6 alkyl, C2-C6 alkenyl, C2-C6 alkynyl, C1-C6 alkoxy, C1-C6 aminoalkoxy, halogen, nitro, cyano, sulfhydryl, alkylthio, amino, C1-C6 aminoalkyl, C1-C6 aminoalkylamino, C1-C6 alkyl linking to a heterocycle, C1-C6 alkylamino linking to a heterocycle, heterocyclyl, amino-substituted heterocyclyl, heterocyclylamino, carbamoyl, morpholin-1-yl, piperidin-1-yl, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or -

$(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10.

The "alkyl" refers to a saturated aliphatic hydrocarbyl group, and this term includes linear and branched hydrocarbyl groups. For example, C1-C20 alkyl, such as C1-C6 alkyl. C1-C20 alkyl refers to an alkyl group containing 1 to 20 carbon atoms, e.g., an alkyl group containing 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, *n*-propyl, isopropyl, *n*-butyl, isobutyl, *sec*-butyl, *tert*-butyl, *n*-pentyl, neopentyl, *n*-hexyl, and the like. The alkyl may be unsubstituted or substituted with one or more substituents including, but not limited to, alkyl, alkoxy, cyano, hydroxy, carbonyl, carboxy, aryl, heteroaryl, amino, halogen, sulfonyl, sulfinyl, phosphonyl, and the like.

The "alkoxy" refers to a group formed by replacing at least one carbon atom in the alkyl group with an oxygen atom. Non-limiting examples of alkoxy are methoxy, ethoxy, *n*-propoxy, 1-methylethoxy (isopropoxy), *n*-butoxy, isobutoxy, *sec*-butoxy, and *tert*-butoxy. Alkoxy may be substituted or unsubstituted. The definition of alkyl is the same as the definition of "alkyl" mentioned above.

"Alkenyl" refers to a linear or branched unsaturated aliphatic hydrocarbon group containing 1 to 10 (e.g., 1, 2, 3, 4, 5, 6, 7, 8, 9 or 10) carbon-carbon double bonds and consisting of 2 to 20 carbon atoms, preferably an alkenyl group consisting of 2 to 12 (e.g., 2, 3, 4, 5, 6, 7, 8, 9, 10, 11 or 12) carbon atoms, more preferably an alkenyl group consisting of 2 to 8 carbon atoms, and further preferably an alkenyl group consisting of 2 to 6 carbon atoms. Non-limiting examples include vinyl, propen-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl, and undecen-3-yl. The alkenyl may be optionally further substituted with 1 or more substituents.

"Alkynyl" refers to a linear or branched unsaturated aliphatic hydrocarbon group containing 1 to 10 (e.g., 1, 2, 3, 4, 5, 6, 7, 8, 9 or 10) carbon-carbon triple bonds and consisting of 2 to 20 carbon atoms, preferably an alkynyl group consisting of 2 to 12 (e.g., 2, 3, 4, 5, 6, 7, 8, 9, 10, 11 or 12) carbon atoms, more preferably an alkynyl group consisting of 2 to 8 carbon atoms, and further preferably an alkynyl group consisting of 2 to 6 carbon atoms. Non-limiting examples include ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyn-3-yl, nonyn-3-yl, decyn-4-yl, undec-3-yl, and dodecyn-4-yl. The alkynyl may be optionally further substituted with one or more substituents.

The "carbocyclyl" refers to a stable non-aromatic monocyclic or polycyclic hydrocarbyl radical consisting of carbon and hydrogen atoms only, and may comprise a fused or bridged ring system, contains 3 to 15 carbon atoms, for example, 3 to 10 (e.g., 3, 4, 5, 6, 7, 8, 9 or 10) carbon atoms. It is saturated or unsaturated, and links to the remainder of the molecule through a single bond. Monocyclic radicals include, for example, cyclopropyl, cyclobutyl, cyclopentyl, cyclohexyl, cycloheptyl and cyclooctyl. Polycyclic radicals include, for example, adamantyl, norbornyl, and decahydronaphthyl. When specifically stated in the specification, the carbocyclyl may be optionally substituted with one or more substituents independently selected from the following: alkyl, halogen, haloalkyl, cyano, nitro, oxo, aryl, aralkyl, carbocyclyl, carbocyclylalkyl, heterocyclyl, heterocyclylalkyl, heteroaryl and heteroarylalkyl.

The "aryl" refers to an all-carbon monocyclic or all-carbon fused ring having a completely conjugated $\pi$-electron system, and typically has 5-14 carbon atoms, e.g., 6, 10, 12, or 14 carbon atoms. Aryl may be unsubstituted or substituted with one or more substituents including, but not limited to, alkyl, alkoxy, cyano, hydroxy, carboxy, aryl, aralkyl, amino, halogen, sulfonyl, sulfinyl, phosphonyl. Examples of unsubstituted aryl include, but are not limited to, phenyl, naphthyl, and anthracenyl.

"Heteroaryl" refers to a substituted or unsubstituted aromatic ring. It may be a 3-8 membered (e.g., 3, 4, 5, 6, 7 or 8 membered) monocyclic ring system, a 5-12 membered (e.g., 5, 6, 7, 8, 9, 10, 11 or 12 membered) bicyclic ring system or a 10-15 membered (e.g., 10, 11, 12, 13, 14 or 15 membered) tricyclic ring system, and it contains 1 to 6 (e.g., 1, 2, 3, 4, 5 or 6) heteroatoms selected from N, O and S, and is preferably 5-8 membered heteroaryl. 1 to 4 (e.g., 1, 2, 3 or 4) N and S optionally substituted in the ring of the heteroaryl can be oxidized to various oxidation states. Heteroaryl may be attached to a heteroatom or carbon atom and it may be a bridged ring or a spiro ring. Non-limiting examples include cyclic pyridinyl, furanyl, thienyl, pyranyl, pyrrolyl, pyrimidinyl, pyrazinyl, pyridazinyl, imidazolyl, piperidinyl, benzimidazolyl, benzopyridinyl and pyrrolopyridinyl. Heteroaryl is optionally further substituted with one or more substituents.

"Cycloalkyl" refers to a saturated cyclic hydrocarbon group, the ring of which may be a 3-10 membered (e.g., 3, 4, 5, 6, 7, 8, 9 or 10 membered) monocyclic ring system, a 4-12 membered (e.g., 4, 5, 6, 7, 8, 9, 10, 11 or 12 membered) bicyclic ring system or a 10-20 membered (e.g., 10, 11, 12, 13, 14, 15, 16, 17, 18, 19 or 20 membered) polycyclic ring system. The ring carbon atoms are preferably 3 to 10 carbon atoms, further preferably 3 to 8 carbon atoms. Non-limiting examples of "cycloalkyl" include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl, cycloheptatrienyl, and the like. When the cycloalkyl is substituted, it may be optionally further substituted with one or more substituents.

"Heterocycloalkyl" refers to a substituted or unsubstituted saturated non-aromatic cyclic group. It may be a 3-8

membered (e.g., 3, 4, 5, 6, 7 or 8 membered) monocyclic ring system, a 4-12 membered (e.g., 4, 5, 6, 7, 8, 9, 10, 11 or 12 membered) bicyclic ring system or a 10-15 membered (e.g., 10, 11, 12, 13, 14 or 15 membered) tricyclic ring system, and it contains 1, 2 or 3 heteroatoms selected from N, O and S, and is preferably 3-8 membered heterocyclyl. 1, 2 or 3 N and S optionally substituted in the ring of "heterocycloalkyl" can be oxidized to various oxidation states; "heterocycloalkyl" may be attached to a heteroatom or a carbon atom and may be a bridged ring or a spiro ring. Non-limiting examples of "heterocycloalkyl" include epoxyethyl, aziridinyl, oxetanyl, azetidinyl, 1,3-dioxolanyl, 1,4-dioxolanyl, 1,3-dioxanyl, azepanyl, piperidinyl, morpholinyl, thiomorpholinyl, 1,3-dithianyl, tetrahydrofuranyl, tetrahydropyrrolyl, tetrahydroimidazolyl, tetrahydrothiazolyl, tetrahydropyranyl, azabicyclo[3.2.1]octyl, azabicyclo[5.2.0]nonyl, oxatricyclo[5.3.1.1]dodecyl, aza-adamantyl and oxaspiro[3.3]heptyl.

The "heterocyclyl" refers to a stable 3 to 18 membered aromatic or nonaromatic ring group consisting of 2 to 8 (e.g., 2, 3, 4, 5, 6, 7 or 8) carbon atoms and 1 to 6 (1, 2, 3, 4, 5 or 6) heteroatoms selected from nitrogen, oxygen and sulfur. Unless otherwise specifically stated in the specification, heterocyclyl may be a monocyclic, bicyclic, tricyclic or tetracyclic ring system, and may include fused or bridged ring systems; and the nitrogen, carbon or sulfur atoms in heterocyclyl may be optionally oxidized; the nitrogen atoms are optionally quaternized; and heterocyclyl may be partially or fully saturated. Examples of such heterocyclyl include, but are not limited to, dioxolanyl, dioxinyl, thienyl[1,3]dithianyl, decahydroisoquinolinyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidinonyl, pyrrolidinyl, pyrazolidinyl, quinuclidinyl, thiazolidinyl, 1,2,4-thiadiazol-5(4H)-ylidene, tetrahydrofuranyl, trioxacyclohexyl, trithianyl, triazinanyl, tetrahydropyranyl, thiomorpholinyl, thiamorpholinyl, 1-oxo-thiomorpholinyl and 1,1-dioxo-thiomorpholinyl. When specifically stated in the specification, heterocyclyl may be optionally substituted with one or more substituents selected from: alkyl, alkenyl, halogen, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, and optionally substituted heteroarylalkyl.

The "alkoxy" refers to formula -O-(alkyl), wherein alkyl is the alkyl defined herein. Non-limiting examples of alkoxy are methoxy, ethoxy, *n*-propoxy, 1-methylethoxy (isopropoxy), *n*-butoxy, isobutoxy, *sec*-butoxy, and *tert*-butoxy. Alkoxy may be substituted or unsubstituted.

The "halogen" refers to fluoro (F), chloro (Cl), bromo (Br), or iodo (I).

The "amino" refers to $-NH_2$.

The "cyano" refers to -CN.

The "nitro" refers to $-NO_2$.

The "hydroxy" refers to -OH.

The "carboxyl" refers to -COOH.

The "thiol" refers to -SH.

The "carbonyl" refers to C=O.

When the "alkyl", "alkoxy", "alkenyl", "alkynyl", "aryl", "heteroaryl", "carbocyclyl", "carbocycle", "heterocyclyl", "heterocycle", "cycloalkyl", "heterocycloalkyl", or "heterocyclyl" described above is substituted, it may be further substituted with 0, 1, 2, 3, 4, 5, 6, 7, 8, 9, or 10 substituents selected from F, Cl, Br, I, hydroxy, sulfhydryl, nitro, cyano, amino, $C_{1-6}$ alkylamino, =O, $C_{1-6}$ alkyl, $C_{1-6}$ alkoxy, $C_{2-6}$ alkenyl, $C_{2-6}$ alkynyl, $-NR_{q4}R_{q5}$, $=NR_{q6}$, $-C(=O)OC_{1-6}$ alkyl, $-OC(=O)C_{1-6}$ alkyl, $-C(=O)NR_{q4}R_{q5}$, $C_{3-8}$ cycloalkyl, $C_{3-8}$ heterocycloalkyl, $C_{6-10}$ aryl, $C_{5-10}$ heteroaryl, $-C(=O)OC_{6-10}$ aryl, $-OC(=O)C_{6-10}$ aryl, $-OC(=O)C_{5-10}$ heteroaryl, $-C(=O)OC_{5-10}$ heteroaryl, $-OC(=O)C_{3-8}$ heterocycloalkyl, $-C(=O)OC_{3-8}$ heterocycloalkyl, $-OC(=O)C_{3-8}$ cycloalkyl, $-C(=O)OC_{3-8}$ cycloalkyl, $-NHC(=O)C_{3-8}$ heterocycloalkyl, $-NHC(=O)C_{6-10}$ aryl, $-NHC(=O)C_{5-10}$ heteroaryl, $-NHC(=O)C_{3-8}$ cycloalkyl, $-NHC(=O)C_{3-8}$ heterocycloalkyl, $-NHC(=O)C_{2-6}$ alkenyl, and $-NHC(=O)C_{2-6}$ alkynyl, wherein the substituent $C_{1-6}$ alkyl, $C_{1-6}$ alkoxy, $C_{2-6}$ alkenyl, $C_{2-6}$ alkynyl, $C_{3-8}$ cycloalkyl, $C_{3-8}$ heterocycloalkyl, $C_{6-10}$ aryl, $C_{5-10}$ heteroaryl, $-NHC(=O)C_{6-10}$ aryl, $-NHC(=O)C_{5-10}$ heteroaryl, $-NHC(=O)C_{3-8}$ heterocycloalkyl, or $-NHC(=O)C_{3-8}$ cycloalkyl is optionally further substituted with 1 to 3 substituents selected from OH, F, Cl, Br, I, $C_{1-6}$ alkyl, $C_{1-6}$ alkoxy, $-NR_{q4}R_{q5}$, and =O; $R_{q1}$ is selected from $C_{1-6}$ alkyl, $C_{1-6}$ alkoxy, and $C_{6-10}$ aryl; $R_{q2}$ and $R_{q3}$ are selected from H and $C_{1-6}$ alkyl, wherein $R_{q4}$ and $R_{q5}$ are selected from H, $C_{1-6}$ alkyl, $-NH(C=NR_{q1})NR_{q2}R_{q3}$, $-S(=O)_2NR_{q2}R_{q3}$, $-C(=O)R_{q1}$, and $-C(=O)NR_{q2}R_{q3}$, wherein $C_{1-6}$ alkyl is optionally further substituted with 1 or more substituents selected from OH, F, Cl, Br, I, $C_{1-6}$ alkyl, $C_{1-6}$ alkoxy, $C_{6-10}$ aryl, $C_{5-10}$ heteroaryl, $C_{3-8}$ cycloalkyl, and $C_{3-8}$ heterocycloalkyl; or $R_{q4}$ and $R_{q5}$, together with the N atom, form a 3- to 8-membered heterocyclyl, wherein the heterocyclyl may contain 1 or more heteroatoms selected from N, O, and S.

The "pharmaceutically acceptable salt" or "pharmaceutically acceptable salt thereof" refers to a salt that maintains the bioavailability and properties of the free acid or free base form of a compound, and is obtained by reacting the free acid with a non-toxic inorganic or organic base, or the free base with a non-toxic inorganic or organic acid.

The "pharmaceutical composition" refers to a mixture of one or more compounds or pharmaceutically acceptable salts or prodrugs thereof, and an additional chemical component, wherein the "additional chemical component" refers to a pharmaceutically acceptable carrier or excipient and/or one or more additional therapeutic agents.

The "carrier" refers to a material that does not cause significant irritation to living organisms and does not

eliminate the biological activity and characteristics of the compound administered, i.e., a diluent, adjuvant, excipient, or carrier that can be administered to a patient along with the active ingredient. Such pharmaceutical carriers can be sterile liquids, such as water and oils, including oils originating from petroleum, animal, plant or synthesis, such as peanut oil, soybean oil, mineral oil, sesame oil, etc. Water is a preferred carrier when the pharmaceutical composition is administered intravenously. A saline aqueous solution, a glucose aqueous solution, and a glycerol solution can also be used as a liquid carrier, especially for injection. "Excipient" refers to an inert substance added to a pharmaceutical composition to facilitate administration of a compound. Non-limiting examples include calcium carbonate, calcium phosphate, starches, cellulose derivatives (including microcrystalline cellulose), gelatin, vegetable oils, polyethylene glycols, diluents, granulating agents, lubricants, binders and disintegrants. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or an antigen-binding fragment, preferably in purified form, together with an appropriate amount of a carrier, to provide a form suitable for administration to a patient. The formulation should be suitable for the administration mode. The formulation may be encapsulated in an ampoule bottle, a disposable syringe, or a multi-dose vial made of glass or plastic.

The "stereoisomer" refers to an isomer that results from different spatial arrangements of atoms within a molecule, including a cis-trans isomer, an enantiomer, and a conformational isomer.

The "optional", "optionally", "selective", or "selectively" means that the subsequent event or condition may, but does not necessarily, occur. This description encompasses instances where the event or condition occurs and instances where it does not. For example, "a heterocyclic group that is selectively substituted with an alkyl group" means that the alkyl group may be, but is not necessarily, present. This description encompasses instances where the heterocyclic group is substituted with an alkyl group and instances where the heterocyclic group is not substituted with an alkyl group.

Anti-Nectin-4 Antibody

The present disclosure provides an antibody or an antigen-binding unit. In one or more embodiments, the antibody or the antigen-binding unit of the present invention is capable of specifically binding to Nectin-4 (e.g., human Nectin-4). In one or more embodiments, the antibody or the antigen-binding unit of the present invention has one or more of the following properties: binding to Nectin-4 (e.g., human Nectin-4) *in vitro,* binding to cells expressing Nectin-4, high affinity, and endocytosis.

In one or more embodiments, the antigen-binding unit of the antibody is Fab, Fab', $F(ab')_2$, Fv, disulfide-stabilized Fv, scFv, or single domain antibody.

In one or more embodiments, the antibody may be a monoclonal antibody.

The binding specificity of the antibody or the antigen-binding unit disclosed herein can be detected by *in-vitro* assays, such as co-immunoprecipitation, radioimmunoassay (RIA), surface plasmon resonance, flow cytometry (Facs), or enzyme-linked immunosorbent assay (ELISA).

The present invention further encompasses an antibody that binds to the same epitope as the antibody disclosed herein. For example, the antibody of the present invention specifically binds to an epitope comprising one or more amino acid residues on human Nectin-4.

In one or more embodiments, the antibody comprises a heavy chain constant region, such as an IgG, IgA, IgE, IgM, or IgD constant region. In one or more embodiments, the antibody or the antigen-binding unit comprises an immunoglobulin heavy chain constant domain selected from a human IgG constant domain, a human IgA constant domain, a human IgE constant domain, a human IgM constant domain, and a human IgD constant domain. In one or more embodiments, the antibody or the antigen-binding unit comprises an IgG1 heavy chain constant region, an IgG2 heavy chain constant region, an IgG3 heavy chain constant region, or an IgG4 heavy chain constant region. In one or more embodiments, the heavy chain constant region is an IgG1 heavy chain constant region or an IgG4 heavy chain constant region. In one or more embodiments, the antibody or the antigen-binding unit comprises a light chain constant region, e.g., a $\kappa$ light chain constant region or a $\lambda$ light chain constant region.

In one or more embodiments, the antibody or the antigen-binding unit of the present invention is connected to a detectable reagent, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., $^{3}H$, $^{14}C$, $^{15}N$, $^{35}S$, $^{90}Y$, $^{99}Tc$, $^{111}In$, $^{125}I$ and $^{131}I$), fluorescent labels (e.g., FITC, rhodamine and lanthanide phosphor), enzymatic labels (e.g., horseradish peroxidase,

β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In one or more embodiments, the labels are connected by spacer arms of various lengths to reduce potential steric hindrance.

In one or more embodiments, the antibody or the antigen-binding unit of the present invention can be used to detect whether Nectin-4 (e.g., human Nectin-4) is present in a sample or not. In one or more embodiments, the antibody comprises a detectable agent. The antibody is a polyclonal antibody, or more preferably, a monoclonal antibody. An intact antibody or antigen-binding unit (e.g., Fab, scFv, or $F(ab')_2$) can be used. The detection method can be used to detect an analyte mRNA, protein, or genomic DNA in a biological sample *in vitro* and *in vivo.* For example, *in-vitro* techniques for the detection of an analyte mRNA include Northern hybridization and *in situ* hybridization. *In-vitro* techniques for the detection of an analyte protein include enzyme-linked immunosorbent assay (ELISA), Western blots, immunoprecipitation, and immunofluorescence. *In-vitro* techniques for the detection of an analyte genomic DNA include Southern hybridization. In addition, *in-vivo* techniques for the detection of an analyte protein include introducing a labeled anti-analyte protein antibody into a patient. For example, the antibody can be labeled with a radioactive marker, the presence and location of which in the patient can then be detected by standard imaging techniques.

In one or more embodiments, Nectin-4 may be detected in a biological sample as part of a clinical testing procedure, e.g., to determine the efficacy of a given treatment regimen. Conjugating (e.g., physically linking) the antibody to a detectable substance may facilitate the detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, or phycoerythrin; one example of the luminescent material includes luminol; examples of bioluminescent materials include luciferase, luciferin, and aequorin; examples of suitable radioactive materials include $^{125}I$, $^{131}I$, $^{35}S$, or$^{3}H$.

In one or more embodiments, the antibody or the antigen-binding unit of the present invention comprises VH CDR1 set forth in SEQ ID NO: 2, a VH CDR2 set forth in SEQ ID NO: 3, and a VH CDR3 set forth in SEQ ID NO: 4.

In one or more embodiments, the antibody or the antigen-binding unit of the present invention comprises a VL CDR1 set forth in SEQ ID NO: 5, a VL CDR2 set forth in SEQ ID NO: 6, and a VL CDR3 set forth in SEQ ID NO: 7.

In one or more embodiments, the antibody or the antigen-binding unit of the present invention comprises VH CDR1 set forth in SEQ ID NO: 2, a VH CDR2 set forth in SEQ ID NO: 3, a VH CDR3 set forth in SEQ ID NO: 4, a VL CDR1 set forth in SEQ ID NO: 5, a VL CDR2 set forth in SEQ ID NO: 6, and a VL CDR3 set forth in SEQ ID NO: 7.

In one or more embodiments, the antibody or the antigen-binding unit of the present invention comprises a heavy chain variable region comprising the amino acid sequence set forth in SEQ ID NO: 8, or an amino acid sequence having appropriate sequence identity to the amino acid sequence set forth in SEQ ID NO: 8, such as at least 80% sequence identity, or at least 90% sequence identity, or at least 95% sequence identity, or at least 99% sequence identity; and/or a light chain variable region comprising the amino acid sequence set forth in SEQ ID NO: 10, or an amino acid sequence having appropriate sequence identity to the amino acid sequence set forth in SEQ ID NO: 10, such as at least 80% sequence identity, or at least 90% sequence identity, or at least 95% sequence identity, or at least 99% sequence identity. In one or more embodiments, the amino acid sequences with sequence identity are at least CDR invariant.

In one or more embodiments, the antibody or the antigen-binding unit of the present invention comprises a heavy chain variable region set forth in SEQ ID NO: 8 and a light chain variable region set forth in SEQ ID NO: 10.

In one or more embodiments, the antibody or the antigen-binding unit of the present invention further comprises a heavy chain constant region comprising the amino acid sequence set forth in SEQ ID NO: 9, or an amino acid sequence having appropriate sequence identity to the amino acid sequence set forth in SEQ ID NO: 9, such as at least 80% sequence identity, or at least 90% sequence identity, or at least 95% sequence identity, or at least 99% sequence identity.

In one or more embodiments, the antibody or the antigen-binding unit of the present invention further comprises a light chain constant region comprising the amino acid sequence set forth in SEQ ID NO: 11, or an amino acid sequence having appropriate sequence identity to the amino acid sequence set forth in SEQ ID NO: 11, such as at least 80% sequence identity, or at least 90% sequence identity, or at least 95% sequence identity, or at least 99% sequence identity.

In one or more embodiments, the heavy chain of the antibody of the present invention comprises the amino acid sequence set forth in SEQ ID NO: 12, or an amino acid sequence having appropriate sequence identity to the amino acid sequence set forth in SEQ ID NO: 12, such as at least 80% sequence identity, or at least 90% sequence identity, or at least 95% sequence identity, or at least 99% sequence identity.

In one or more embodiments, the light chain of the antibody of the present invention comprises the amino acid sequence set forth in SEQ ID NO: 14, or an amino acid sequence having appropriate sequence identity to the amino acid sequence set forth in SEQ ID NO: 14, such as at least 80% sequence identity, or at least 90% sequence identity, or at least 95% sequence identity, or at least 99% sequence identity.

In one or more embodiments, the heavy chain or the fragment thereof of the antibody or the antigen-binding unit of the present invention further comprises a signal peptide, such as MELGLCWVFLVAILEGVQC (SEQ ID NO: 18), of which the DNA sequence is atggagctgggcctgtgttgggtgtttctggtggccatcctggagggcgtgcagtgc (SEQ ID NO: 19).

In one or more embodiments, the light chain or the fragment thereof of the antibody or the antigen-binding unit of the present invention further comprises a signal peptide, such as MDMRVPAQLLGLLLLWFPGSRC (SEQ ID NO: 20), of which the DNA sequence is atggacatgagagtgcctgcccagctgctgggcctgctgctgctgtggttccctggcagcagatgc (SEQ ID NO: 16).

In one or more embodiments, the antibody or the antigen-binding unit of the present invention binds to Nectin-4 with an equilibrium dissociation constant (KD) of about 1 μM or less. In one or more embodiments, the antibody or the antigen-binding unit of the present invention binds to Nectin-4 with a KD of about 100 nM to about 1 pM or less. In one or more embodiments, the antibody or the antigen-binding unit of the present invention binds to Nectin-4 with a KD of about 10 nM to about 1 pM or less. In one or more embodiments, the antibody or the antigen-binding unit of the present invention binds to Nectin-4 with a KD of about 10 nM to about 1 nM or less.

In one or more embodiments, the antibody or the antigen-binding unit of the present invention binds to human Nectin-4 (e.g., the antigen Nectin-4-His set forth in SEQ ID NO: 1) with a KD of about 100 nM to about 1 pM or less. In one or more embodiments, the antibody or the antigen-binding unit of the present invention binds to human Nectin-4 with a KD of about 10 nM to about 1 pM or less; or about 10 nM to about 1 nM or less.

Antibody-Drug Conjugate

The antibody or the antigen-binding unit of the present invention can be conjugated to a drug to form an anti-Nectin-4 antibody-drug conjugate (anti-Nectin-4 ADC). Antibody-drug conjugates (ADCs) can increase the therapeutic potency of antibodies in treating a disease (e.g., a cancer) due to their ability to selectively deliver one or more drugs to the target tissue (e.g., a tumor expressing or overexpressing Nectin-4). In one or more embodiments, the antibody-drug conjugate (ADC) of the present invention comprises the anti-Nectin-4 antibody or the antigen-binding unit described herein and at least one drug (e.g., exatecan). The ADC of the present invention has one or more of the following properties: binding to Nectin-4 (e.g., human Nectin-4) *in vitro,* binding to cells expressing Nectin-4, high affinity, endocytosis, and reducing or inhibiting the growth of cancer cells or tumors.

The antigen-binding unit of the present invention may be conjugated to the drug described herein. In one or more embodiments, the antigen-binding unit described herein is conjugated to a drug via a linker to form the anti-Nectin-4 ADC.

The anti-Nectin-4 ADC of the present invention comprises an antibody or an antigen-binding unit that is linked to one or more drugs and specifically binds to Nectin-4 (e.g., human Nectin-4). The specificity of the ADC may be determined by the specificity of the antibody (e.g., anti-Nectin-4 antibody) or the antigen-binding unit. In one or more embodiments, the anti-Nectin-4 antibody is linked to one or more drugs (e.g., DNA topoisomerase inhibitors) that are delivered to cells expressing or overexpressing Nectin-4, particularly cancer cells expressing or overexpressing Nectin-4. In one or more embodiments, the anti-Nectin-4 antibody-drug conjugate comprises an anti-Nectin-4 antibody conjugated to a drug (e.g., exatecan) via a linker. The anti-Nectin-4 antibody or the antigen-binding unit described herein provides the ADC with the ability to bind to Nectin-4, such that the drug attached to the antibody can be delivered to cells expressing or overexpressing Nectin-4, particularly cancer cells expressing or overexpressing Nectin-4.

In one or more embodiments, the ADC has a structure of Formula I-A or a stereoisomer thereof, or a pharmaceutically acceptable salt or solvate thereof,

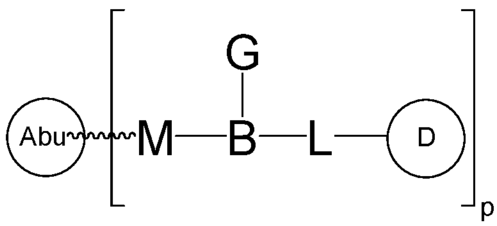

Formula I-A

wherein Abu, M, B, G, L, D, and p are as defined herein.

In one or more embodiments, Abu is antibody ASG-AM.

In one or more embodiments, the ADC is ASG-AM-ExaD8 or ASG-AM-ExaD4, or a pharmaceutically acceptable salt or solvate thereof.

In one or more embodiments, the ADC has a structure of Formula I-B or a stereoisomer thereof, or a pharmaceutically acceptable salt or solvate thereof,

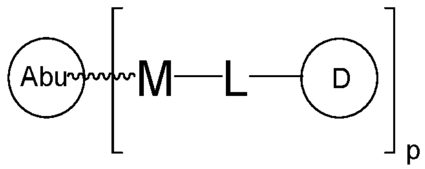

Formula I-B

wherein Abu, M, L, D, and p are as defined herein.

In one or more embodiments, Abu is antibody ASG-22 or ASG-AM.

In one or more embodiments, the ADC is ASG-22-MMAE or ASG-AM-MMAE, or a pharmaceutically acceptable salt or solvate thereof.

In one or more embodiments, the ADC has a structure of Formula I-C or a stereoisomer thereof, or a pharmaceutically acceptable salt or solvate thereof,

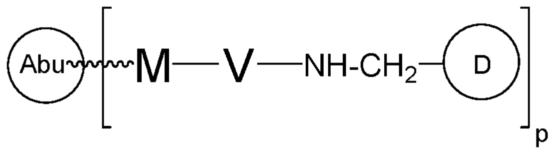

Formula I-C

wherein Abu, M, V, D, and p are as defined herein.

In one or more embodiments, Abu is antibody ASG-AM.

In one or more embodiments, the ADC is ASG-AM-Dxd, or a pharmaceutically acceptable salt or solvate thereof.

Pharmaceutical composition

The antibody or the antigen-binding unit of the present invention or the antibody-drug conjugate comprising the antibody or the antigen-binding unit of the present invention may be incorporated into a pharmaceutical composition suitable for administration. The principles and considerations involved in preparing such compositions, as well as guidelines for selecting components, are well known in the art.

Such compositions typically comprise the antibody or the antigen-binding unit or the antibody-drug conjugate, and a pharmaceutically acceptable carrier. In one or more embodiments, the antigen-binding unit is the smallest inhibitory fragment that specifically binds to the target protein. For example, a peptide that is based on the variable region sequence of an antibody and retains the ability to bind to a target protein sequence is used. In one or more embodiments, the pharmaceutical composition further comprises an anti-cancer agent (e.g., an immune checkpoint inhibitor).

As used herein, the term "pharmaceutically acceptable carrier" is intended to include any and all solvents, stabilizers, buffers, dispersion media, coatings, antibacterial agents, isotonic, absorption delaying agents, and the like that are compatible with the administration. Suitable carriers are described in *Remington's Pharmaceutical Sciences.* Such carriers or diluents can selectively include, but are not limited to, water, normal saline, Ringer's solution, glucose solution, and 5% human serum albumin.

In one or more embodiments, the formulation to be used for *in vivo* administration must be sterile, which can be readily achieved by filtration through sterile filter membranes.

The pharmaceutical compositions can be formulated in dosage unit form for ease of administration and uniformity of doses. As used herein, the dosage unit form refers to physically separable units that are suitable as unitary doses for a patient to be treated; each unit contains a predetermined amount of one or more of the antibodies, the antigen-binding units, or the antibody-drug conjugates calculated to produce the desired therapeutic effect in association with the required pharmaceutical carrier.

The pharmaceutical compositions can be placed in a container or dispenser and packaged together with instructions for administration.

The pharmaceutical compositions of the present invention may, depending on the particular condition to be treated, further comprise additional active ingredients, preferably those with complementary activities that do not adversely affect each other. In one or more embodiments, the composition can comprise a reagent that enhances its functionality, such as a cytotoxic reagent, a cytokine, a chemotherapeutic agent, or a growth inhibitory agent. Such active ingredients are properly present in combination in amounts that are effective for the intended purpose.

The composition disclosed herein may be formulated in a neutral or salt form.

Treatment Method and Use

The antibody or the antigen-binding unit or the ADC described herein may be used in a variety of applications including, but not limited to, treatment methods, such as the treatment of a tumor. In one or more embodiments, the antibody or the antigen-binding unit or the ADC may be used to inhibit tumor growth, reduce tumor volume, and/or reduce the tumorigenicity of a tumor. The method of use may be *in vitro, ex vivo,* or *in vivo.*

In one or more embodiments, the method for inhibiting tumor growth comprises contacting a tumor *in vitro* with an antibody, an antigen-binding unit, or an ADC, or a pharmaceutical composition comprising same. For example, an immortalized cell line or cancer cell expressing Nectin-4 is cultured in a medium supplemented with the antibody, the antigen-binding unit, or the ADC, or the pharmaceutical composition comprising same. In one or more embodiments, tumor cells are isolated from a patient sample and transferred to the medium containing the antibody, the antigen-binding unit, or the ADC, or the pharmaceutical composition comprising same for culture. In one or more embodiments, the method for inhibiting tumor growth comprises contacting a tumor or a tumor cell *in vivo* with an antibody, an antigen-binding unit, or an ADC, or a pharmaceutical composition comprising same.

The antibody, the antigen-binding unit, or the ADC, or the pharmaceutical composition comprising same of the present invention can be used for the diagnosis, prognosis, monitoring, treatment, alleviation, and/or prevention of a disease or disorder associated with the abnormal expression of Nectin-4 in a patient. When the disease or disorder associated with the abnormal expression of Nectin-4 in the patient is identified by using a conventional method, the antibody, the antigen-binding unit, or the ADC, or the pharmaceutical composition comprising same of the present invention may be administered. In one or more embodiments, the extent of Nectin-4 expression is detected by immunohistochemistry (IHC), flow cytometry, nucleic acid hybridization, or the like.

In one or more embodiments, the present invention relates to: a method for treating an associated disease with Nectin-4 as the treatment target, thereby ameliorating, slowing, inhibiting, treating, or preventing any disease or disorder associated with the abnormal expression of Nectin-4 (e.g., Nectin-4 overexpression); a method for treating tumors (including benign tumors and cancers) in a patient, a method for alleviating a symptom of a tumor (including benign tumors and cancers) in a patient, and a method for avoiding the recurrence of a tumor (including benign tumors and cancers) in a patient, comprising: administering to the patient an effective amount of the antibody or the antigen-binding unit or the ADC described herein.

In one or more embodiments, the present disclosure provides a method for preventing, treating, or ameliorating a disease, comprising: administering to a patient in need an effective amount of the antibody or the antigen-binding unit or the ADC, or the pharmaceutical composition comprising same described herein. In one or more embodiments, the present invention provides use of the antibody or the antigen-binding unit or the ADC in preparing a medicament for preventing, treating, or ameliorating a disease. In one or more embodiments, the disease is a disease associated with the expression of Nectin-4. In one or more embodiments, the disease is a disease associated with the abnormal expression of Nectin-4. In one or more embodiments, the disease is a disease associated with the overexpression of Nectin-4. In one or more embodiments, the disease is a tumor expressing Nectin-4. In one or more embodiments, the disease is a tumor overexpressing Nectin-4. In one or more embodiments, the disease is a cancer overexpressing Nectin-4. In one or more embodiments, the disease is a cancer overexpressing human Nectin-4.

In one or more embodiments, the present invention provides a method for treating a tumor (including benign tumors and cancers), comprising: administering to a patient in need an effective amount of the antibody or the antigen-binding unit or the ADC, or the pharmaceutical composition comprising the same. Examples of the cancer include, but are not limited to, a solid tumor, a hematological cancer, and a metastatic lesion. Specific examples of such cancers include, but are not limited to, breast cancer (e.g., triple-negative breast cancer (TNBC); topical or metastatic TNBC), pancreatic cancer, bladder cancer, urothelial carcinoma, melanoma, lung cancer (e.g., non-small cell lung cancer, squamous cell cancer, or lung adenocarcinoma), head and neck cancer (e.g., head and neck squamous cell carcinoma), cervical cancer (e.g., cervical squamous cell carcinoma), ovarian cancer, choriocarcinoma, skin cancer, esophageal cancer (e.g., esophageal adenocarcinoma), gastric cancer, uterine cancer (e.g., endometrial cancer), gallbladder cancer, liver cancer, hepatocellular carcinoma, urinary tract cancer, renal pelvic carcinoma, ureteral cancer, colorectal cancer, colon cancer, and prostate cancer. In one or more embodiments, the cancer is a metastatic or advanced cancer. In one or more embodiments, the patient is a patient with an increased expression level of Nectin-4. In one or more embodiments, the patient is a human. The antibody or the antigen-binding unit or the ADC of the present invention may be administered to a human patient for therapeutic purposes. Furthermore, the antibody or the antigen-binding unit or the ADC of the present invention may be administered to a non-human mammal expressing Nectin-4 (for veterinary purposes or as an animal model of a human disease). Such an animal model of a human disease can be used to assess the therapeutic efficacy (e.g., dose testing and time course of administration) of the antibody or the antigen-binding unit or the ADC disclosed herein.

The specific dose and treatment regimen for any particular patient will depend on a variety of factors including the

particular antibody or derivative thereof (e.g., ADC or pharmaceutical composition) used, the age and body weight, general health condition, sex and diet of the patient, the time of administration, frequency of metabolism, drug combination, and the severity of the particular disease being treated. These factors are judged by medical caregivers included within the scope of those of ordinary skill in the art. The dose will also depend on the individual patient to be treated, the route of administration, the type of the formulation, the nature of the compound used, the severity of the disease, and the efficacy desired. The dose employed can be determined by pharmacological and pharmacokinetic principles well known in the art. In one or more embodiments, an effective dose ranges from about 0.1 mg/kg to about 100 mg/kg, and the frequency of administration may be, for example, once a month. For example, the route of administration may be intravenous infusion, intravenous bolus injection, subcutaneous injection, intramuscular injection, and the like. It should be noted that the dose may vary with the type and severity of the condition to be alleviated. It will be further appreciated that for any specific patient, specific dosage regimens should be adjusted over time according to the patient's need and the professional judgment by the person administering the composition or supervising the administration of the composition, and that dose ranges illustrated herein are exemplary only and are not intended to limit the scope or practice of the claimed composition.

The modes of administration for the antibody or the antigen-binding unit or the ADC include, but are not limited to, intradermal, intramuscular, intraperitoneal, intravenous, subcutaneous, nasal, epidural, and oral administration. The mode of administration may be systemic or local. In addition, it may be desirable to introduce the antibody or the antigen-binding unit or the ADC of the present invention into the central nervous system by any suitable route, including intracerebroventricular and intrathecal injections; the intracerebroventricular injection may be assisted by an intracerebroventricular catheter connected to, for example, a reservoir (which may be an Ommaya reservoir). Pulmonary administration is also possible, for example, by the use of an inhaler or nebulizer, and by the use of nebulized formulations.

Various known delivery systems may be used to administer the antibody or the antigen-binding unit, or the polynucleotide encoding same, or the ADC of the present invention, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), or the construction of nucleic acids as part of a retrovirus or other vectors.

Combination Therapy

In one or more embodiments, the antibody or the antigen-binding unit or the ADC of the present invention may be administered in combination with an additional therapeutic or prophylactic modality, including the administration of one or more of the antibody or the antigen-binding unit or the ADC of the present invention together with or in combination with one or more additional therapeutic agents or modalities. For a combination therapy, the antibody or the antigen-binding unit or the ADC may be administered simultaneously or separately with the additional therapeutic agent. When administered separately, the antibody or the antigen-binding unit or the ADC of the present invention may be administered prior to or subsequent to the administration of the additional therapeutic agent.

In one or more embodiments, when the antibody, the antigen-binding unit, or the ADC of the present invention is administered to a patient, the antibody or the antigen-binding unit, the ADC, the pharmaceutical composition or the immunoconjugate disclosed herein, and one or more additional therapies, such as therapeutic modalities and/or other formulations (e.g., a therapeutic agent), may also be administered in combination to the patient.

In one or more embodiments, the antibody or the antigen-binding unit or the ADC of the present invention can be used with an immune checkpoint inhibitor. In one or more embodiments, the antibody or the antigen-binding unit or the ADC of the present invention is administered in combination with an additional therapeutic or prophylactic modality, such as radiotherapy.

Such combination therapies encompass both combined administration (wherein two or more agents are contained in the same formulation or separate formulations) and separate administration (wherein the antibody or the antigen-binding unit or the ADC of the present invention may be administered prior to, concurrently with, and/or subsequent to the administration of the additional therapy, e.g., therapeutic modalities or therapeutic agents). The antibody or the antigen-binding unit or the ADC, and/or the additional therapy, e.g., therapeutic agents or therapeutic modalities, can be administered when a disease is active or when the disease is in alleviation or less active. The antibody or the antigen-binding unit or the ADC can be administered prior to, concurrently with, or subsequent to the additional therapy, or during the alleviation of the disease.

Antibody preparation method

Using conventional recombinant DNA techniques, one or more CDRs of the antibody of the present invention can be inserted into framework regions. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the combination of framework regions and CDRs that specifically bind to at least one epitope of a target antigen. One or more amino acid substitutions are

made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

Antibodies can be prepared by using conventional recombinant DNA techniques. Vectors and cell lines for antibody production can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

A gene of the antibody (e.g., anti-Nectin-4 antibody) or the antigen-binding unit thereof may be inserted into an expression vector by standard methods (e.g., linking the antibody gene fragment to complementary restriction sites on the vector, or blunt end ligation if no restriction sites are present). The expression vector may be a plasmid, a retrovirus, a YAC, an EBV-derived episome, or the like. To express the antibody (e.g., anti-Nectin-4 antibody), the DNA encoding the full-length light and heavy chains can be inserted into an expression vector, such that the genes are operably linked to transcriptional and translational control sequences. The "operably link" means that the antibody genes are linked into a vector such that transcriptional and translational control sequences within the vector perform their intended functions of regulating the transcription and translation of the antibody genes.

The consensus sequence J region of the heavy and light chains can be used to design oligonucleotides for use as primers to introduce useful restriction sites into the J region for subsequent linkage of V region fragments to C region fragments. The C region cDNA may be modified by site-directed mutagenesis to place a restriction site at a similar position in the human sequence. In one or more embodiments, the expression vector already carries antibody constant region sequences prior to the insertion of the antibody-related light or heavy chain gene sequences. For example, one method for converting the anti-Nectin-4 antibody-related VH and VL sequences into full-length antibody genes is to insert them into an expression vector that already encodes a heavy chain constant region and a light chain constant region, such that the VH segment is operably linked to the CH segment within the vector and the VL segment is operably linked to the CL segment within the vector.

The recombinant expression vector may encode a signal peptide that facilitates the secretion of the heavy and light chains of an antibody from a host cell. Alternatively, the antibody heavy and light chain genes may be cloned into a vector encoding a signal peptide that facilitates the secretion of the antibody heavy and light chains from a host cell, such that the signal peptide is linked in frame to the amino termini of the antibody heavy and light chain genes. The signal peptide may be an immunoglobulin signal peptide or a heterologous signal peptide (i.e., a signal peptide from a non-immunoglobulin protein), for example, MELGLCWVFLVAILEGVQC (SEQ ID NO: 18) or MDMRVPAQLLGLLLLWFPGSRC (SEQ ID NO: 20).

In addition to the antibody heavy and light chain genes, the recombinant expression vector may also carry regulatory sequences that control the expression of the antibody chain genes in the host cell. The regulatory sequences include promoters, enhancers, and other expression control elements (e.g., polyadenylation signals) that control the transcription or translation of antibody chain genes. Such regulatory sequences are described, for example, in Goeddel, Gene Expression Technology: Methods in Enzymology 185, Academic Press, San Diego, CA, 1990. Those skilled in the art will appreciate that the design of an expression vector, including the selection of regulatory sequences, may depend on such factors as the selection of host cells to be transformed, the expression level of the desired protein, and the like. Suitable regulatory sequences for the expression in mammalian host cells include viral elements that direct high-level protein expression in mammalian cells, such as promoters and/or enhancers derived from cytomegalovirus (CMV) (e.g., the CMV promoter/enhancer), simian virus 40 (SV40) (e.g., the SV40 promoter/enhancer), adenovirus (e.g., the adenovirus major late promoter (AdMLP)), and polyoma. For further description of viral regulatory elements and sequences thereof, see, e.g., U.S. Pat. Nos. 5,168,062, 4,510,245, and 4,968,615.

In addition to the antibody chain genes and regulatory sequences, the recombinant expression vector may carry other sequences, such as a sequence that regulates the replication of the vector in the host cell (e.g., an origin of replication) and a selectable marker gene. The selectable marker gene facilitates the selection of host cells into which the vector has been introduced (see, e.g., U.S. Pat. Nos. 4,399,216, 4,634,665, and 5,179,017). For example, generally, a marker gene that imparts resistance to drugs (e.g., G418, hygromycin, or methotrexate) to the host cell into which the vector has been introduced can be selected. Suitable selectable marker genes include the dihydrofolate reductase (DHFR) gene (for use in DHFR-host cells with methotrexate selection/amplification), the neo gene (for G418 selection), and the GS gene. For the expression of heavy and light chains, an expression vector encoding the heavy and light chains is transfected into a host cell by standard techniques. In one or more embodiments, the inserted gene fragment should comprise a selectable marker gene, and common selectable marker genes include screening genes such as dihydrofolate reductase, glutamine synthetase, neomycin resistance, and hygromycin resistance, to facilitate the screening and separation of cells that have been successfully transfected. The constructed plasmid is transfected to a host cell without

the above genes, and the successfully transfected cells are expanded in a large quantity in a selective culture medium to produce the desired target protein. The antibody will be purified by one or more purification procedures. The purification may be conducted by conventional methods, for example, by centrifuging a cell suspension and collecting the supernatant, and centrifuging again to further remove impurities. Protein A affinity column, ion exchange column, and other methods may be used for purifying the antibody protein.

The antibody (e.g., anti-Nectin-4 antibody) of the present invention can be prepared by the recombinant expression of the heavy and light chain genes of the antibody in a host cell. For example, the host cell is transfected with one or more recombinant expression vectors carrying heavy and light chain DNA fragments encoding the antibody such that the heavy and light chains are expressed in the host cell, the expressed antibody can be secreted into the medium in which the host cell is cultured, and the antibody can be isolated from the medium. The "transfect" refers to a wide variety of techniques commonly used to introduce a foreign DNA into eukaryotic host cells, such as electroporation, lipofection, calcium phosphate precipitation, DEAE-dextran transfection, and the like. Standard recombinant DNA methods for obtaining the antibody heavy and light chain genes, incorporating these genes into expression vectors, and introducing the vectors into host cells are well known in the art, such as those described in U.S. Pat. No. 4,816,397. The DNAs expressing the heavy chain and the light chain of the antibody may be placed in the same vector or placed in different vectors; if placed in different vectors, the vectors expressing the heavy and light chains of the antibody may transfect host cells in a proper ratio (e.g., Tihomir S. Dodev et al., A tool kit for rapid cloning and expression of recombinant antibodies, Scientific Reports volume 4, Article number: 5885 (2014)). In one or more embodiments, the antibody expression vector comprises at least one promoter element, an antibody coding sequence, a transcription termination signal, and a polyA tail. Other elements may include enhancers, Kozak sequences, and donor and recipient sites flanking the inserted sequence for RNA splicing. Efficient transcription can be achieved by early and late promoters of SV40, long terminal repeats from retroviruses such as RSV, HTLV1, and HIVI, and early promoters of cytomegalovirus, and promoters from other cells such as actin promoter can also be used. Proper expression vectors may include pIRES1neo, pRetro-Off, pRetro-On, pLXSN, pLNCX, pcDNA3.1(+/-), pcDNA/Zeo(+/-), pcDNA3.1/Hygro(+/-), pSVL, pMSG, pRSVcat, pSV2dhfr, pBC12MI, pCS2, pCHO1.0, or the like.

The antibody of the present invention (e.g., anti-Nectin-4 antibody) can be expressed in eukaryotic host cells. In some certain embodiments, the antibody is expressed in eukaryotic cells, such as mammalian host cells. Exemplary host cells for expressing the antibody of the present invention include Chinese hamster ovary cells (CHO cells) or CHO-S, CHO-dhfr-, CHO/DG44 or ExpiCHO obtained by the modification of CHO cells, NSO myeloma cells, COS cells, Cos1 cells, Cos7 cells, SP2 cells, CV1 cells, murine L cells, or human embryonic kidney HEK293 cells or HEK293T, HEK293F or HEK293E cells obtained by modification of HEK293 cells. After a recombinant expression vector encoding an antibody chain gene is introduced into a host cell, the host cell is cultured in a medium for a period of time sufficient to allow the antibody to be expressed in the host cell or allow the antibody to be secreted into the medium to produce the antibody. The antibody can be isolated from the medium using standard protein purification methods.

Antibody-producing cell lines can be selected, constructed, and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

For the recombinant expression of the antibody (e.g., anti-Nectin-4 antibody) of the present invention, the host cell can be co-transfected with two recombinant expression vectors, a first recombinant expression vector encoding the heavy chain of the antibody and a second recombinant expression vector encoding the light chain of the antibody. The two recombinant expression vectors may contain the same selectable marker, or they may each contain separate selectable markers. Alternatively, a host cell may be transfected with a recombinant expression vector encoding the heavy and light chains of the antibody.

The antibody (e.g., anti-Nectin-4 antibody) of the present invention may also be produced by chemical synthesis (e.g., by the methods described in Solid Phase Peptide Synthesis, second edition, 1984, The Pierce Chemical Co., Rockford, Ill.). Variant antibodies may also be generated using a cell-free platform (see, e.g., Chu et al., Biochemia No. 2, 2001 (Roche Molecular Biologicals) and Murray et al., 2013, Current Opinion in Chemical Biology, 17: 420-426).

The antibody (e.g., anti-Nectin-4 antibody) produced by recombinant expression may be purified by any method known in the art for purifying immunoglobulin molecules, for example, by chromatography (e.g., ion exchange, affinity, and sizing column chromatography), centrifugation, differential solubility, or any other standard technique for purifying proteins. For example, protein A or protein G affinity chromatography may be used, which provides primarily the IgG fraction in immune serum. In addition, the specific antigen targeted by the immunoglobulin, or an epitope thereof, may be immobilized on a column to purify the immune-specific antibody by immunoaffinity chromatography. The antibody (e.g., anti-Nectin-4 antibody) of the present invention can be fused to heterologous polypeptide sequences known in the art to facilitate the purification. For the purification of immunoglobulins, reference can be made to D. Wilkinson's article (The Scientist, published by the Scientist, Inc., Philadelphia Pa., Vol. 14, No. 8 (Apr. 17, 2000), pp. 25-28).

In addition, mutations can be introduced in the nucleotide sequence encoding the antibody of the present invention using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids, or a substitution of less than 2 amino acids relative to the VH CDR1, VH CDR2, and VH CDR3 of the original heavy chain variable region and the VL CDR1, VL CDR2, or VL CDR3 of the original light chain variable region. Alternatively, mutations may be introduced randomly along all or part of the encoding sequence, for example, by saturation mutagenesis, and the resulting mutants may be screened for the biological activity to identify mutants that retain the activity. In one or more embodiments, the substitutions described herein are conservative amino acid substitutions.

Synthesis Method

The present invention further provides preparation method for drug conjugates , such as antibody-drug conjugates, and intermediates. The drug conjugates , such as antibody-drug conjugates, and intermediates of the present invention can be prepared by using known formulations and methods. In one or more embodiments, the methods for preparing are described below.

Preparation method of

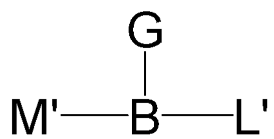

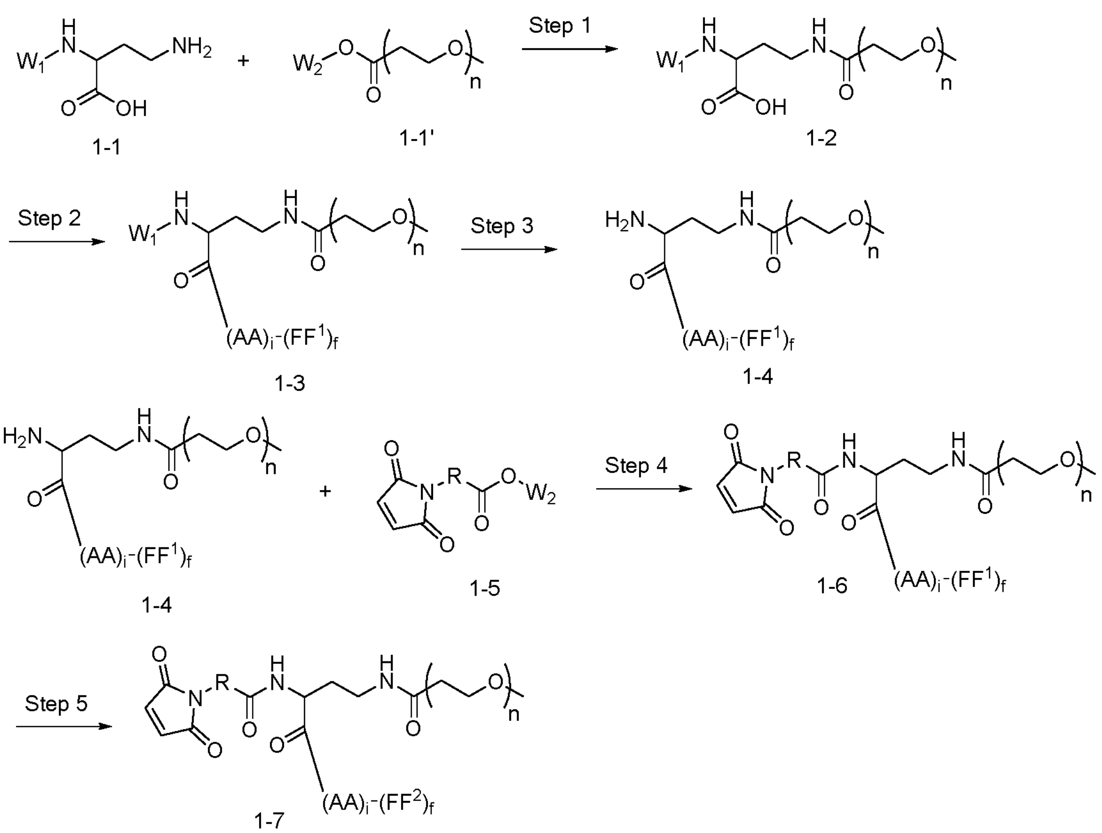


1-1 1-1' 1-2 1-3 1-4 1-4 1-5 1-6 1-7

Step 1: reacting a compound of general Formula 1-1 with a compound of general Formula 1-1' under a basic condition to obtain a compound of general Formula 1-2;

Step 2: reacting the compound of general Formula 1-2 with general Formula $(AA)_i$-$(FF^1)_f$ in the presence of a

condensing agent under a basic condition to obtain a compound of general Formula 1-3;

Step 3: removing the amino-protecting group $W_1$ of the compound of general Formula 1-3 to obtain a compound of general Formula 1-4;

Step 4: reacting the compound of general Formula 1-4 with a compound of general Formula 1-5 under a basic condition to obtain a compound of general Formula 1-6; and

Step 5: reacting the compound of general Formula 1-6 with bis(p-nitrophenyl)carbonate under a basic condition to obtain a compound of general Formula 1-7.

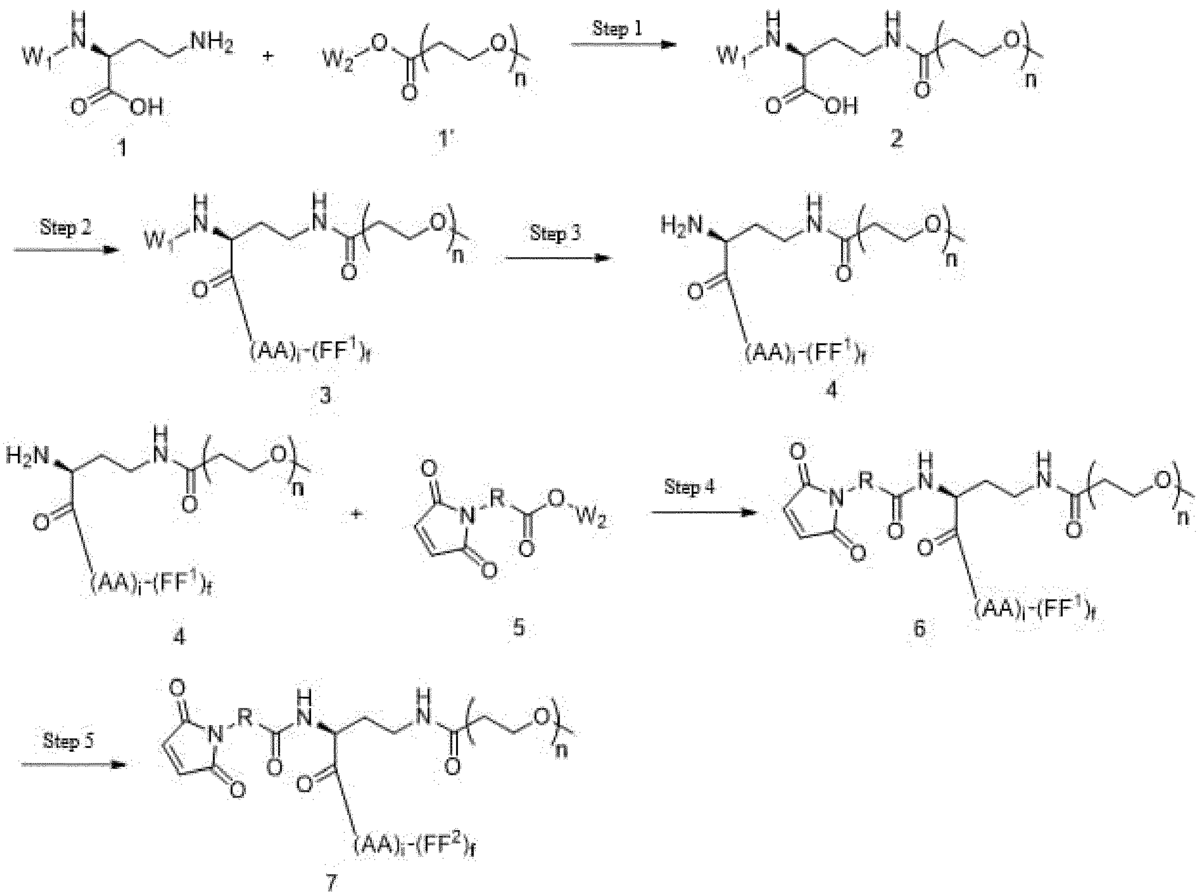


Step 1: reacting a compound of general Formula 1 with a compound of general Formula 1' under a basic condition to obtain a compound of general Formula 2;

Step 2: reacting the compound of general Formula 2 with general Formula $(AA)_i$-$(FF^1)_f$ in the presence of a condensing agent under a basic condition to obtain a compound of general Formula 3;

Step 3: removing the amino-protecting group $W_1$ of the compound of general Formula 3 to obtain a compound of general Formula 4;

Step 4: reacting the compound of general Formula 4 with a compound of general Formula 5 under a basic condition to obtain a compound of general Formula 6; and

Step 5: reacting the compound of general Formula 6 with bis(p-nitrophenyl)carbonate under a basic condition to obtain a compound of general Formula 7;

wherein

$W_1$ is an amino-protecting group, e.g., 9-fluorenylmethyloxycarbonyl; $W_2$ is a carboxylic acid active ester, for example, a succinimidyl ester;

wherein B, G, n, R, AA, i, f are described as herein;

M' is

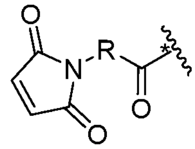

,

wherein * links to B, R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_{2O})_r-CH_2-$, $-(CH_2)_rC(O)NR^m(CH_2CH_2O)_r-$, - $(CH_2)_rC(O)NR^m(CH_2CH_2O)_r-CH_2-$, $-(CH_2CH_2O)_rC(O)NR^m(CH_2CH_2O)_r-$, - $(CH_2CH_2O)_rC(O)NR^m(CH_2CH_2O)_r-CH_2-$ and $-(CH_2CH_2O),C(O)NR^m(CH_2),-$; wherein each $R^m$ is independently H, C1-C6 alkyl, C3-C8 carbocyclyl, phenyl or benzyl; and each r is independently 1, 2, 3, 4, 5, 6, 7, 8, 9 or 10; or R is $-(CH_2)_r-$, or r is 1 or 5;

L' is -(AA)i-(FF')$_r$-, wherein AA is amino acid or polypeptide, i is 1, 2, 3, 4, 5, 6, 7, 8, 9, 10, 11, 12, 13, 14, 15, 16, 17, 18, 19 or 20; or each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu and Gly-Phe-Leu-Gly; or AA is Val-Cit, i is 1; each FF' is

independently

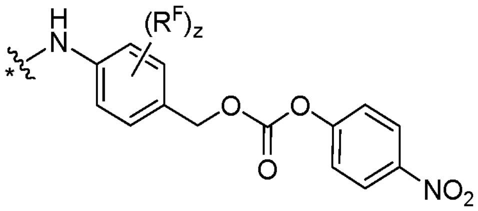

,

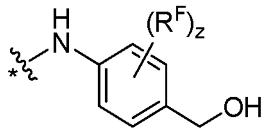

,

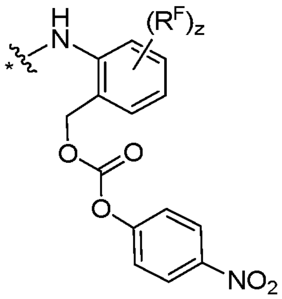

,

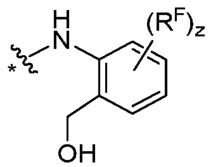

,

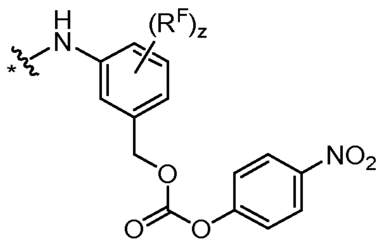

or

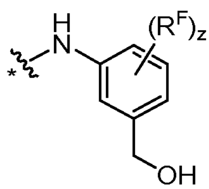

,

wherein $R^F$ is C1-C6 alkyl, C1-C6 alkoxy, $-NO_2$ or halogen; wherein * links to AA, or z is 0 or $R^F$ is F,

or z is 0, 1, 2, 3 or 4, or z is 1 or 2; or each FF' is independently

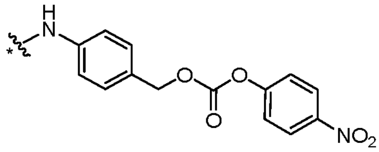,

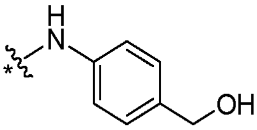, 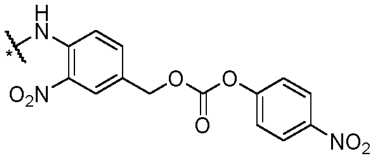, 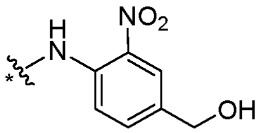,

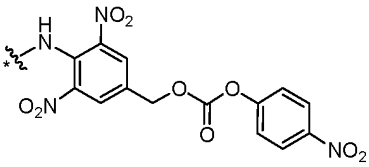, 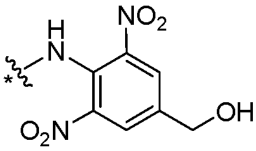, 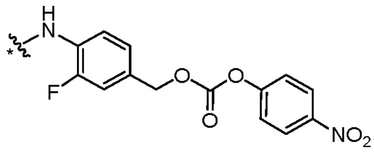,

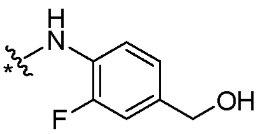, 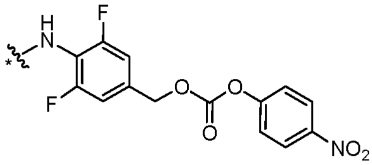, 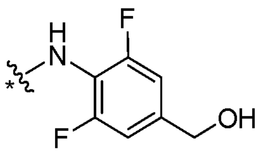,

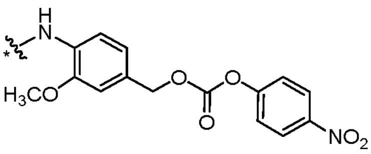, 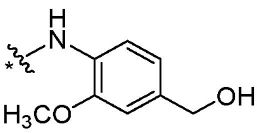, 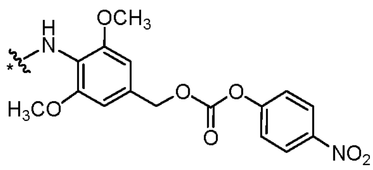,

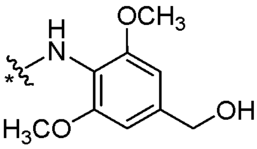, 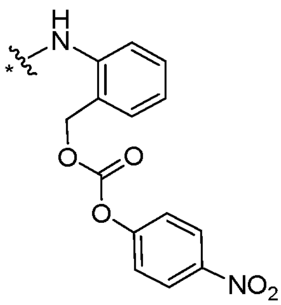, 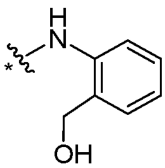, 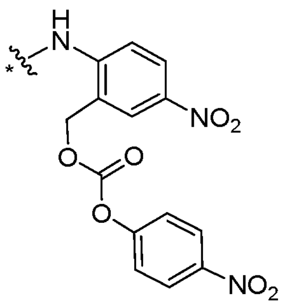,

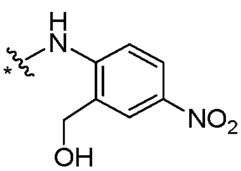, 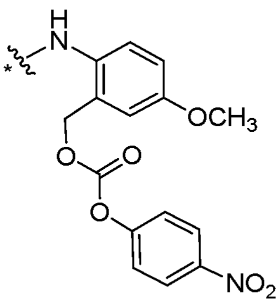, 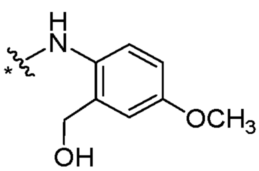, 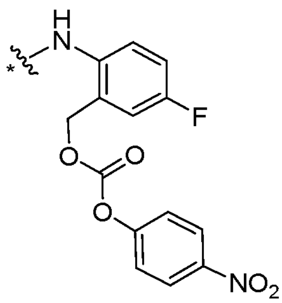,

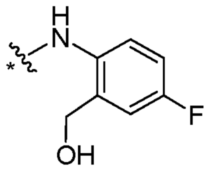 , 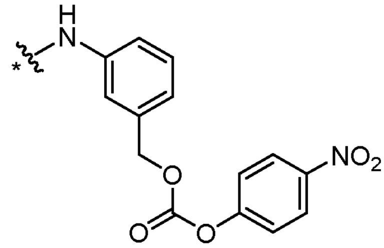 , 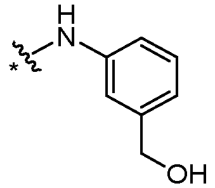 ,

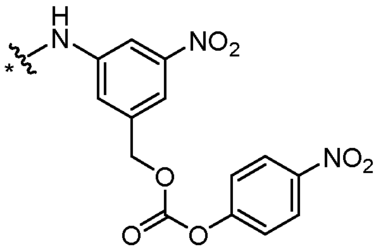 , 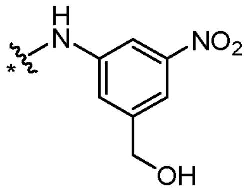 , 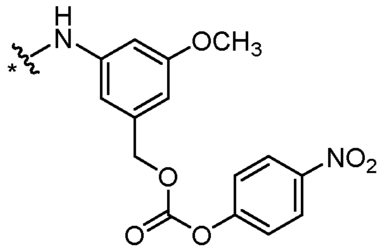 ,

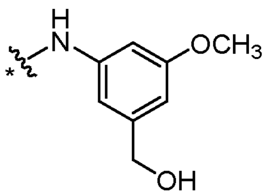 , 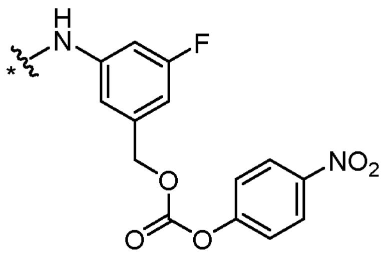 , or 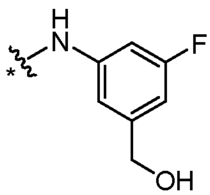 ,

wherein * links to AA, f is 1, 2, 3, 4, 5, 6, 7, 8, 9 or 10; or FF' is p-aminophenyl (4-nitrophenyl) carbonate or p-aminobenzyl

alcohol, f is 1; or L' is

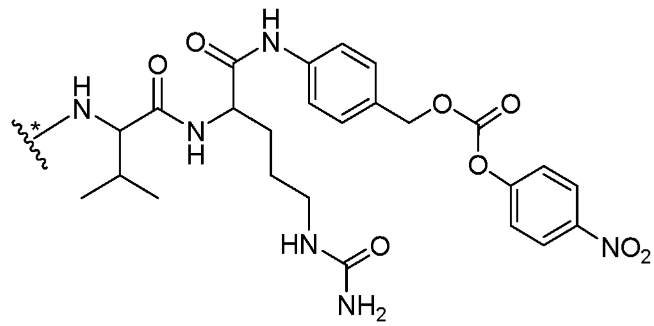

or

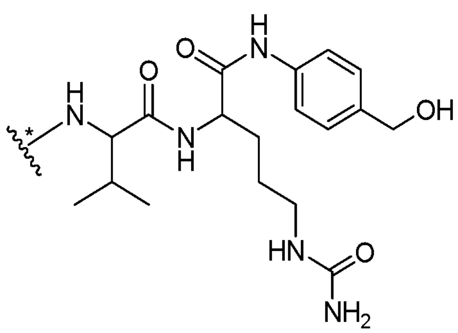

,

wherein * links to B, or L' is

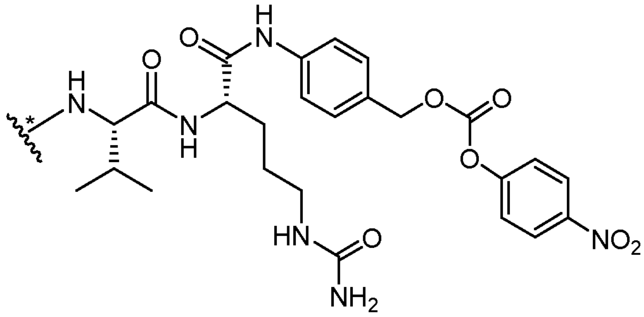

or

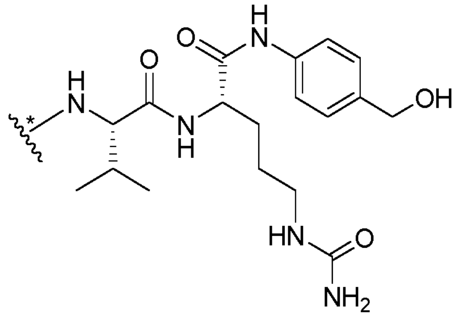

,

wherein * links to B;

each $FF^1$ is independently

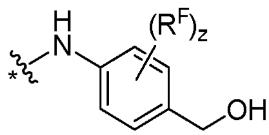

,

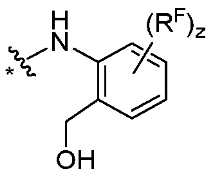

or

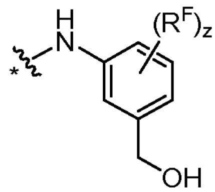

,

wherein

each $R^F$ is independently C1-C6 alkyl, C1-C6 alkoxy, $-NO_2$ or halogen; or z is 0, 1, 2, 3 or 4; wherein * links to AA;

each $FF^2$ is independently

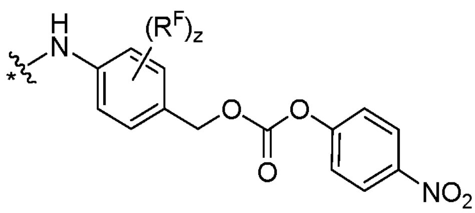

,

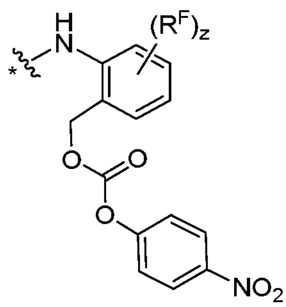

or

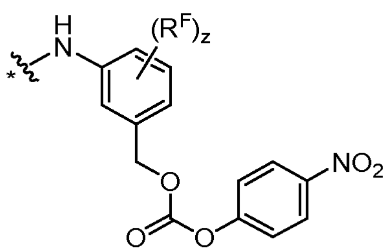

,

wherein each $R^F$ is independently C1-C6 alkyl, C1-C6 alkoxy, $-NO_2$

or halogen; or z is 0, 1, 2, 3 or 4; wherein * links to AA.

The basic conditions described above can be provided using reagents including organic bases and inorganic bases, wherein the organic bases include, but are not limited to, triethylamine, diethylamine, N-methylmorpholine, pyridine, piperidine, N,N-diisopropylethylamine, n-butyllithium, lithium diisopropylamide, potassium acetate, sodium tert-butoxide, or potassium tert-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from N,N,N,N-tetramethyl-O-(7-azabenzotriazol-1-yl) uronium hexafluorophosphate, 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, *N*,*N*'-dicyclohexylcarbodiimide, *N*,*N*'-diisopropylcarbodiimide, *O*-benzotriazol-*N*,*N*,*N*',*N* -tetramethyluronium tetrafluoroborate, 1-hydroxybenzotriazole, 1-hydroxy-7-azobenzotriazol, O-benzotriazol-*N*,*N*,*N'*,*N'*-tetramethyluronium hexafluorophosphate, 2-(7-azobenzotriazol)-*N*,*N*,*N*',*N*'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method of

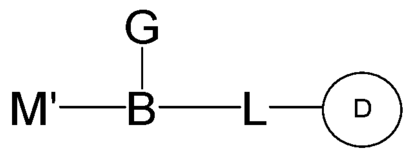

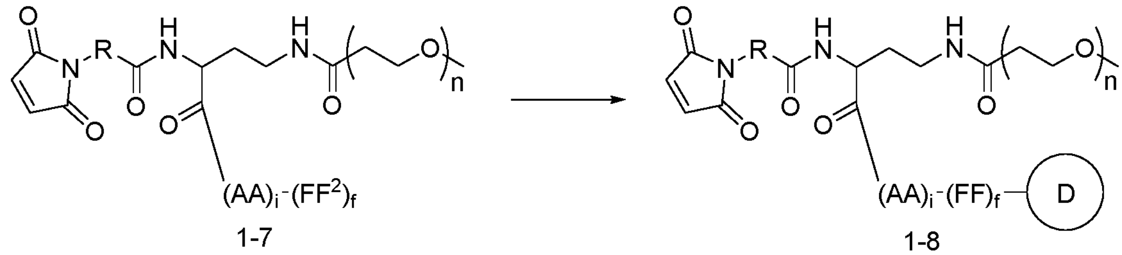

1-7 1-8

The compound of general Formula 1-7 and D are reacted in the presence of a condensing agent under a basic condition to obtain a compound of general Formula 1-8.

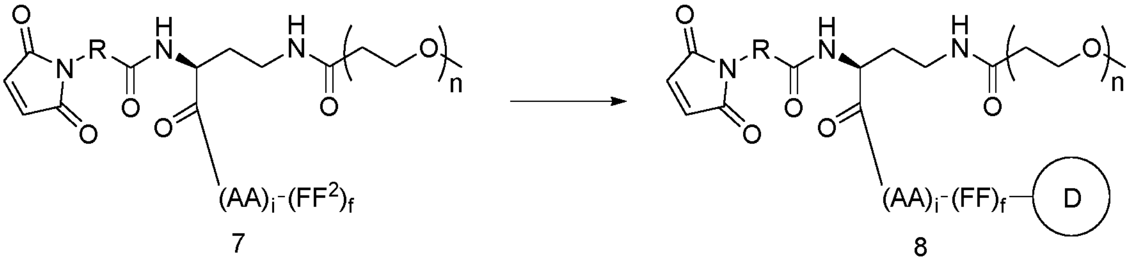

7 8

The compound of general Formula 7 and D are reacted in the presence of a condensing agent under a basic condition to obtain a compound of general Formula 8.

Wherein M', B, L, D, G, n, R, AA, FF, $FF^2$, i, f are as described herein.

The basic conditions described above can be provided using reagents including organic bases and inorganic bases, wherein the organic bases include, but are not limited to, triethylamine, diethylamine, *N*-methylmorpholine, pyridine, piperidine, *N*,*N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from *N*,*N*,*N'*,*N'*-tetramethyl-O-(7-azabenzotriazol-1-yl) uronium hexafluorophosphate, 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, *N*,*N*'-dicyclohexylcarbodiimide, *N*,*N*'-diisopropylcarbodiimide, *O*-benzotriazol-*N*,*N*,*N*',*N'*-tetramethyluronium tetrafluoroborate, 1-hydroxybenzotriazole, 1-hydroxy-7-azobenzotriazol, *O*-benzotriazol-*N*,*N*,*N'*,*N'*-tetramethyluronium hexafluorophosphate, 2-(7-azobenzotriazol)-*N*,*N*,*N*',*N*'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method of

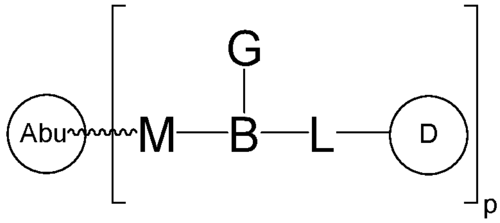

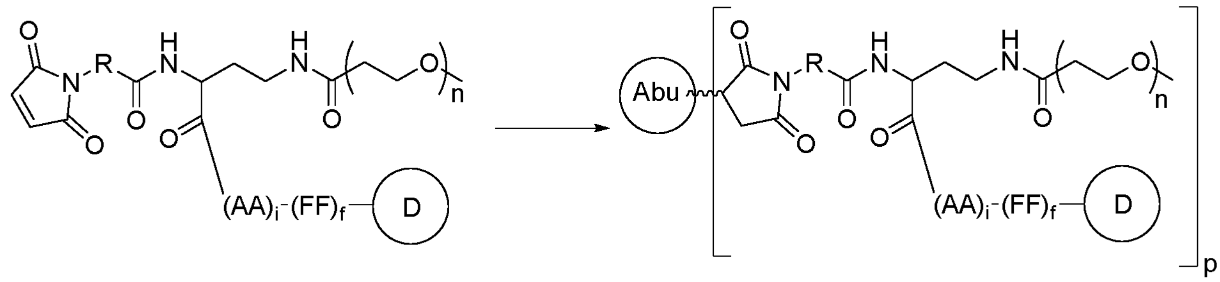

1-8 1-9

The compound of general Formula 1-8 is conjugated to Abu under a weakly acidic condition to obtain a compound of general Formula 1-9.

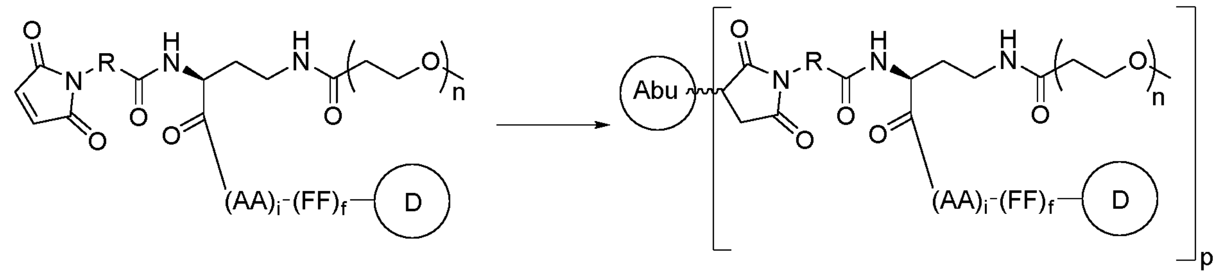

8 9

The compound of general Formula 8 is conjugated to Abu under a weakly acidic condition to obtain a compound of general Formula 9.

Wherein Abu, M, B, L, D, G, n, R, AA, FF, i, f are as described in formula I-A.

The weakly acidic condition described above may be provided using reagents including organic acids and inorganic acids, wherein the organic acids include, but are not limited to, acetic acid, benzoic acid, tartaric acid, oxalic acid, malic acid, citric acid, ascorbic acid, citric acid, salicylic acid, caffeic acid, sorbic acid, quinic acid, oleanolic acid, succinic acid, chlorogenic acid, formic acid, and propionic acid, and the inorganic acids include, but are not limited to, carbonic acid, nitrous acid, acetic acid, hypochlorous acid, hydrofluoric acid, sulfurous acid, hydrosulfuric acid, silicic acid, metasilicic acid, phosphoric acid, metaphosphoric acid, sodium bicarbonate, and sodium bisulfite.

The drug conjugate can be purified by a conventional method, e.g., preparative high performance liquid chromatography (prep-HPLC) or the like.

The present application will be illustrated with reference to specific examples, but the content of the present application is not limited thereto.

The reagents and instruments used in the following methods are those conventional in the art and commercially available unless otherwise specified; the methods used are all conventional in the art, and those skilled in the art can undoubtedly implement the methods and acquire the corresponding results according to the content described in the examples.

**Example 1: Expression of human Nectin-4 extracellular antigen Nectin-4-His**

The human Nectin-4 extracellular antigen Nectin-4-His was prepared using conventional methods: The DNA sequence encoding the antigen Nectin-4-His (acquired by replacing the signal peptide sequence of the human Nectin-4 extracellular sequence (sequence source Q96NY8) with the albumin signal peptide and adding an 8× HIS tag (HHHHHHHH) at the C-terminus to construct Nectin-4-His, with the amino acid sequence set forth in SEQ ID NO: 1)

was cloned into an expression vector. Eukaryotic cells (HEK293F cells) were transiently transfected, or CHO cells were stably transfected. Stable cell lines were selected and purified for expression to give the antigen Nectin-4-His. MKWVTFISLLFLFSSAYSGELETSDVVTVVLGQDAKLPCFYRGDSGEQVGQVAWARVDAGEG AQELALLHSKYGLH VSPAYEGRVEQPPPPRNPLDGSVLLRNAVQADEGEYECRVSTFPAGSFQ ARLRLRVLVPPLPSLNPGPALEEGQGLT LAASCTAEGSPAPSVTWDTEVKGTTSSRSFKHSRSA AVTSEFHLVPSRSMNGQPLTCVVSHPGLLQDQRITHILHVSF LAEASVRGLEDQNLWHIGREG AMLKCLSEGQPPPSYNWTRLDGPLPSGVRVDGDTLGFPPLTTEHSGIYVCHVSNE FSSRDSQV TVDVLDPQEDSGKQVDLVSASHHHHHHHH (SEQ ID NO: 1, with the albumin signal peptide underlined).

The acquired protein was sequenced and analyzed, and the results indicate the consistency with expectations.

**Example 2: Anti-Nectin-4 antibody and preparation thereof**

The heavy chain of anti-Nectin-4 antibody ASG-AM is set forth in SEQ ID NO: 12, and the light chain is set forth in SEQ ID NO: 14. In order to facilitate the expression in host cells, N-terminal signal peptides were added to the heavy chain and the light chain. The heavy chain signal peptide is set forth in SEQ ID NO: 18, with the DNA sequence set forth in SEQ ID NO: 19, and the light chain signal peptide is set forth in SEQ ID NO: 20, with the DNA sequence set forth in SEQ ID NO: 16. The amino acid sequences of the heavy and light chains of the reference antibody ASG-22 are shown in Table 2. DNA sequences encoding the heavy chain and the light chain of the antibody were cloned into an expression vector to construct a recombinant expression vector. HEK293F cells were transiently transfected with the vector, cultured, and purified to acquire the antibodies. Upon verification, the acquired antibodies were consistent with expectations.

**Table 2. The amino acid sequences of the reference antibody ASG-22**

| name | SEQ ID NO | sequence |
|---|---|---|
| heavy chain | 12 | EVQLVESGGGLVQPGGSLRLSCAASGFTFSSYNMNWVRQAPGKG LEWVSYISSSSSTIYYADSVKGRFTISRDNAKNSLSLQMNSLRDED TAVYYCARAYYYGMDVWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTH TCPPCPAPELLGGPSVFLFPPKPKDTLMISRTPEVTCVVVDVSHED PEVKFNWYVDGVEVHNAKTKPREEQYNSTYRVVSVLTVLHQDW LNGKEYKCKVSNKALPAPIEKTISKAKGQPREPQVYTLPPSRDELT KNQVSLTCLVKGFYPSDIAVEWESNGQPENNYKTTPPVLDSDGSF FLYSKLTVDKSRWQQGNVFSCSVMHEALHNHYTQKSLSLSPGK |
| light chain | 17 | DIQMTQSPSSVSASVGDRVTITCRASQGISGWLAWYQQKPGKAPK FLIYAASTLQSGVPSRFSGSGSGTDFTLTISSLQPEDFATYYCQQAN SFPPTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |

**Example 3: Affinity measurement of antibody ASG-AM by SPR**

The candidate antibodies (ASG-22 and ASG-AM) were analyzed for their affinity for the human Nectin-4 extracellular antigen using surface plasmon resonance (SPR) technology (BIAcore: GE Healthcare, T200).

The capture was conducted on a Protein A chip (Cat# 29127556, GE Healthcare): Antibody dilutions (5 μg/mL) were directed through the experimental flow channels (Fc2 and Fc4) at a flow rate of 10 μL/min for 10 s. Then antigen Nectin-4-His (prepared from Example 1) dilutions at different concentrations (0 nM, 1.23 nM, 3.7 nM, 11.1 nM, 33.3 nM, and 100 nM) were sequentially introduced with the flow rate adjusted to 30 μL/min through the surfaces of the experimental flow channels (Fc2 and Fc4) and reference flow channels (Fc1 and Fc3) with 120 s of association and 180 s of dissociation. Finally, the chip regenerated in Glycine 1.5. Fitting and analysis were performed using BiaEvaluation 3.2, with a 1:1 model for the fitting. The results of the fitting are shown in Table 3 below.

Antibody ASG-AM showed a significant increase in affinity as compared with the reference antibody ASG-22.

**Table 3. Affinities of antibodies for antigens**

| Sample | Ka (1/Ms) | Kd (1/s) | KD (M) | Rmax (RU) | $Chi^2$ ($RU^2$) |
|---|---|---|---|---|---|
| ASG-AM | 2.98E+05 | 5.35E-04 | 1.79E-09 | 197.9 | 7.16 |
| ASM-22 | 6.06E+05 | 5.23E-03 | 8.64E-09 | 164.2 | 1.28 |

### Example 4: Affinity measurement of antibody ASG-AM by BLI

The affinity of candidate antibodies (ASG-22 and ASG-AM) for the human Nectin-4 extracellular antigen was detected using the bio-layer interferometry (BLI) technology on the Octet platform (Fortebio, Octet QKe).

Antibodies were loaded onto a Protein A biosensor (Fortebio, 18-5010) at a concentration of 20 μg/mL with a loading time of 60 s. Nectin-4-His antigen concentrations were set at 200 nM, 100 nM, 50 nM, 25 nM, and 0 nM. The antigen association time was 180 s, and the dissociation time was 200 s. Data were then processed and analyzed using Acquisition 8.2 to give affinity constants (see Table 4). The results indicate that the antibody ASG-AM has a significantly improved affinity compared with the reference antibody ASG-22.

**Table 4. Affinities of antibodies for antigens**

| Sample | KD (M) | kon (1/Ms) | kdis (1/s) | RMax | Full R^2 |
|---|---|---|---|---|---|
| ASG-AM | 2.66E-10 | 8.96E+04 | 2.38E-05 | 0.6488 | 0.9985 |
| ASG-22 | 1.43E-08 | 1.13E+05 | 1.62E-03 | 0.5303 | 0.9992 |

### Example 5: Binding curves of antibody ASG-AM to cells

The binding activity of antibody ASG-AM to T-47D cells (human ductal breast carcinoma cells, sourced from the Cell Bank of the Chinese Academy of Sciences, Cat. No.: TCHu 87) was detected by FACS and compared with that of the reference antibody ASG-22. The $EC_{50}$ of the binding of candidate antibodies to the cells was analyzed using the four-parameter method. The method is briefly described as follows: T-47D cells were digested with pancreatin. Each candidate antibody was serially 3-fold diluted from 100 nM and co-incubated with T-47D cells at 4 °C for 0.5-1 h. Subsequently, a PE-labeled anti-human Fc secondary antibody (Invitrogen, 12-4998-82) was added. The signal intensity of the red fluorescence was detected by a flow cytometer. Graphpad Prism was used for plotting and calculating the $EC_{50}$. The binding curves and $EC_{50}$ results are shown in FIG. 1. The results show that the binding curves of antibody ASG-AM and reference antibody ASG-22 to T-47D cells were substantially consistent.

### Example 6: Endocytosis assay

The relative quantity of antibodies remaining on the cell surface after incubation at 37 °C for a certain period was detected by FACS to reflect the efficiency of endocytosis. The method is briefly described as follows: T-47D cells (human ductal breast carcinoma cells, sourced from the Cell Bank of the Chinese Academy of Sciences, Cat. No.: TCHu 87) were used. Each test antibody was adjusted to a concentration of 10 μg/mL and co-incubated with cells (at a density of $0.5 \times 10^6$ cells/mL) on ice for 1 h for binding. The cells were washed thrice with pre-cooled PBS and then incubated at 37 °C for 0 h, 2 h, and 4 h. The endocytosis reaction was terminated with 0.2% $N_3Na$, and the cells were washed thrice with PBS. Subsequently, a PE-labeled anti-human Fc secondary antibody (Invitrogen 12-4998-82) was added, and the cells were incubated at 4 °C for 30 min. The red fluorescence signal was detected by a flow cytometer. The endocytosis results of the test antibodies, as shown in Table 5 below, indicate no significant difference in the endocytosis efficiency between the two.

**Table 5. Endocytosis results of antibodies**

| Name | 0 h | 2h | 4h | Endocytosis at 2 h | Endocytosis at 4 h |
|---|---|---|---|---|---|
| ASG-22 | 24098.3 | 11216.0 | 6033 | 53.46% | 74.97% |
| ASG-AM | 23561.0 | 12727.3 | 4840 | 45.98% | 79.46% |

**Example 7: Synthesis Procedures for Compound (1S,9S)-1-amino-9-ethyl-4,5-difluoro-9-hydroxy-1,2,3,9,12,15-hexahydro-10H,13H-benzo[de]pyrano[3',4':6,7]indolizino[1,2-b]quinolin-10,13-dione hydrochloride (D-1)**

1. Synthesis of*N*,*N*'-(3,4-difluoro-8-oxo-5,6,7,8-tetrahydronaphthalene-1,7-diyl)diacetamide

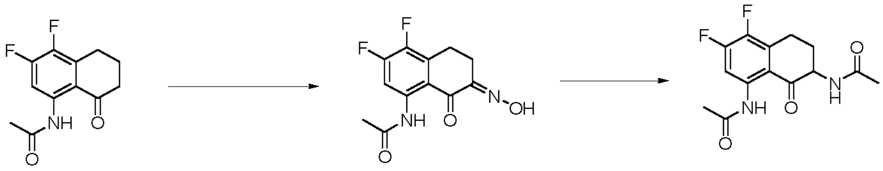

A solution of potassium tert-butoxide in tetrahydrofuran (42 mL, 1 M) was added to a dry reaction flask under a nitrogen atmosphere, stirred, and cooled to 0-5 °C. N-(3,4-difluoro-8-oxo-5,6,7,8-tetrahydronaphthalen-1-yl)acetamide (CAS No.: 143655-49-6, 5 g, 21 mmol) dissolved in tetrahydrofuran (25 mL) was slowly added dropwise to the reaction flask, followed by tert-butyl nitrite (4.32 g, 2 eq) (with temperature controlled at 0-5 °C). The resulting mixture was warmed to 15-20 °C and stirred for 2 hours (h). After the completion of the reaction, the mixture was cooled to 0-5 °C. Acetic acid (25 mL) and acetic anhydride (25 mL) were added dropwise (with temperature controlled at no more than 10 °C). After the dropwise addition, the mixture was stirred for 20 minutes (min). With temperature maintained at 5-10 °C, zinc powder (8 eq) was added according to the amount of N-(3,4-difluoro-8-oxo-5,6,7,8-tetrahydronaphthalen-1-yl)acetamide. The mixture was stirred at 20-25 °C for 1 h. The mixture was filtered, and the solid was rinsed with ethyl acetate (50 mL). The temperature was reduced to 0-5 °C, and 15% aqueous solution of $NaCO_3$ (50 mL) was added dropwise for three washings. Ethyl acetate (25 mL) was added for extraction. The organic phases were pooled and washed with a saturated aqueous solution of NaCl. Ethyl acetate (10 mL) was added, and the mixture was stirred at 40 °C for 30 min, slowly cooled to 0-5 °C, and stirred for 2 h. The mixture was filtered, and the solid was washed with ethyl acetate/petroleum ether (1/2, 10 mL). Drying was performed in vacuo to obtain a gray powder (2.1 g, 33.7%). LC-MS: $[M+H]^+$=297.

2. Synthesis of N-(8-amino-5,6-difluoro-1-oxo-1,2,3,4-tetrahydronaphthalen-2-yl)acetamide

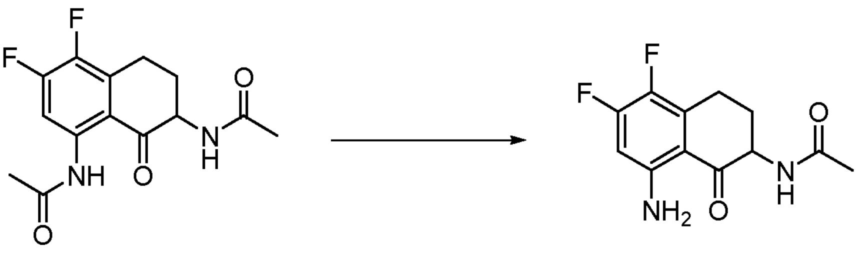

N,N-(3,4-difluoro-8-oxo-5,6,7,8-tetrahydronaphthalene-1,7-diyl)diethylamide (500 mg, 1.68 mmol) was added to a 2 M solution of hydrochloric acid in ethanol (5 mL). The resulting mixture was stirred at 50 °C for 4 h. After the completion of the reaction as detected, water (7.5 mL) was added. The mixture was cooled to 0-5 °C, and triethylamine (1.03 g) was added dropwise. The mixture was stirred for 3 h. The mixture was filtered, and washing was performed successively with 40% cold aqueous solution of ethanol (3 mL) and water (3 mL). Drying was performed *in vacuo* to obtain a gray powder (320 mg, 74.6%). LC-MS: $[M+H]^+$=255.

3. Synthesis of *N*-((9*S*)-9-ethyl-4,5-difluoro-9-hydroxy-10,13-dioxo-2,3,9,10,13,15-hexahydro-1*H*,12*H*-benzo[*de*]pyrano[3',4':6,7]indolizino[1,2-*b*]quinolin-1-yl)acetamide

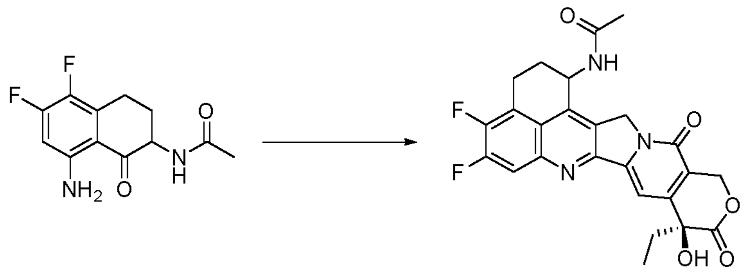

*N*-(8-amino-5,6-difluoro-1-oxo-1,2,3,4-tetrahydronaphthalen-2-yl)acetamide (1.1 g, 1 eq), (*S*)-4-ethyl-4-hydroxy-7,8-dihydro-1*H*-pyrano[3,4-f]indolizine-3,6,10(4*H*)-trione (1.15 g, 1 eq) and toluene (50 ml) were added to a reaction flask under a nitrogen atmosphere. The mixture was warmed to reflux and stirred for 1 h. Then pyridinium 4-toluenesulfonate (100 mg) was added, and the mixture was stirred at reflux for another 20 h. The mixture was cooled to room temperature and stirred for 1 h. The mixture was filtered, and the solid was successively washed with acetone (10 mL) and cold ethanol (5 mL). Drying was performed *in vacuo* to obtain a taupe powder (1.1 g, 53%). LC-MS: $[M+H]^+$=482.

4. Synthesis of (1*S*,9*S*)-1-amino-9-ethyl-4,5-difluoro-9-hydroxy-1,2,3,9,12,15-hexahydro-10*H*,13*H*-benzo[de]pyrano[3',4':6,7]indolizino[1,2-b]quinolin-10,13-dione hydrochloride

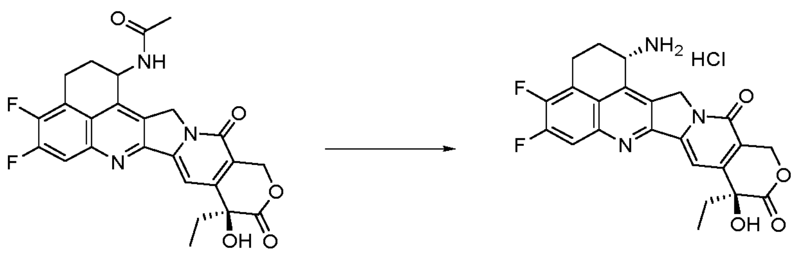

N-((9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)acetamide (1.1 g, 2.28 mmol) and a 6 M aqueous solution of hydrochloric acid (44 mL) were added to a reaction flask. The mixture was refluxed with stirring under a nitrogen atmosphere for 4 h. The mixture was concentrated to remove the solvent, and the residue was purified by HPLC to obtain a white powder (200 mg, 18%). LC-MS: $[M+H]^+$=440.

**Example 8: Synthesis Procedures for 4-((305,335,36S)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07)**

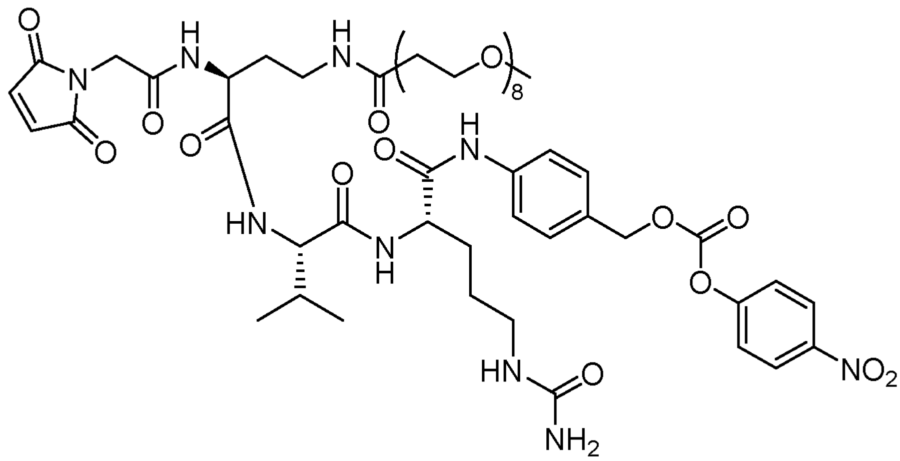

1) Synthesis of (*S*)-30-((((9H-fluoren-9-yl)methoxy)carbonyl)amino)-26-oxo-2,5,8,11,14,17,20,23-octaoxa-27-aza-hentriacontan-31-oic acid (CB01).

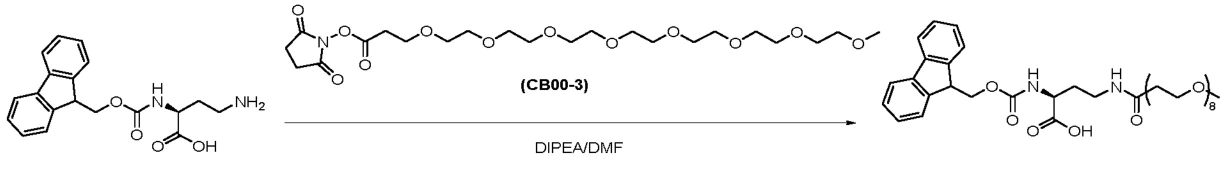


(CB00-2) (CB01)

Under a nitrogen atmosphere at 0-5 °C, 8.14 g of *N*2-fluorenylmethoxycarbonyl-L-2,4-diaminobutyric acid (CB00-2) was dissolved in 40 mL of dimethylformamide (DMF), and 10 g of *N*-succinimidyl 4,7,10,13,16,19,22,25-octaoxahexacosanoate (CB00-3) and 10 mL of DMF were added. 6.5 mL of DIPEA was dropwise added with the temperature maintained at 0-5 °C. 1 h after the addition, the mixture was stirred at room temperature for 4 h. After the completion of the reaction, DMF was removed under reduced pressure, and the residue was purified by silica gel column chromatography (with dichloromethane and methanol volume ratio 20:1 as elution solvents) to obtain CB01 in the form of a pale yellow oil (14.2 g).

2) Synthesis of (9*H*-fluoren-9-yl)methyl [(*S*)-1-[[(*S*)-1-[[4-(hydroxymethyl)phenyl]amino]-1-oxo-5-ureidopentan-2-yl] amino]-3-methyl-1-oxobutan-2-yl]carbamate (CB02).

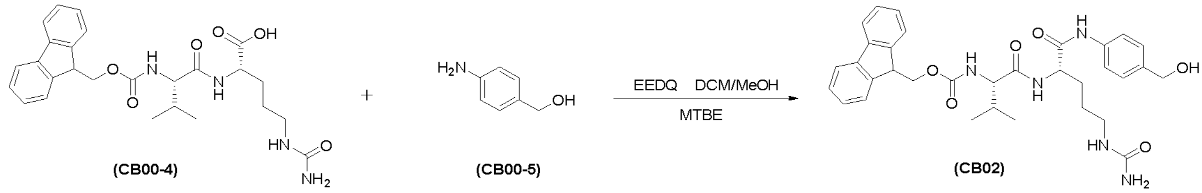


(CB00-4) + (CB00-5) (CB02)

11 g of (*S*)-2-((*S*)-2-((((9H-fluoren-9-yl)methoxy)carbonyl)amino)-3-methylbutanamido)-5-ureidopentanoic acid (CB00-4) and 5.5 g of (4-aminophenyl)methanol (CB00-5) were dissolved in 400 mL of dichloromethane and 200 mL of methanol at room temperature under a nitrogen atmosphere, and 17 g of 2-ethoxy-1-ethoxycarbonyl-1,2-dihydroquinoline (EEDQ) were added in portions with mechanical stirring. The resulting mixture was allowed to react for 15 h in the absence of light. After the completion of the reaction, the solvent was removed under reduced pressure to obtain a paste solid, which was subjected to silica gel column chromatography (with dichloromethane and methanol volume ratio 20:1 as elution solvents) to obtain an off-white solid (11.9 g).

3) Synthesis of (*S*)-2-((*S*)-2-amino-3-methylbutanamido)-*N*-(4-(hydroxymethyl)phenyl)-5-ureidopentanamide (CB03)

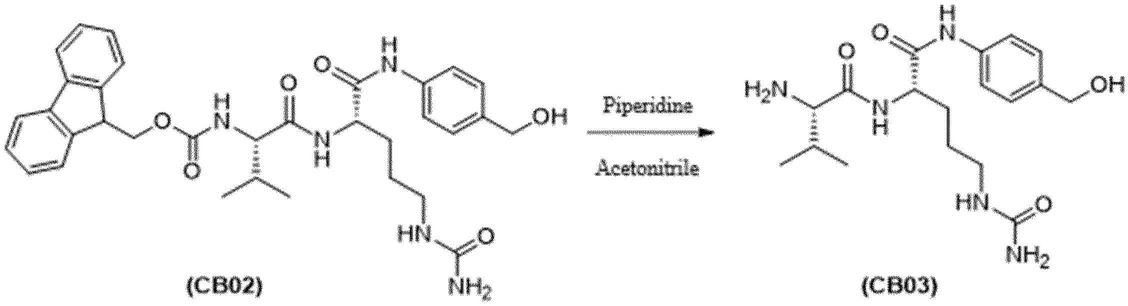


(CB02) (CB03)

Under a nitrogen atmosphere at room temperature, 300 mL of acetonitrile was added to 11.9 g of (9H-fluoren-9-yl)methyl [(*S*)-1-[[(S)-1-[[4-(hydroxymethyl)phenyl]amino]-1-oxo-5-ureidopentan-2-yl]amino]-3-methyl-1-oxobutan-2-yl]carbamate (CB02), and 18 mL of piperidine was added dropwise with stirring. After the dropwise addition, the mixture was allowed to react at room temperature for 2 h. After the completion of the reaction, the mixture was distilled under reduced pressure to remove the solvent and piperidine, and the residue was subjected to silica gel column chromatography (with dichloromethane and methanol volume ratio 20:1 as elution solvents) to obtain CB03 in the form of a white solid (7.5 g).

4) Synthesis of (9*H*-fluoren-9-yl)methyl ((30*S*,33*S*, 36*S*)-41-amino-36-((4-(hydroxymethyl)phenyl)carbamoyl)-33-isopropyl-26,31,34,41-tetraoxo-2,5,8,11,14,17,20,23-octaoxa-27,32,35,40-tetraaza-30-yl)carbamate (CB04).

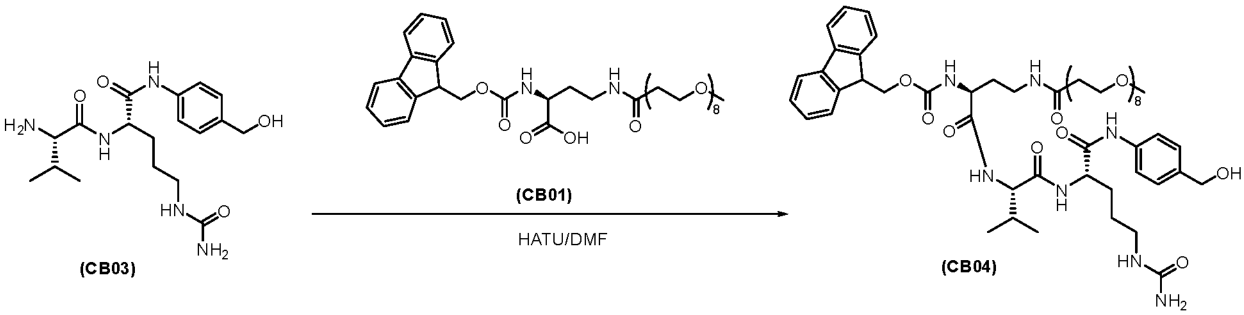

(CB03) (CB01) (CB04)

At 0 °C under a nitrogen atmosphere, 14.2 g of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01) was dissolved in 100 mL of DMF, and 11 g of *N*,*N*,*N'*,*N'*-tetramethyl-*O*-(7-azabenzotriazol-1-yl)urea hexafluorophosphate (HATU) was added in portions. After 30 min of stirring, 7.5 g of (*S*)-2-((*S*)-2-amino-3-methylbutanamido)-N-(4-(hydroxymethyl)phenyl)-5-ureidopentanamide (CB03) was added, and the mixture was allowed to react for 2.5 h with 0 °C maintained. After the completion of the reaction, the mixture was distilled under reduced pressure to remove the solvent, and the residue was subjected to silica gel column chromatography (with dichloromethane and methanol volume ratio 10:1 as elution solvents) to obtain CB04 in the form of a solid (9.66 g).

5) Synthesis of *N*-((*S*)-3-amino-4-(((S)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05).

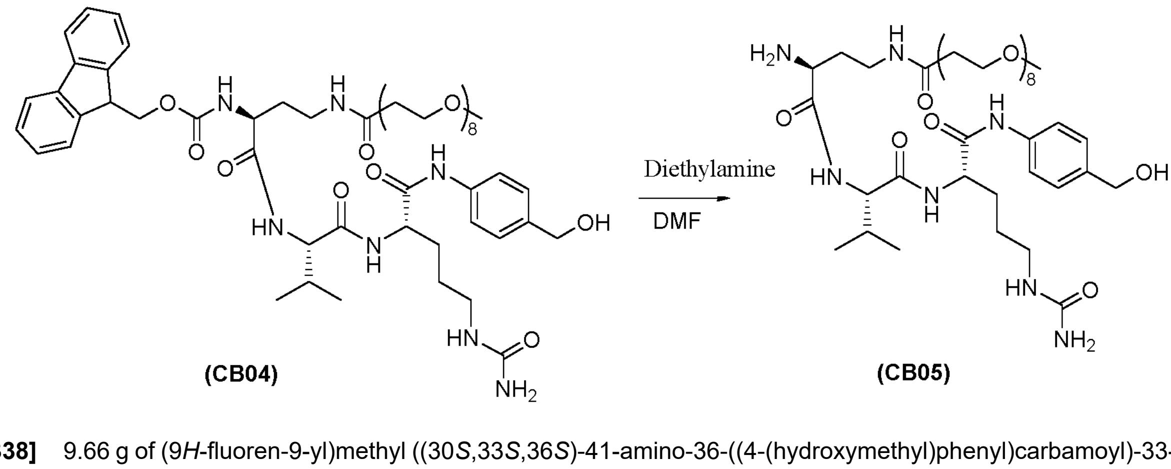

(CB04) (CB05)

9.66 g of (9*H*-fluoren-9-yl)methyl ((30*S*,33*S*,36*S*)-41-amino-36-((4-(hydroxymethyl)phenyl)carbamoyl)-33-isopropyl-26,31,34,41-tetraoxo-2,5,8, 11, 14, 17,20,23-octaoxa-27,32,35,40-tetraaza-30-yl)carbamate (CB04) was dissolved in 50 mL of DMF at room temperature under a nitrogen atmosphere, and 12 mL of diethylamine was added. The mixture was stirred for 1.5 h. After the completion of the reaction, the mixture was distilled under reduced pressure to remove the solvent, and the residue was subjected to silica gel column chromatography (with dichloromethane and methanol volume ratio 7.5:1 as elution solvents) to obtain CB05 in the form of a pale yellow solid (7.7 g).

6) Synthesis of *N*-((*S*)-3-(2-(2,5-dioxo-2,5-dihydro-1*H*-pyrrol-1-yl)acetamido)-4-(((*S*)-1-(((*S*)-1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06).

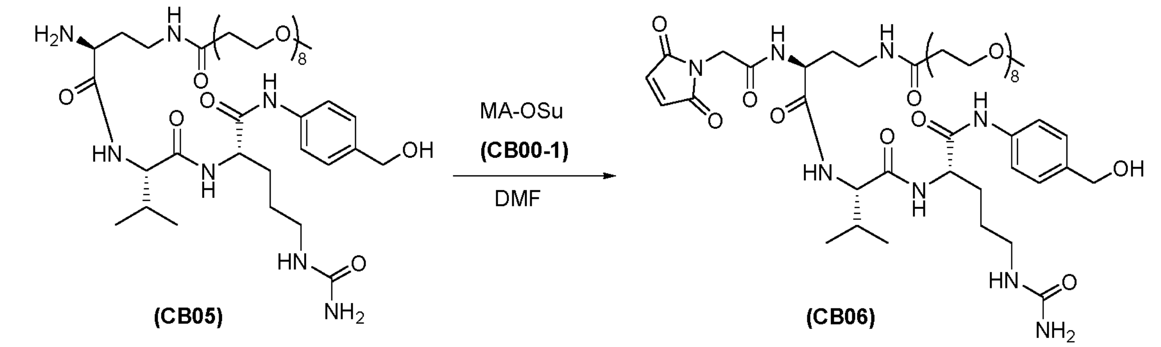


(CB05) (CB06)

7.7 g of *N*-((*S*)-3-amino-4-(((S)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05) was dissolved in 40 mL of DMF, and succinimidyl maleimidoacetate (CB00-1) was added in portions under a nitrogen atmosphere at 0-5°C. The mixture was allowed to react at 0-5°C for 4 h. After the completion of the reaction, the mixture was distilled under reduced pressure to remove the solvent, and the residue was subjected to silica gel column chromatography (with dichloromethane and methanol as volume ratio 10:1 elution solvents) to obtain CB06 in the form of a pale yellow solid (9.5 g).

7) Synthesis of 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07).

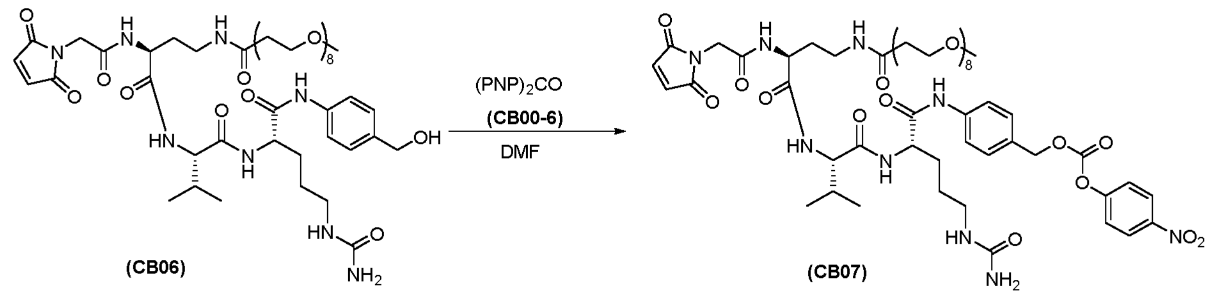


(CB06) (CB07)

9.5 g of *N*-((*S*)-3-(2-(2,5-dioxo-2,5-dihydro-1*H*-pyrrol-1-yl)acetamido)-4-(((*S*)-1-(((S)-1-((4-(hydroxymethyl) phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06) was dissolved in 50 mL of DMF, and 14.0 g of bis(*p*-nitrophenyl)carbonate ($(PNP)_2CO$) was added under a nitrogen atmosphere at 0 °C, followed by 8.2 mL of *N*,*N*-diisopropylethylamine (DIPEA) after dissolution. The mixture was allowed to react for 4 h with the 0 °C maintained. After the completion of the reaction, the mixture was distilled under reduced pressure to remove the solvent, and the residue was subjected to silica gel column chromatography (with dichloromethane and methanol volume ratio 8:1 as elution solvents) to obtain CB07 in the form of a white solid (2.6 g).

**Example 9: Synthesis of 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxa-15,20,23-triazapentacosan-25-amido)benzyl (4-nitrophenyl) carbonate (CB14).**

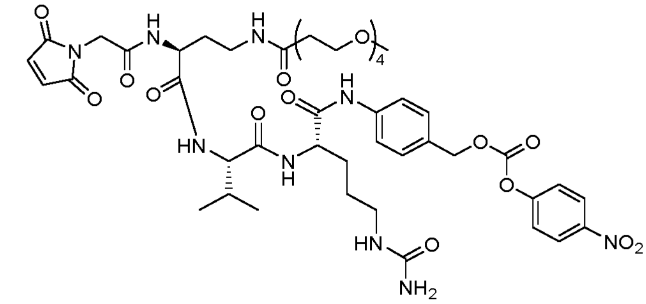

(CB14)

CB14 was prepared by referring to the synthesis of CB07 in Example 8, with *N*-succinimidyl 4,7,10,13,16,19,22,25-octaoxahexacosanoate replaced by *N*-succinimidyl 4,7,10,13-tetraoxatetradecanoate. CB14 was eventually obtained in the form of a white solid.

**Example 10: Synthesis of Intermediate (CB07-Exatecan)**

Synthesis of 4-((30*S*,33*S*,36*S*)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl) carbamate.

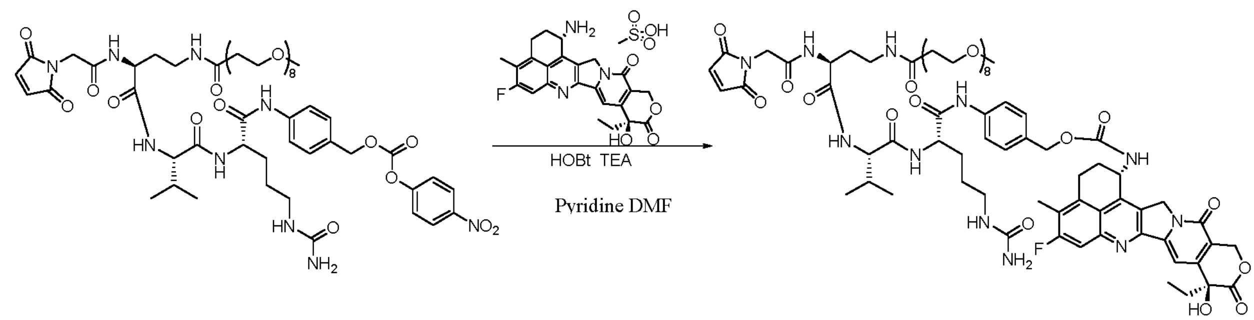


4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07) (2.6 g, 2.21 mmol) and N,N-dimethylformamide (23 mL) were added to a reaction flask R1, stirred under a nitrogen atmosphere and cooled to 0-5 °C. At the same time, (1S,9S)-1-amino-9-ethyl-5-fluoro-9-hydroxy-4-methyl-1,2,3,9,12,15-hexahydro-10H,13H-benzo[de]pyrano[3',4':6,7]indolizino[1,2-b]quinolin-10,13-dione methanesulfonate (Exatecan methane sulfonate, 0.98 g, 1.84 mmol, Advanced ChemBlocks) and N,N-dimethylformamide (5 mL) were taken and added to another reaction flask R2. Triethylamine (230 mg, 2.27 mmol) was added dropwise at 0-5 °C. The mixture was stirred until complete dissolution was achieved. The solution in reaction flask R2 was added dropwise to reaction flask R1. Reaction flask R2 was then washed with N,N-dimethylformamide (2 mL), and the washing solution was added to reaction flask R1. 1-Hydroxybenzotriazole (497 mg, 3.68 mmol) and pyridine (1.45 g, 18.4 mmol) were weighed into reaction flask R1. The mixture was stirred at 0-5°C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder (1.6 g, 59%). LC-MS: $[1/2M+H]^+$=737.

**Example 11: Synthesis of Intermediate (CB07-D-1)**

Synthesis of 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1S,9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)carbamat.

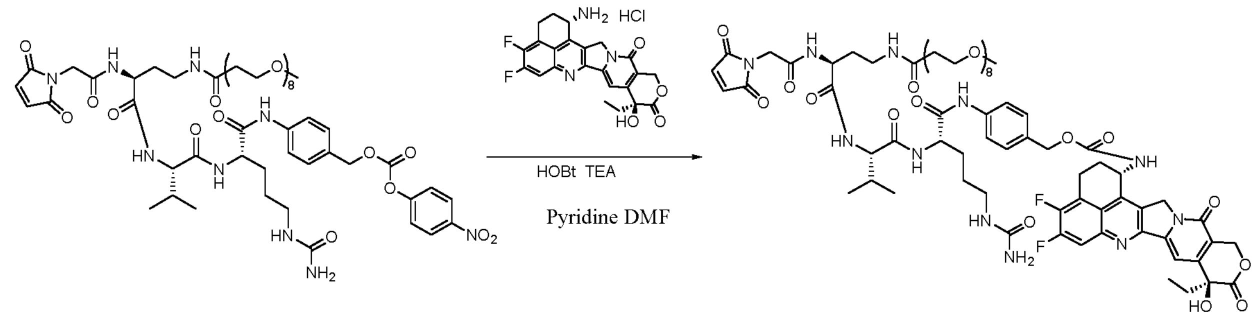


4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07) (220 mg, 0.189 mmol) and *N*,*N*-dimethylformamide (5 mL) were added to a reaction flask R1, stirred under a nitrogen atmosphere and cooled to 0-5 °C. At the same time, (1*S,*9*S*)-1-amino-9-ethyl-4,5-difluoro-9-hydroxy-1,2,3,9,12,15-hexahydro-10*H*,13*H*-benzo[*de*]pyrano[3',4':6,7]indolizino[1,2-b]quinolin-10,13-dione hydrochloride (D-1) (90 mg, 0.189 mmol) and *N*,*N*-dimethylformamide (5 mL) were taken and added to another reaction flask R2. Three drops of triethylamine were added at 0-5 °C. The mixture was stirred until complete dissolution was achieved. The solution in reaction flask R2 was added dropwise to reaction flask R1, and 1-hydroxybenzotriazole (60 mg, 0.44 mmol) and pyridine (0.5 mL) were weighed into reaction flask R1. The mixture was stirred at 0-5°C for 10 min, warmed to room temperature, and stirred for 3 h. After the reaction was completed, the mixture was concentrated under reduced pressure to remove the solvent. The residue was purified by preparative high performance liquid chromatography (prep-HPLC) and lyophilized to obtain a white powder (55 mg, 20%). LC-MS: $[1/2M+H]^+$=739.

**Example 12: Synthesis of Intermediate (CB14-Exatecan)**

Synthesis of 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxa-15,20,23-triazapentacosan-25-amido)benzyl ((1*S*,9,*S*)-9-ethyl-5-fluoro-9-hydroxy-4-methyl-10,13-dioxo-2,3,9,10,13,15-hexahydro-1*H*,12*H*-benzo[de]pyrano[3',4':6,7]indolizino[1,2-b]quinolin-1-yl) carbamate.

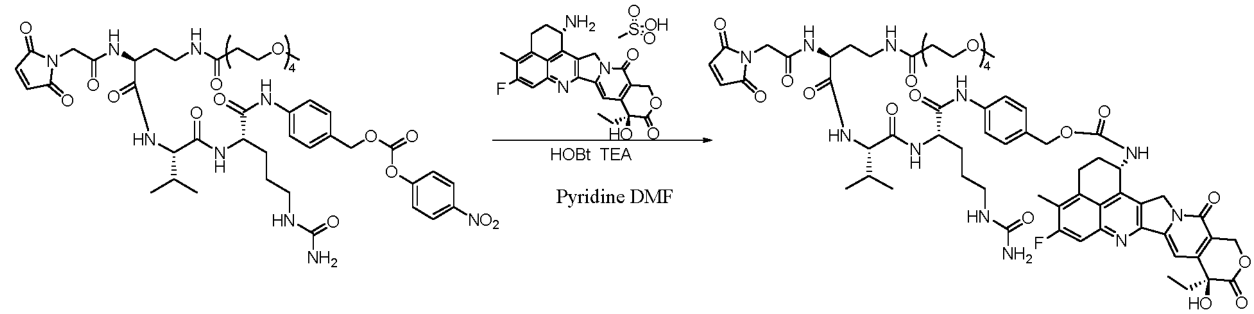


Similarly, 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxa-15,20,23-triazapentacosan-25-amido)benzyl (4-nitrophenyl) carbonate (190 mg, 0.19 mmol)and *N*,*N*-dimethylformamide (23 mL) were added to a reaction flask R1, stirred under a nitrogen atmosphere, and cooled to 0-5 °C. At the same time, (1*S*,9*S*)-1-amino-9-ethyl-5-fluoro-9-hydroxy-4-methyl-1,2,3,9,12,15-hexahydro-10*H*,13*H*-benzo[de]pyrano[3',4':6,7]indolizino[1,2-*b*]quinolin-10,13-dione methanesulfonate (Exatecan methanesulfonate; 101 mg, 0.19 mmol; Advanced ChemBlocks) and N,N-dimethylformamide (5 mL) were taken and added to another reaction flask R2. Triethylamine (3 drops) was added dropwise at 0-5 °C. The mixture was stirred until complete dissolution was achieved. The solution in reaction flask R2 was added dropwise to reaction flask R1. Reaction flask R2 was then washed with *N,N*-dimethylformamide (1 mL), and the washing solution was added to reaction flask R1. 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder.

**Example 13: Preparation of ASG-22-MMAE**

The structural formula of ASG-22-MMAE is as follows

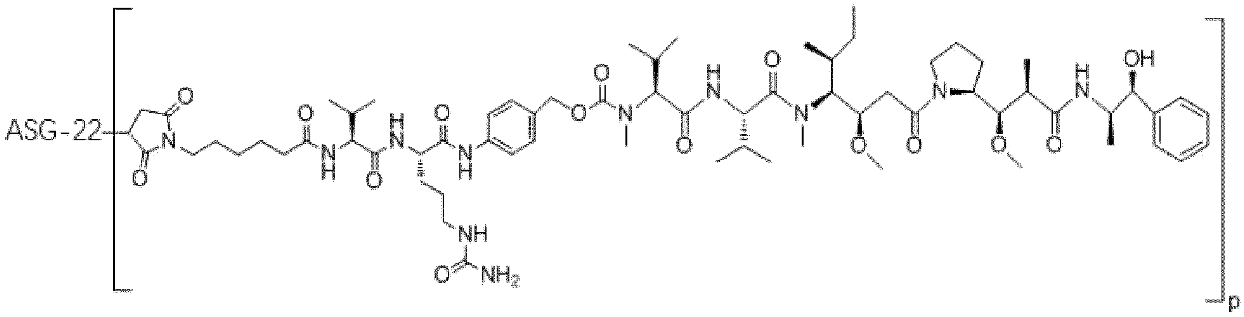

Antibody ASG-22 was adjusted to a concentration of 10 mg/mL using 10 mM succinic acid, and 2.5 molar equivalents of TCEP was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of MC-VC-PAB-MMAE was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value was determined using reversed-phase chromatography, and the DAR value of ASG-22-MMAE was 3.14.

Determination of DAR value:

Procedures

Sample treatment: The test sample was diluted with water to about 1 mg/mL before 0.5 M DTT (dithiothreitol) was added. The sample was reduced in a 37 °C water bath for 30 min and centrifuged at 13,000 g for 5 min, and the supernatant was taken for liquid chromatography analysis.

Instrument conditions: The mobile phases were 0.1% DFA (difluoroacetic acid) in ACN (acetonitrile) and 0.05% DFA in $H_2O$, with a flow rate of 0.5 mL/min. The liquid chromatography separation was performed on a Waters BioResolve column (2.1 × 100 mm, 2.7 μm), and the detection wavelength was set at 214 nm.

Result calculation: After reduction, the sample can be separated by reversed-phase chromatography to give chromatographic peaks of light and heavy chains conjugated to different amounts of drug. The percentage content of each peak was calculated by area normalization, and the drug-to-antibody ratio (DAR) was calculated according to the percentage content of the peaks.

$$\mathrm{DAR} = 2 \times \left(\frac{\mathrm{L1}}{\mathrm{L0} + \mathrm{L1}} + \frac{\mathrm{H1} + 2 \times \mathrm{H2} + 3 \times \mathrm{H3}}{\mathrm{H0} + \mathrm{H1} + \mathrm{H2} + \mathrm{H3}}\right)$$

L0 denotes the percentage content of light chains not conjugated to any small molecule drugs, L1 denotes the percentage content of light chains conjugated to 1 small molecule drug, H0 denotes the percentage content of heavy chains not conjugated to any small molecule drugs, H1 denotes the percentage content of heavy chains conjugated to 1 small molecule drug, H2 denotes the percentage content of heavy chains conjugated to 2 small molecule drugs, and H3 denotes the percentage content of heavy chains conjugated to 3 small molecule drugs.

**Example 14: Preparation of ASG-AM-MMAE**

The structural formula of ASG-AM-MMAE is as follows

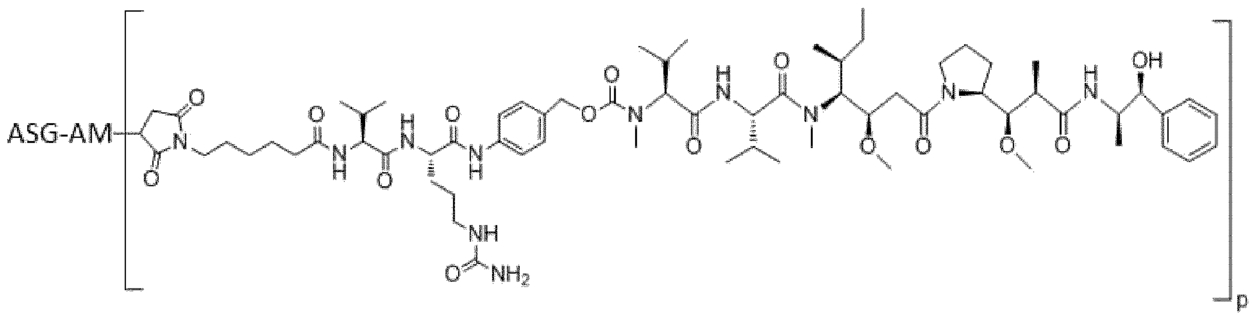

Antibody ASG-AM was adjusted to a concentration of 10 mg/mL using 10 mM succinic acid, and 2.5 molar equivalents of TCEP was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of MC-VC-PAB-MMAE was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of ASG-AM-MMAE was 3.48.

**Example 15: Preparation of ASG-AM-ExaD8**

The structural formula of ASG-AM-ExaD8 is as follows

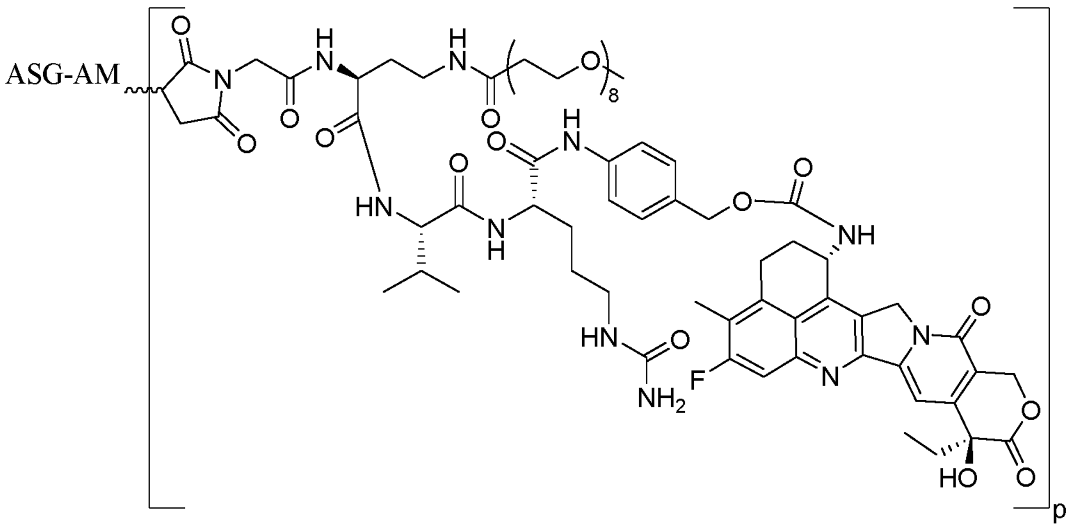

Antibody ASG-AM was adjusted to a concentration of 18 mg/mL using 10 mM succinic acid, and 4.5 molar equivalents of tris(2-carboxyethyl)phosphine) (TCEP) was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 12 molar equivalents of CB07-Exatecan was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of ASG-AM-ExaD8 was determined to be 7.59 by using reversed-phase chromatography.

**Example 16: Preparation of ASG-AM-ExaD4**

The structural formula of ASG-AM-ExaD4 is as follows

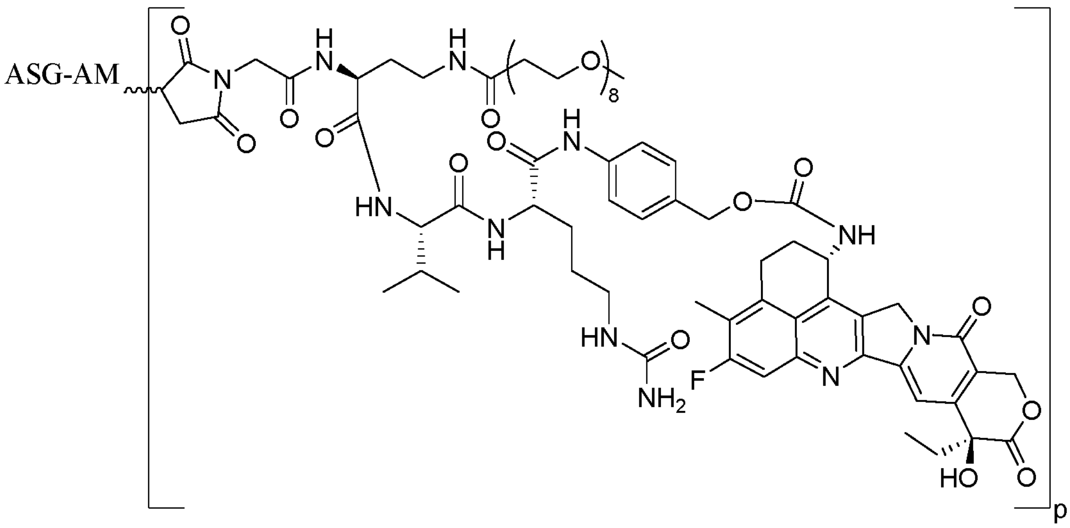

Antibody ASG-AM was adjusted to a concentration of 18 mg/mL using 10 mM succinic acid, and 2.5 molar equivalents of TCEP was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by measuring the absorbance at 412 nm of the reactant of sulfhydryl and DTNB (5,5'-dithiobis(2-nitrobenzoic acid), Aldrich).

In the conjugation reaction, 6 molar equivalents of CB07-Exatecan was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of ASG-AM-ExaD4 was determined to be 4.03 by using reversed-phase chromatography.

**Example 17: Preparation of ASG-AM-Dxd**

The structural formula of ASG-AM-Dxd is as follows

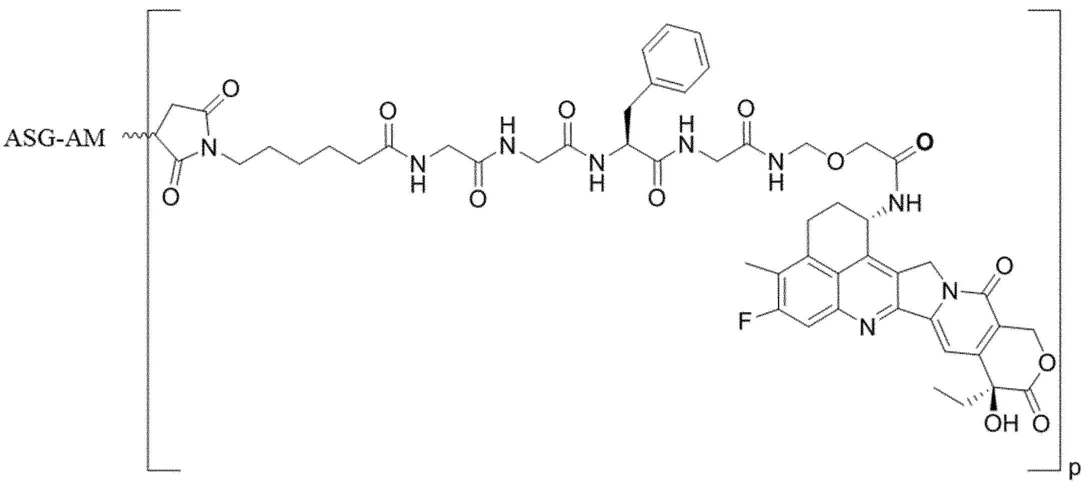

Antibody ASG-AM was adjusted to a concentration of 18 mg/mL using 10 mM succinic acid, and 4.5 molar equivalents of TCEP was added. Then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined

by measuring the absorbance at 412 nm of the reactant of sulfhydryl and DTNB (5,5'-dithiobis(2-nitrobenzoic acid), Aldrich).

In the conjugation reaction, 12 molar equivalents of Deruxtecan (MCE, Cat.#HY-13631E) was added, and after the mixture was stirred at 25 °C for 1 h, 0.1 M acetylcysteine was added until a final concentration of 2 mM. The mixture was stirred for another 15 min, and then the reaction was terminated. The reaction mixture was filtered through a 0.22-micron filter and eluted with 10 mM succinic acid in a Sephadex G-25 resin medium.

The DAR value of ASG-AM-Dxd was determined to be 7.88 by using reversed-phase chromatography.

**Example 18: Comparison of ADC in vitro efficacy**

T-47D cells, OVCAR-3 cells, and MDA-MB-468 cells were used in this experiment. The cells were digested and separately plated on a 96-well plate at a density of 6000 cells/well. After incubation for 4 h in an incubator, the test drugs were added. The test drugs were serially 4-fold diluted from 12.5 μg/mL to a total of 9 concentrations. After a 3-day incubation in the incubator, a relative cell proliferation analysis was conducted using the cell counting kit-8 (CCK-8, Dojindo, Japan) reagents. The results of the experiment (see FIGs. 2A-C) show that ASG-22-MMAE and ASG-AM-MMAE exhibited similar *in vitro* efficacies.

**Example 19: In vitro efficacy of ASG-AM-Dxd**

The ASG-AM-Dxd *in vitro* efficacies on various cells were compared, with reference to the procedures in Example 18. The incubation period after treatment was adjusted to 7 days. The $EC_{50}$ results of the test substance on different cells are shown in Table 6 below.

**Table 6. $EC_{50}$ results of ASG-AM-Dxd on different cells**

| Cell | |
|---|---|
| | $EC_{50}$ (nM) |
| MDA-MB-468 | 63.43 |
| OVCAR3 | 18.85 |
| Jeg3 | 101.20 |

**Example 20: Efficacy of ADCs in MDA-MB-468 xenograft tumor model**

The *in vivo* anti-tumor activity of ADCs was evaluated using a human breast cancer MDA-MB-468 nude mouse subcutaneous xenograft tumor model.

Female nude mice were grafted subcutaneously with MDA-MB-468 tumor cells to establish the MDA-MB-468 nude mouse xenograft tumor model. On day 18 after grafting, when the mean tumor volume reached about 140 $mm^3$, the animals were randomized based on the tumor volume, with 6 groups of 6 mice each. The day of grouping was designated as Day 0. 1 mg/kg and 5 mg/kg treatment groups were set for ASG-22-MMAE and ASG-AM-MMAE. On the day of grouping (Day 0), the drugs were given to the mice alone via intravenous (IV) tail vein injection, with a total of 1 dose. ASG-AM-Dxd was administered at a dose of 5 mg/kg on the day of grouping (Day 0) via intravenous (IV) tail vein injection, with a total of 1 dose. In the solvent control group (normal saline), the solvent was administered alone on the day of grouping via intravenous (IV) tail vein injection, with a total of 1 dose. The results are shown in Table 7 and FIG. 3.

On day 21 post-dose, the mean tumor volume of the solvent control group was 382.85 ± 23.2 $mm^3$, and the experiment ended on Day 21. Compared with the solvent control group, on Day 21, the relative tumor proliferation rates (T/C) for the ASG-22-MMAE treatment groups (1 mg/kg and 5 mg/kg) were 49.82% (TGI = 49.14%, $P < 0.001$) and 17.56% (TGI = 81.91%, $P < 0.001$), respectively.

The relative tumor proliferation rates (T/C) for the ASG-AM-MMAE treatment groups (1 mg/kg and 5 mg/kg) were 53.76% (TGI = 44.70%, $P < 0.001$) and 21.51% (TGI = 78.06%, $P < 0.001$), respectively.

The relative tumor proliferation rate (T/C) for the ASG-AM-Dxd (5 mg/kg) treatment group was 13.98% (TGI = 85.71%, $P < 0.001$).

In this experiment, by Day 21, the mean body weight of the animals in the solvent control group had increased, with an increase of 3.11%. The mean body weight of animals in the low- and high-dose ASG-22-MMAE treatment groups, the low- and high-dose ASG-AM-MMAE treatment groups, and the ASG-AM-Dxd treatment group, all increased, with increases of 1.03%, 2.42%, 3.93%, 2.98%, and 1.46%, respectively. In the experiment, the number of drug-related deaths was 0, and no other drug-related adverse effects were observed.

**Table 7. Effects of ADCs on animal tumor volume in human breast cancer MDA-MB-468 nude mouse xenograft tumor model**

| Group | Number of animals | Test substances | Dose mg/kg | Tumor volume ($mm^3$, Mean ± SEM) | | Tumor volume inhibition rate (%TGI) Day 21 | Relative tumor proliferation rate (%T/C) Day 21 |
|---|---|---|---|---|---|---|---|
| | | | | Day 0 | Day 21 | | |
| 1 | 6 | Normal saline | NA | 140.29 ± 9.02 | 382.85 ± 23.21 | N/A | N/A |
| 2 | 6 | ASG-22-MMAE | 1 | 140.84 ± 9.30 | 194.71 ± 24.5 *** | 49.14 | 49.82 |
| 3 | 6 | ASG-22-MMAE | 5 | 140.47 ± 8.46 | 69.26 ± 28.28 *** | 81.91 | 17.56 |
| 4 | 6 | ASG-AM-MMAE | 1 | 139.95 ± 8.39 | 211.73 ± 26.40*** | 44.70 | 53.76 |
| 5 | 6 | ASG-AM-MMAE | 5 | 140.58 ± 7.53 | 84.00 ± 9.09 *** | 78.06 | 21.51 |
| 6 | 6 | ASG-AM-Dxd | 5 | 138.28 ± 5.85 | 54.76 ± 9.12*** | 85.71 | 13.98 |

Note: 1. Day 0 denotes the day of grouping and treatment; 2. *: $P < 0.05$ compared with the vehicle control group; **: $P < 0.01$ compared with the vehicle control group; ***: $P < 0.001$ compared with the vehicle control group.

**Example 21: Pharmacological evaluation in JEG-3 cell subcutaneous xenograft tumors Objective:**

This is a preclinical pharmacodynamic study to evaluate the efficacy of test drugs ASG-AM-ExaD4, ASG-AM-ExaD8, and ASG-AM-Dxd in a human choriocarcinoma JEG-3 cell subcutaneous xenograft female NOD/SCID mouse animal model.

**Design**

The study included 5 groups, with 6 animals in each group, and the treatment was started on the day of the grouping. The detailed study design is shown in Table 8 below.

**Table 8. Administration routes, dosages, and regimens in human choriocarcinoma JEG-3 subcutaneous xenograft model**

| Group | Number of animals | Treatment | Dose (mg/kg) | Route of admmistration | Dose volume (μL/g) | Treatment cycle |
|---|---|---|---|---|---|---|
| 1 | 6 | Vehicle control | -- | *i. v.* | 10 | Single dose |
| 2 | 6 | ASG-AM-ExaD4 | 5 | *i.v.* | 10 | Single dose |
| 3 | 6 | ASG-AM-ExaD4 | 8 | *i.v.* | 10 | Single dose |
| 4 | 6 | ASG-AM-ExaD8 | 5 | *i.v.* | 10 | Single dose |
| 5 | 6 | ASG-AM-Dxd | 5 | *i.v.* | 10 | Single dose |

Note: The day of grouping was Day 0, and the treatment was started on Day 0.

**Procedures**

JEG-3 cells were cultured in a MEM medium containing 10% fetal bovine serum (supplemented with 0.01 mM NEAA). JEG-3 cells in the exponential phase were collected and resuspended in PBS to a proper concentration for subcutaneous tumor grafting in mice.

$1 \times 10^7$ JEG-3 cells resuspended in a 1:1 mixture of PBS and matrigel were grafted in the mice subcutaneously at the right dorsum (0.2 mL/mouse). The growth of the tumors was observed periodically. When the mean volume of the tumors reached 129.56 $mm^3$, the mice were randomized by tumor size and the body weight for treatment. The day of grouping was designated as Day 0, and the treatment was started on Day 0.

**Results**

There was no significant abnormality or weight loss in all groups of mice during the study.

The tumor growth in the treatment groups and the control group of the JEG-3 xenograft model is shown in Table 9 below and FIG. 4. After a single dose in the treatment groups, significantly inhibited tumor growth was observed, with the efficacy of ASG-AM-ExaD4 and ASG-AM-ExaD8 being significantly superior to that of ASG-AM-Dxd.

**Table 9. Efficacy analysis for each group in JEG-3 xenograft model**

| Group | Mean tumor volume (Day 0)[a] | Mean tumor volume (Day 13)[a] | TGI (%)[b] | *P* Value relative to control group)[c] |
|---|---|---|---|---|
| **Group** 1<br>Vehicle, 0 mg/kg, single dose, i.v. | 129.61±7.16 | 2072.05±230.27 | | - |
| **Group 2**<br>ASG-AM-ExaD4, 5 mg/kg | 129.65±7.61 | 834.00±243.25 | 63.74% | 6.59e-03** |
| **Group 3**<br>ASG-AM-ExaD4, 8 mg/kg | 129.06±7.39 | 589.11±160.61 | 76.32% | 5.69e-04*** |
| **Group 4**<br>ASG-AM-ExaD8, 5 mg/kg | 129.73±7.27 | 154.84±23.28 | 98.71% | 1.05e-07*** |
| **Group 5**<br>ASG-AM-Dxd, 5 mg/kg | 129.73±7.29 | 1358.51±206.11 | 36.74% | 3.87e-01[ns] |

Note: a. Data were expressed in "mean ± standard error";
b. TGI% = [1 - (Ti - T0)/(Ci - C0)] × 100, where T0 and C0 are the mean tumor volumes of the treatment group and vehicle control group on the day of grouping (Day 0), respectively, and Ti and Ci are the mean tumor volumes of the treatment group and vehicle control group on Day 13, respectively;
c. *$P$ < 0.05, **$P$ < 0.01, and ***$P$ < 0.001 compared with the tumor volume of the vehicle control

**Claims**

1. An antibody or an antigen-binding unit, wherein the antibody or the antigen-binding unit specifically binds to Nectin-4 and comprises one or more of (a)-(f):

   (a) a VH CDR1 comprising the amino acid sequence set forth in SEQ ID NO: 2;
   (b) a VH CDR2 comprising the amino acid sequence set forth in SEQ ID NO: 3;
   (c) a VH CDR3 comprising the amino acid sequence set forth in SEQ ID NO: 4;
   (d) a VL CDR1 comprising the amino acid sequence set forth in SEQ ID NO: 5;
   (e) a VL CDR2 comprising the amino acid sequence set forth in SEQ ID NO: 6; and
   (f) a VL CDR3 comprising the amino acid sequence set forth in SEQ ID NO: 7.

2. The antibody or the antigen-binding unit according to claim 1, comprising: a VL CDR3 set forth in SEQ ID NO: 7, and optionally, one or more of a VH CDR1 set forth in SEQ ID NO: 2, a VH CDR2 set forth in SEQ ID NO: 3, a VH CDR3 set forth in SEQ ID NO: 4, a VL CDR1 set forth in SEQ ID NO: 5, and a VL CDR2 set forth in SEQ ID NO: 6.

3. The antibody or the antigen-binding unit according to claim 1 or 2, comprising: a VL CDR1 set forth in SEQ ID NO: 5, a

VL CDR2 set forth in SEQ ID NO: 6, and a VL CDR3 set forth in SEQ ID NO: 7.

**4.** The antibody or the antigen-binding unit according to any one of claims 1-3, comprising: a VH CDR1 set forth in SEQ ID NO: 2, a VH CDR2 set forth in SEQ ID NO: 3, a VH CDR3 set forth in SEQ ID NO: 4, a VL CDR1 set forth in SEQ ID NO: 5, a VL CDR2 set forth in SEQ ID NO: 6, and a VL CDR3 set forth in SEQ ID NO: 7.

**5.** The antibody or the antigen-binding unit according to any one of claims 1-4, comprising: a heavy chain variable region and/or a light chain variable region, wherein

the heavy chain variable region comprises the amino acid sequence set forth in SEQ ID NO: 8, an amino acid sequence having at least 80%, at least 90%, at least 95%, at least 96%, at least 97%, at least 98%, or at least 99% sequence identity to the amino acid sequence set forth in SEQ ID NO: 8, or an amino acid sequence having one or more conservative amino acid substitutions compared with the amino acid sequence set forth in SEQ ID NO: 8; and/or
the light chain variable region comprises the amino acid sequence set forth in SEQ ID NO: 10, an amino acid sequence having at least 80%, at least 90%, at least 95%, at least 96%, at least 97%, at least 98%, or at least 99% sequence identity to the amino acid sequence set forth in SEQ ID NO: 10, or an amino acid sequence having one or more conservative amino acid substitutions compared with the amino acid sequence set forth in SEQ ID NO: 10.

**6.** An antibody or an antigen-binding unit, comprising a heavy chain variable region and a light chain variable region, wherein the heavy chain variable region comprises the amino acid sequence set forth in SEQ ID NO: 8, and the light chain variable region comprises the amino acid sequence set forth in SEQ ID NO: 10.

**7.** The antibody or the antigen-binding unit according to any one of claims 1-6, further comprising a heavy chain constant region and/or a light chain constant region, wherein alternatively, the heavy chain constant region is selected from IgG1, IgG2, IgG3, and IgG4 types, and the light chain constant region is a $\lambda$ chain or $\kappa$ chain constant region.

**8.** The antibody or the antigen-binding unit according to claim 7, wherein the heavy chain constant region comprises the amino acid sequence set forth in SEQ ID NO: 9, an amino acid sequence having at least 80%, at least 90%, at least 95%, at least 96%, at least 97%, at least 98%, or at least 99% sequence identity to the amino acid sequence set forth in SEQ ID NO: 9, or an amino acid sequence having one or more conservative amino acid substitutions compared with the amino acid sequence set forth in SEQ ID NO: 9; and/or
the light chain constant region comprises the amino acid sequence set forth in SEQ ID NO: 11, an amino acid sequence having at least 80%, at least 90%, at least 95%, at least 96%, at least 97%, at least 98%, or at least 99% sequence identity to the amino acid sequence set forth in SEQ ID NO: 11, or an amino acid sequence having one or more conservative amino acid substitutions compared with the amino acid sequence set forth in SEQ ID NO: 11.

**9.** An antibody, wherein the heavy chain of the antibody comprises the amino acid sequence set forth in SEQ ID NO: 12, and the light chain of the antibody comprises the amino acid sequence set forth in SEQ ID NO: 14.

**10.** A biomaterial, wherein the biomaterial is selected from:

(1) a nucleic acid encoding the antibody or the antigen-binding unit according to any one of claims 1-9;
(2) a vector, a host cell, or a microorganism comprising (1); and
(3) an expression product, a suspension, or a supernatant of (2) described above.

**11.** A method for preparing the antibody or the antigen-binding unit according to any one of claims 1-9, comprising: culturing the host cell according to claim 10 to express the antibody or the antigen-binding unit according to any one of claims 1-9, and isolating the antibody or the antigen-binding unit from the host cell.

**12.** An antibody-drug conjugate, or a pharmaceutically acceptable salt or solvate thereof, comprising: the antibody or the antigen-binding unit according to any one of claims 1-9 conjugated to a drug via a linker.

**13.** An antibody-drug conjugate having a structure shown in Formula I-A, Formula I-B or Formula I-C or a stereoisomer thereof, or a pharmaceutically acceptable salt or solvate thereof:

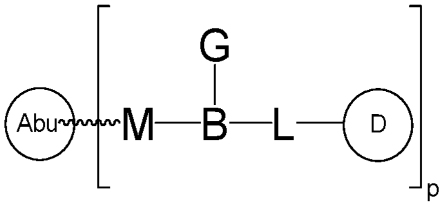

Formula I-A

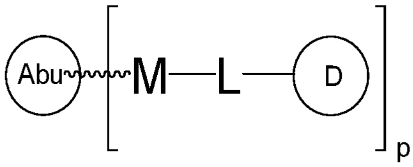

Formula I-B

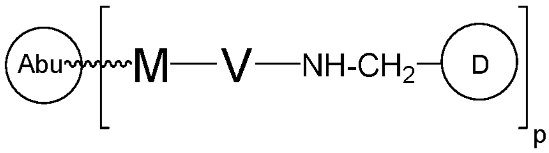

Formula I-C

wherein Abu is the antibody or the antigen-binding unit according to any one of claims 1-9; D is a drug;
M is

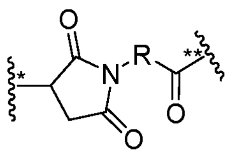

,

R is selected from $-(CH_2)_r-$, $-(CHR^m)_r-$, C3-C8 carbocyclyl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, - $(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, - $(CH_2)_rC(O)NR^m(CH_2CH_2O)_r-CH_2-$, $-(CH_2CH_2O)_rC(O)NR^m(CH_2CH_2O)_r-$, - $(CH_2CH_2O)_rC(O)NR^{TM}(CH_2CH_2O)_r-CH_2-$, and $-(CH_2CH_2O)_rC(O)NR^m(CH_2)_r-$; wherein each $R^m$ is independently H, C1-C6 alkyl, C3-C8 carbocyclyl, phenyl, or benzyl; and each r is independently 1, 2, 3, 4, 5, 6, 7, 8, 9, or 10; in Formula I-A, * links to Abu, and ** links to B; in Formula I-B, * links to Abu, and ** links to L; in Formula I-C, * links to Abu, and ** links to V;
B is

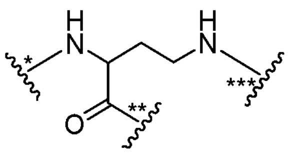

,

wherein * links to M, ** links to L, and *** links to G;
L is $-(AA)_i-(FF)_f-$, wherein AA is an amino acid or polypeptide, and i is 1, 2, 3, 4, 5, 6, 7, 8, 9, 10, 11, 12, 13, 14, 15, 16, 17, 18, 19, or 20; each FF is independently

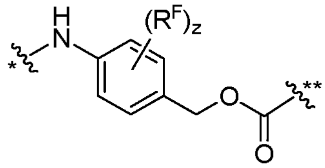

,

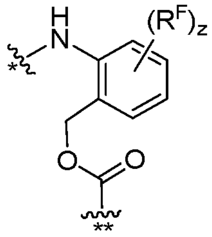

, or

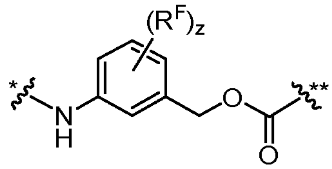

,

wherein each $R^F$ is independently C1-C6 alkyl, C1-C6 alkoxy, $-NO_2$, or halogen, wherein * links to AA, and ** links to D, and z is 0, 1, 2, 3, or 4; f is 1, 2, 3, 4, 5, 6, 7, 8, 9, or 10;
G is

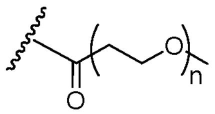

,

wherein n is 1-24;
V is

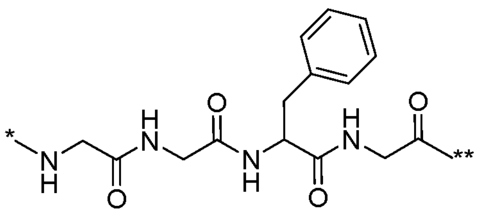

or

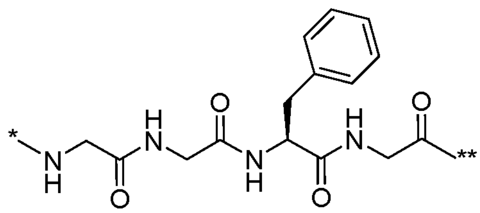

,

wherein * links to M, and ** links to $-NH-CH_2-$;
p is 1-10.

**14.** The antibody-drug conjugate or the pharmaceutically acceptable salt or solvate thereof according to claim 13, wherein B is

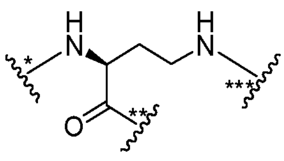

,

, wherein * links to M, ** links to L, and *** links to G.

**15.** The antibody-drug conjugate or the pharmaceutically acceptable salt or solvate thereof according to claim 13 or 14, wherein each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

**16.** The antibody-drug conjugate or the pharmaceutically acceptable salt or solvate thereof according to claim 15, wherein

AA is Val-Cit.

**17.** The antibody-drug conjugate or the pharmaceutically acceptable salt or solvate thereof according to any one of claims 13-16, wherein i is 1.

**18.** The antibody-drug conjugate or the pharmaceutically acceptable salt or solvate thereof according to any one of claims 13-17, wherein each FF is independently

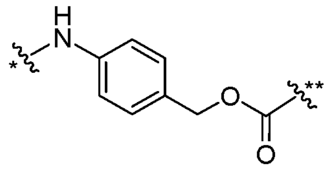,

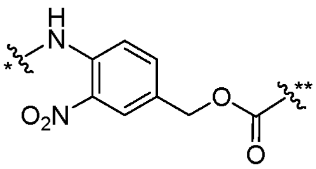, 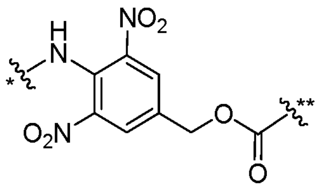, 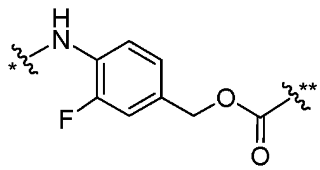,

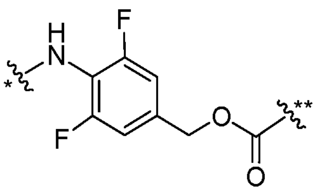, 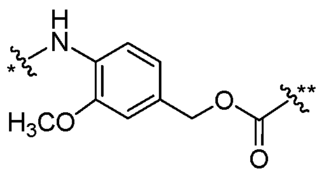, 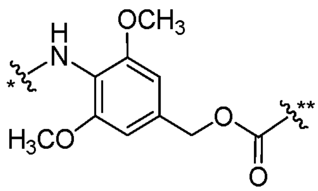, 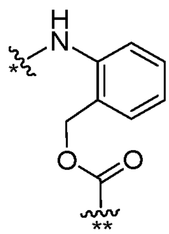,

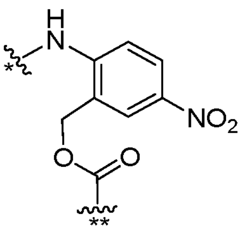, 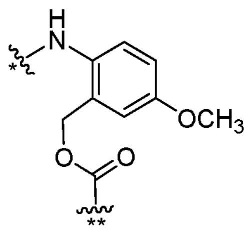, 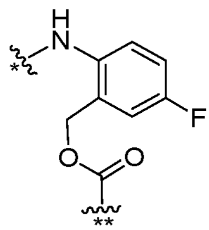, 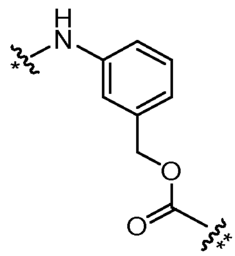,

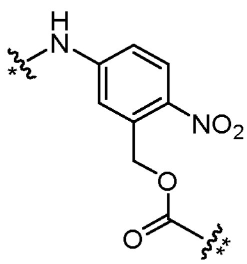, 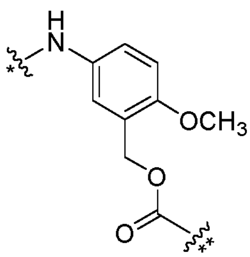, or 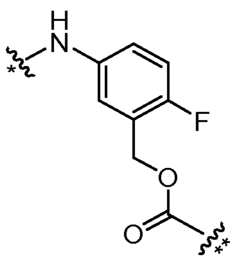,

wherein * links to AA, and ** links to D.

**19.** The antibody-drug conjugate or the pharmaceutically acceptable salt or solvate thereof according to claim 18, wherein FF is

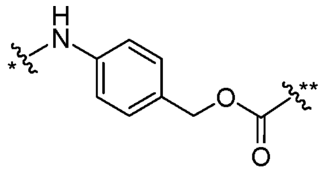

,

wherein * links to AA, and ** links to D.

**20.** The antibody-drug conjugate or the pharmaceutically acceptable salt or solvate thereof according to any one of claims 13-19, wherein f is 1.

**21.** The antibody-drug conjugate or the pharmaceutically acceptable salt or solvate thereof according to any one of claims 13-20, wherein L is

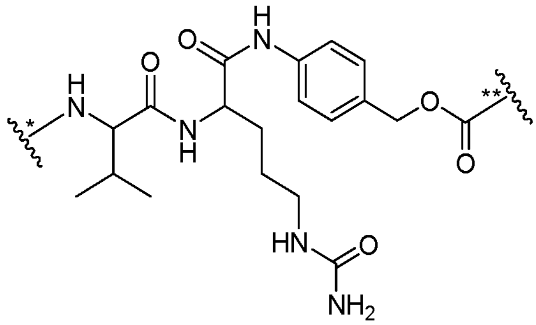

or

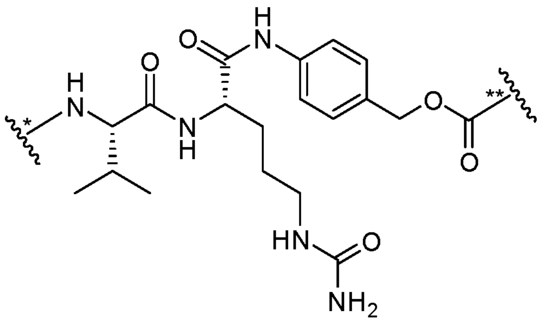

,

wherein in Formula I-A, * links to B, and ** links to D; in Formula I-B, * links to M, and ** links to D.

**22.** An antibody-drug conjugate having a structure shown in Formula I-A-1 or I-A-2 or a stereoisomer thereof, or a pharmaceutically acceptable salt or solvate thereof, wherein the Formulas I-A-1 and I-A-2 are:

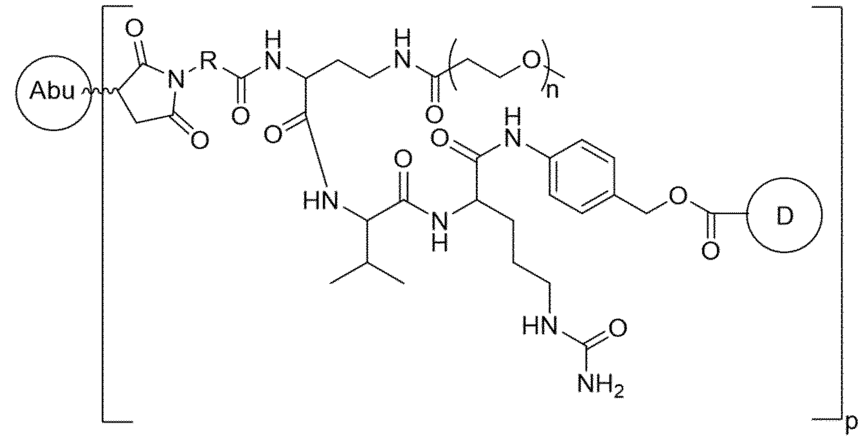

Formula I-A-1

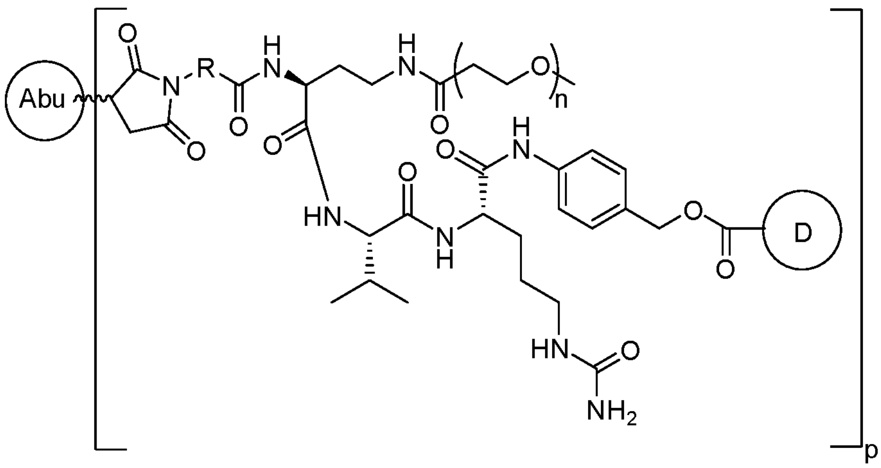

Formula I-A-2

wherein

Abu is the antibody or the antigen-binding unit according to any one of claims 1-9;
R is selected from: $-(CH_2)_r-$, $-(CHR^m)_r-$, C3-C8 carbocyclyl, $-O-(CH_2)_r-$, arylene, $-(CH_2)_r$-arylene-, - arylene-$(CH_2)$ r-, $-(CH_2)_r$-(C3-C8 carbocyclyl)-, -(C3-C8 carbocyclyl)-$(CH_2)_r$-, C3-C8 heterocyclyl, - $(CH_2)_r$-(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_{2O})_r-CH_2-$, $-(CH_2)$,C(O) $NR^m(CH_2CH_2O)$,-, $-(CH_2)$,C(O)$NR^m(CH_2CH_2O)$,-$CH_2$-, - $(CH_2CH_2O)_rC(O)NR^m(CH_2CH_2O)_r-$, $-(CH_2CH_2O)_{r-}$ $C(O)NR^m(CH_2CH_2O)_r-CH_2-$, and - $(CH_2CH_2O)_rC(O)NR^m(CH_2)_r-$, wherein each $R^m$ is independently H, C1-C6 alkyl, C3-C8 carbocyclyl, phenyl, or benzyl, and each r is independently 1, 2, 3, 4, 5, 6, 7, 8, 9, or 10;
D is a drug;
n is an integer of 1-24;
p is 1-10.

**23.** The antibody-drug conjugate, the pharmaceutically acceptable salt thereof, or the solvate thereof according to any one of claims 13-22, wherein R is $-(CH_2)_r-$, and r is 1 or 5.

**24.** An antibody-drug conjugate having a structure shown in Formula I-A-3 or I-A-4 or a stereoisomer thereof, or a pharmaceutically acceptable salt or solvate thereof, wherein the Formulas I-A-3 and I-A-4 are:

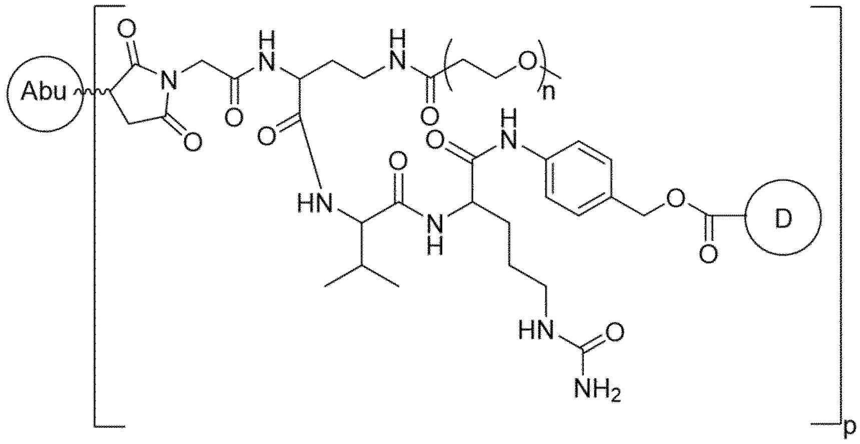

Formula I-A-3

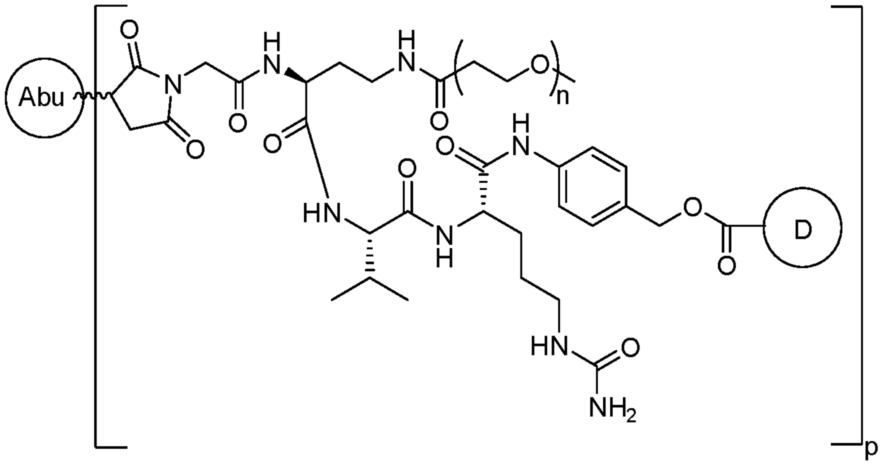

Formula I-A-4

wherein

Abu is the antibody or the antigen-binding unit according to any one of claims 1-9;
D is a drug;
n is an integer of 1-24;
p is 1-10.

**25.** The antibody-drug conjugate or the pharmaceutically acceptable salt or solvate thereof according to any one of claims 13-24, wherein n is 4-12; or n is 4-8; or n is 4 or 8.

**26.** An antibody-drug conjugate having a structure shown in Formula I-A-5, I-A-6, I-A-7, I-A-8, I-A-9, I-A-10 or I-A-11 or a stereoisomer thereof, or a pharmaceutically acceptable salt or solvate thereof, wherein the Formulas I-A-5, I-A-6, I-A-7, I-A-8, I-A-9, I-A-10, and I-A-11 are:

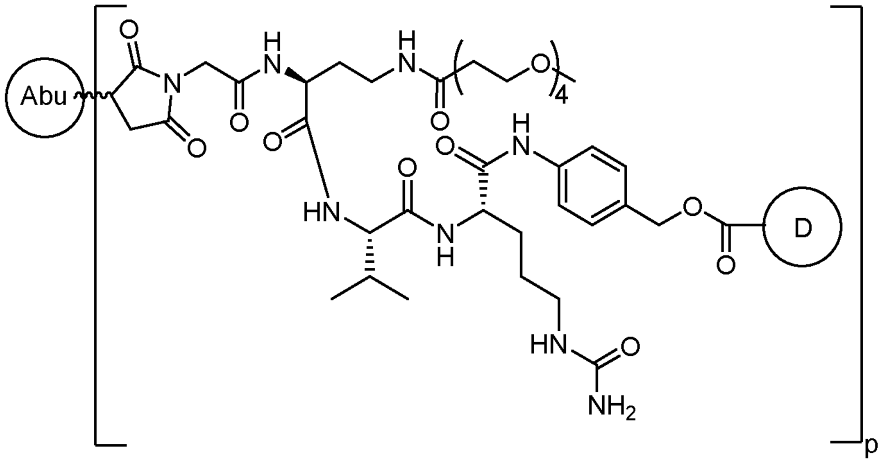

Formula I-A-5

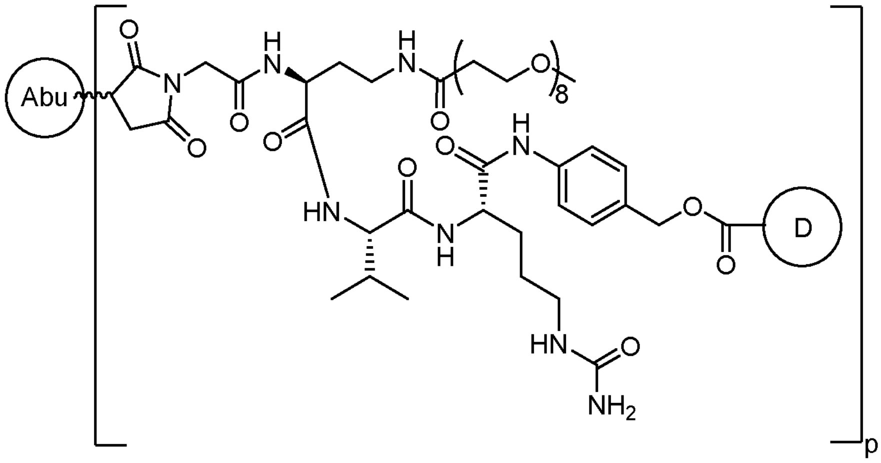

Formula I-A-6

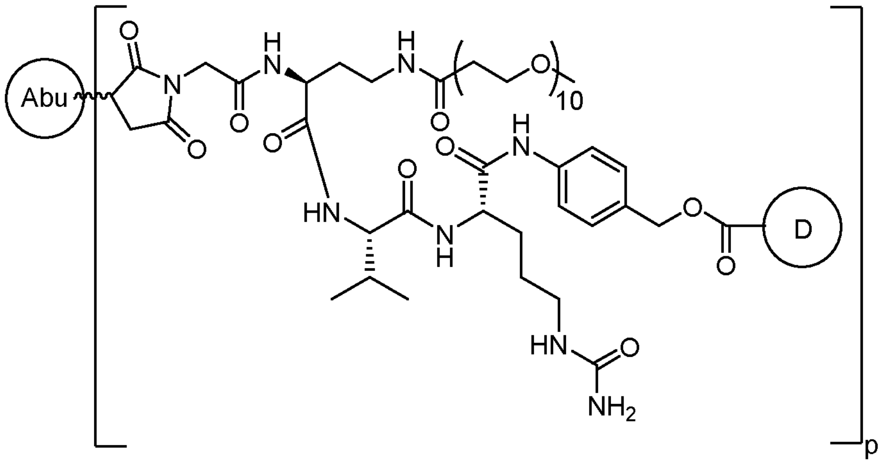

Formula I-A-7

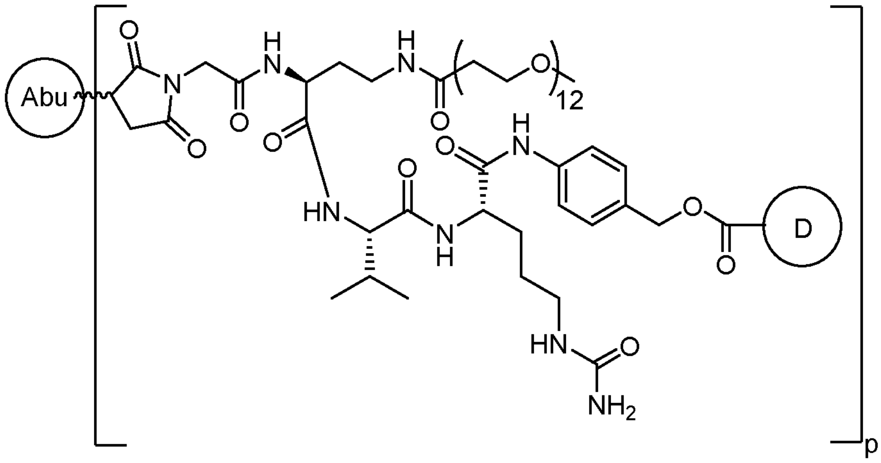

Formula I-A-8

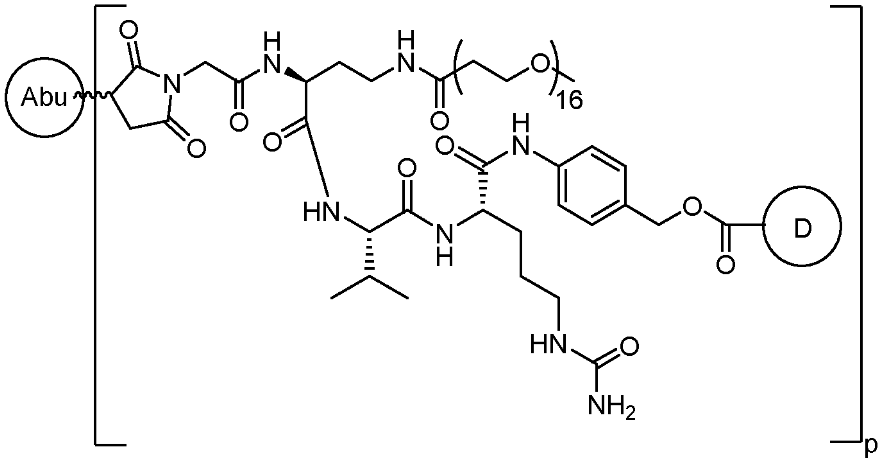

Formula I-A-9

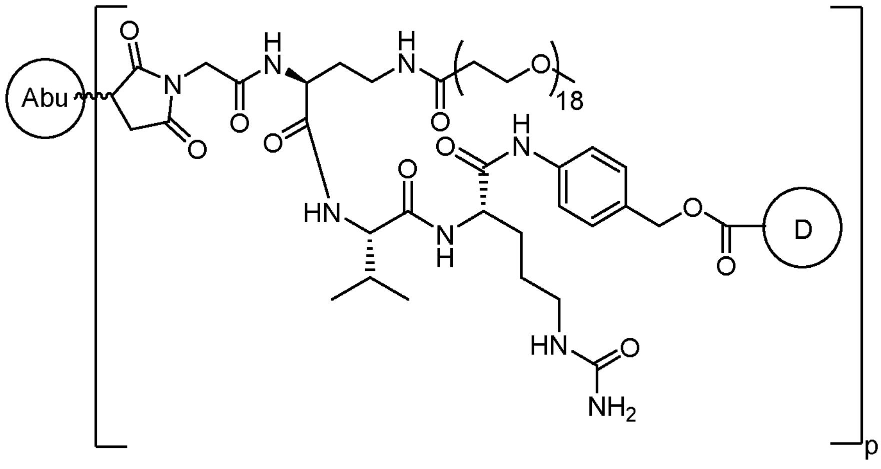

or

Formula I-A-10

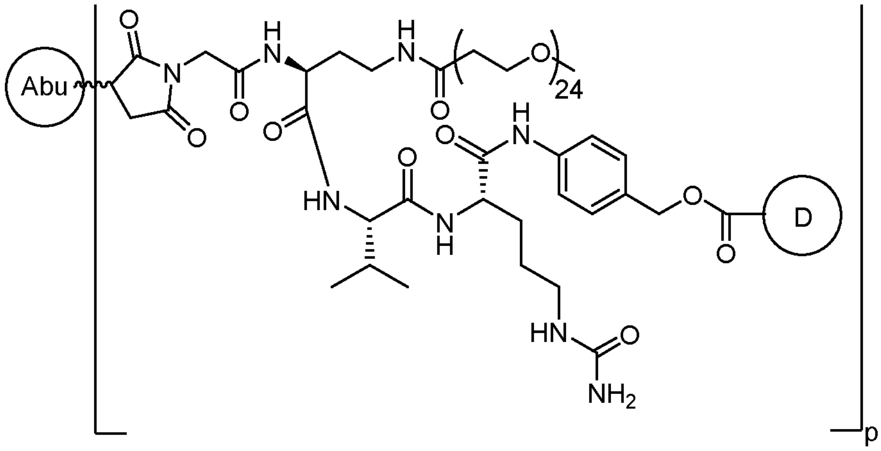

Formula I-A-11

wherein

Abu is the antibody or the antigen-binding unit according to any one of claims 1-9;
D is a drug;
p is 1-10.

**27.** The antibody-drug conjugate or the pharmaceutically acceptable salt or solvate thereof according to any one of claims 12-26, wherein the drug is an anti-cancer drug, a cytotoxic drug, a cell differentiation factor, a stem cell trophic factor, a steroid drug, a drug for treating autoimmune diseases, an antiinflammatory drug, or a drug for treating an infectious disease; or the drug is an anti-cancer drug; or the drug is a tubulin inhibitor, a DNA damaging agent, or a DNA topoisomerase inhibitor; or the tubulin inhibitor is selected from dolastatin, an auristatin, and a maytansine; or the drug is an auristatin selected from MMAE, MMAF, and AF; or the drug is a DNA damaging agent selected from a calicheamicins, a duocarmycin, and anthramycin derivative PBD (pyrrolobenzodiazepine); or the drug is a DNA topoisomerase inhibitor or a salt thereof selected from irinotecan, irinotecan hydrochloride, camptothecin, 9-aminocamptothecin, 9-nitrocamptothecin, 10-hydroxycamptothecin, 9-chloro-10-hydroxycamptothecin, camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride, and *N*-[2-(dimethylamino)ethyl]-4-acridinecarboxamide; or the DNA topoisomerase inhibitor is camptothecin, 10-hydroxycamptothecin, topotecan, belotecan, irinotecan, 22-hydroxyacuminatine, or exatecan.

**28.** The antibody-drug conjugate or the pharmaceutically acceptable salt or solvate thereof according to any one of claims 12-26, wherein the drug is

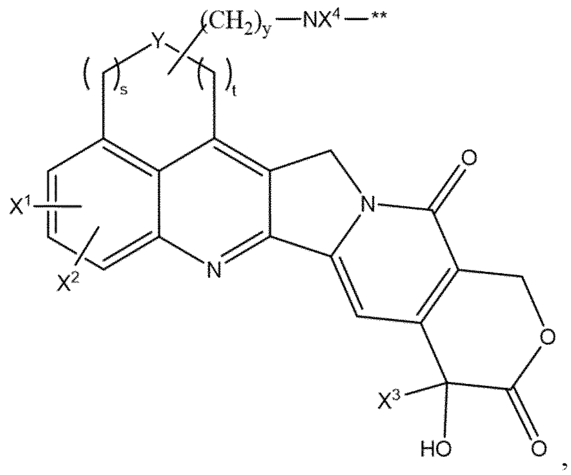

wherein
$X^1$ and $X^2$ are each independently:

H,
hydroxy,
C1-C6 alkyl,
C1-C6 alkyl substituted with one or more hydroxy, halogen, nitro, or cyano groups,
C2-C6 alkenyl,
C2-C6 alkynyl,
C1-C6 alkoxy,
C1-C6 aminoalkoxy,
halogen,
nitro,
cyano,
sulfhydryl,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclyl, wherein the heterocyclyl is optionally substituted with one or more C1-C6 alkyl, C1-C6 alkoxy, amino, halogen, nitro, or cyano groups,
C1-C6 alkylamino linking to a heterocyclyl, wherein the heterocyclyl is optionally substituted with C1-C6 alkyl or C1-C6 alkoxy, and the amino is optionally substituted with an amino-protecting group, halogen, nitro, cyano, or a protecting group,
amino-substituted heterocyclyl optionally substituted at a nitrogen atom of the heterocyclic moiety or at the amino moiety with a protecting group or one or more C1-C6 alkyl groups,
heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl, morpholin-1-yl, or piperidin-1-yl;
$X^3$ is C1-C6 alkyl;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, - $(CH_2)_qC(O)NR^n(CH_2)_q-CH_3$, $-(CH_2CH_2O)_q-CH_3$, $-(CH_2CH_2O)_q-CH_2-CH_3$, - $(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$, or - $(CH_2cH_2O)_qC(O)NR^n(CH_2)_q-CH_3$, wherein each $R^n$ is independently H, C1-C6 alkyl, C3-C8 carbocyclyl, phenyl, or benzyl, and each q is independently 1, 2, 3, 4, 5, 6, 7, 8, 9, or 10; or $X^4$ is H or C1-C6 alkyl;
** is a linking point;

y is 0, 1, or 2;
Y is O, S, or $CR^{1D}R^{2D}$, wherein $R^{1D}$ and $R^{2D}$ are each independently H or C1-C6 alkyl;
s and t are each independently 0, 1, or 2, but not both 0;
or the drug is

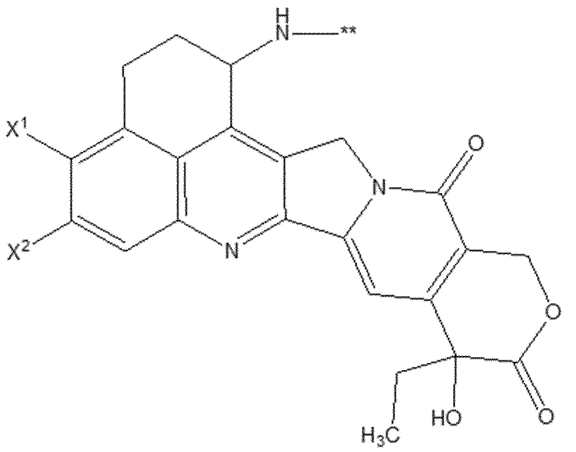

,

wherein $X^1$ and $X^2$ are each independently C1-C6 alkyl, halogen, or -OH; or the C1-C6 alkyl is $-CH_3$; or the halogen is F; ** is a linking point;

or the drug is 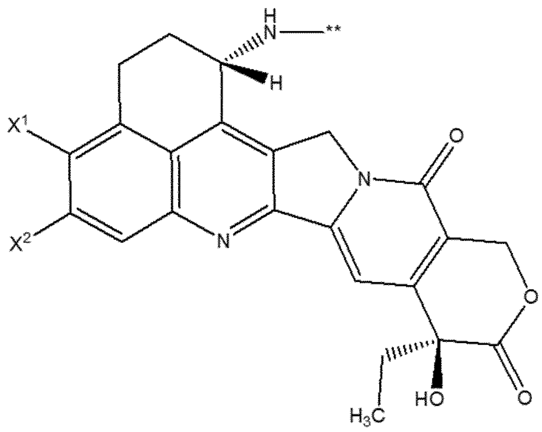 , wherein $X^1$ and $X^2$ are each independently C1-C6 alkyl, halogen, or -OH; or the C1-C6 alkyl is $-CH_3$; or the halogen is F; ** is a linking point; or the drug is

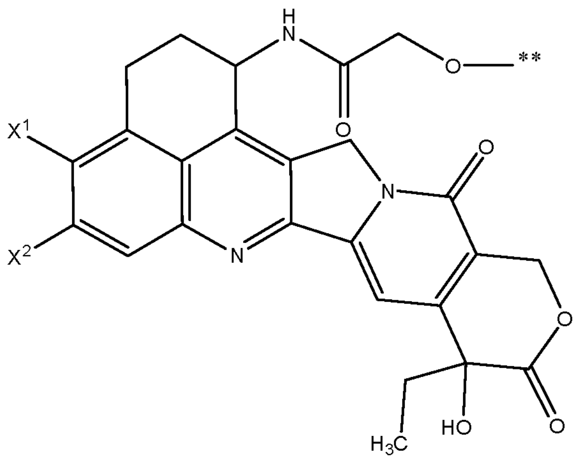

,

wherein $X^1$ and $X^2$ are each independently C1-C6 alkyl, halogen, or -OH; or the C1-C6 alkyl is $-CH_3$; or the halogen is F; ** is a linking point; or the drug is

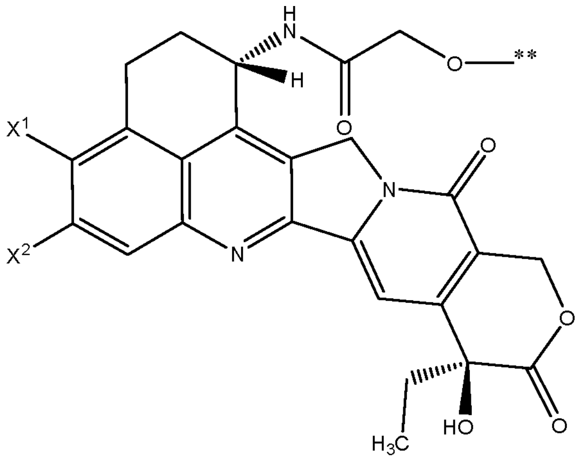

,

wherein $X^1$ and $X^2$ are each independently C1-C6 alkyl, halogen, or -OH; or the C1-C6 alkyl is $-CH_3$; or the halogen is F; ** is a linking point;
or the drug is

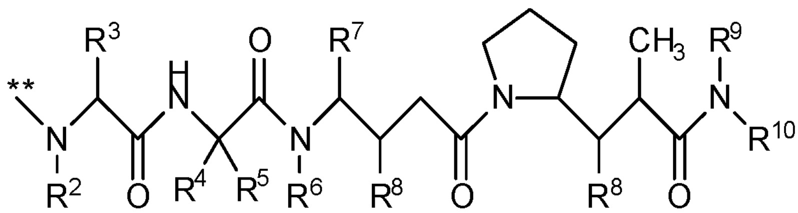

,

wherein ** is a linking point;
$R^2$ is H or C1-C8 alkyl;
$R^3$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or $C_1$-$C_8$ alkyl-(C3-C8 heterocyclyl);
$R^4$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);
$R^5$ is H or methyl;
or $R^4$ and $R^5$ are linked to form a carbocyclyl having a formula of $-(CR^aR^b)_j-$, wherein $R^a$ and $R^b$ are each independently H, C1-C8 alkyl, or C3-C8 carbocyclyl, and j is 2, 3, 4, 5, or 6;
$R^6$ is H or C1-C8 alkyl;
$R^7$ is H, C1-C8 alkyl, C3-C8 carbocyclyl, aryl, C1-C8 alkyl-aryl, C1-C8 alkyl-(C3-C8 carbocyclyl), C3-C8 heterocyclyl, or C1-C8 alkyl-(C3-C8 heterocyclyl);
each $R^8$ is independently H, OH, $C_1$-$C_8$ alkyl, C3-C8 carbocyclyl, or O-(C1-C8 alkyl);
$R^9$ is H or C1-C8 alkyl; and
$R^{10}$ is $-C(R^8)_2-C(R^8)_2$-aryl, $-C(R^8)_2-C(R^8)_2$-(C3-C8 heterocyclyl), or $-C(R^8)_2-C(R^8)_2$-(C3-C8 carbocyclyl);
or the drug is

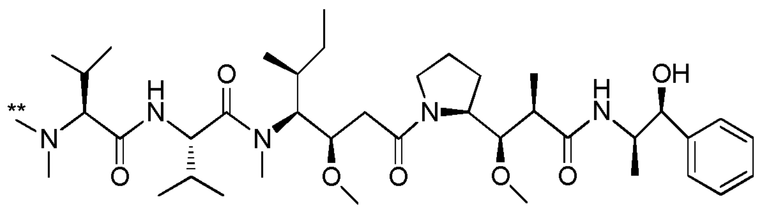

,

wherein ** is a linking point.

**29.** An antibody-drug conjugate having a structure shown in Formula I-A-12, I-A-13, I-A-14, I-A-15, I-A-16, I-A-17, I-A-18, I-A-19, I-A-20, I-A-21, I-A-22, I-A-23, I-A-24, I-A-25, I-B-1 or I-C-1 or a stereoisomer thereof, or a pharmaceutically acceptable salt or solvate thereof, wherein the Formulas I-A-12, I-A-13, I-A-14, I-A-15, I-A-16, I-A-17, I-A-18, I-A-19, I-A-20, I-A-21, I-A-22, I-A-23, I-A-24, I-A-25, I-B-1, and I-C-1 are:

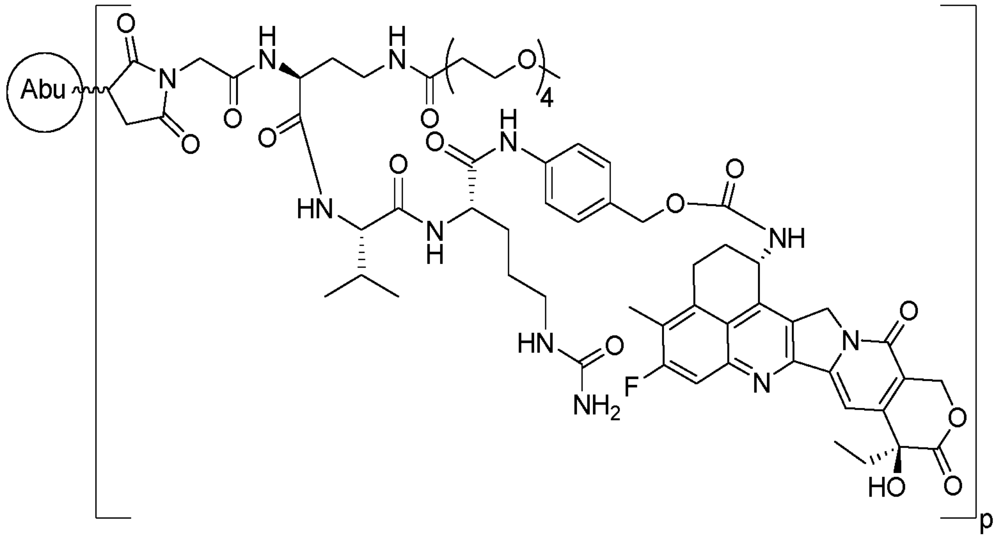

Formula I-A-12

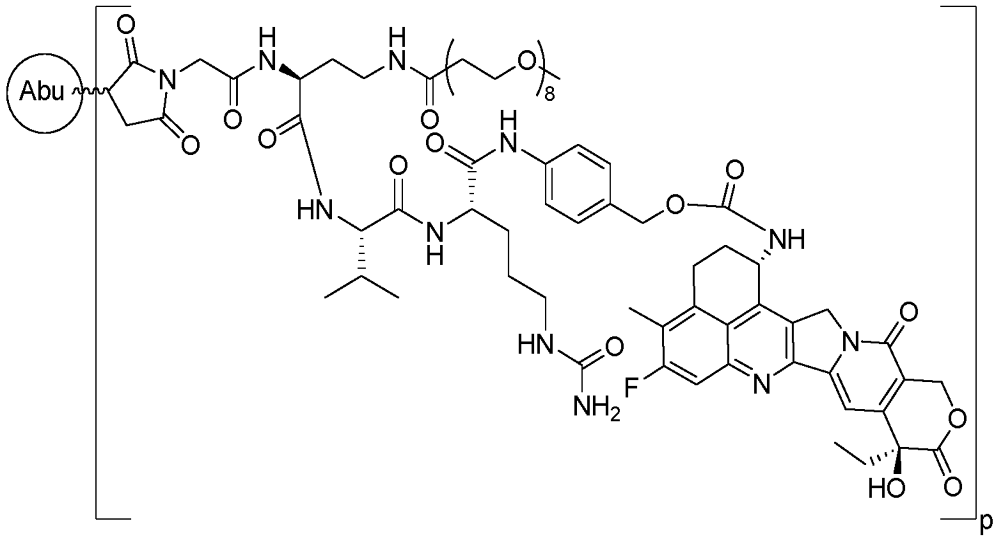

Formula I-A-13

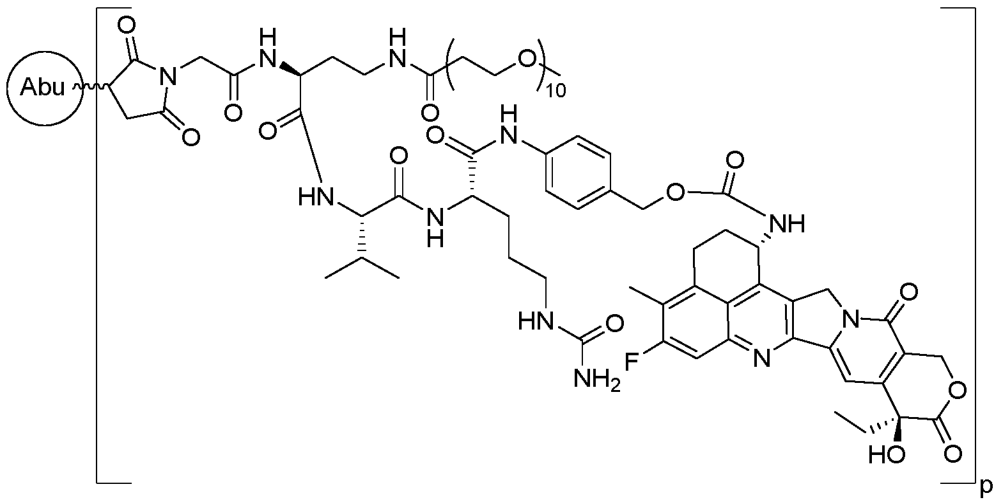

Formula I-A-14

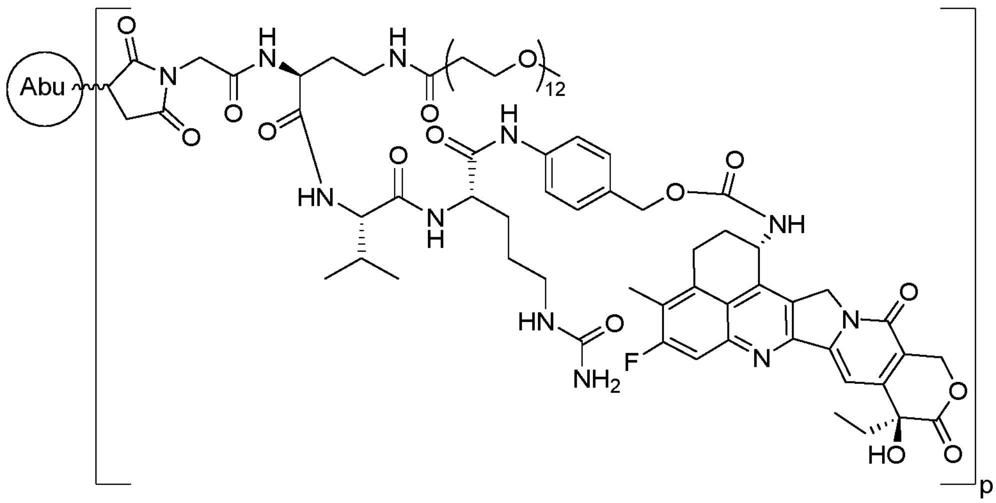

Formula I-A-15

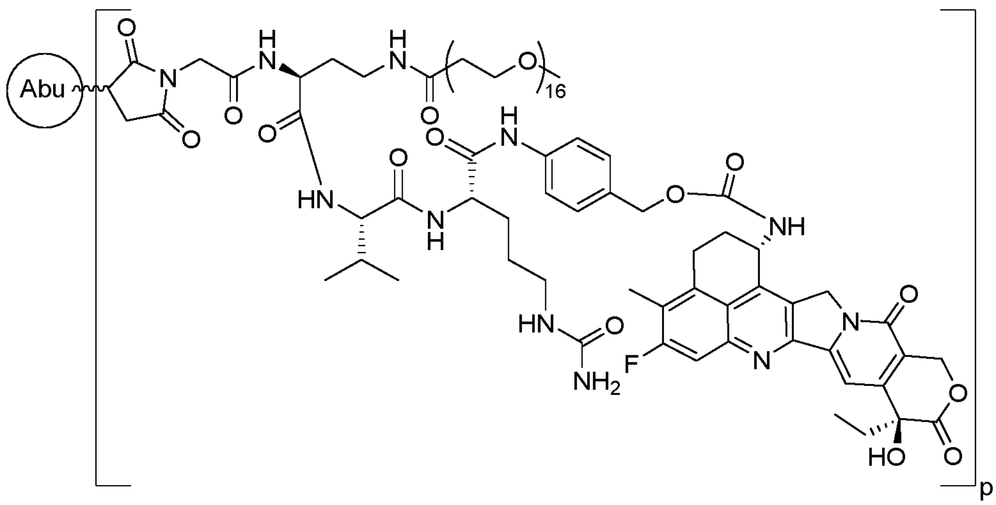

Formula I-A-16

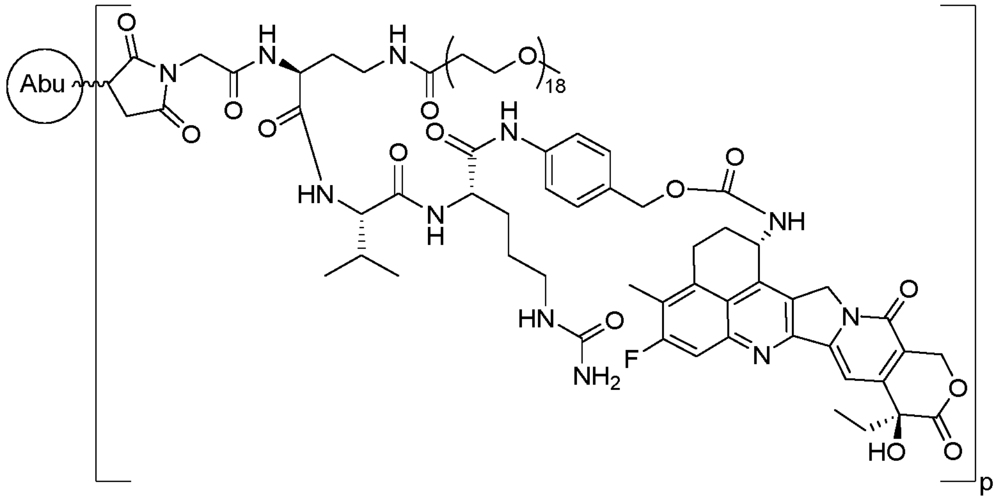

Formula I-A-17

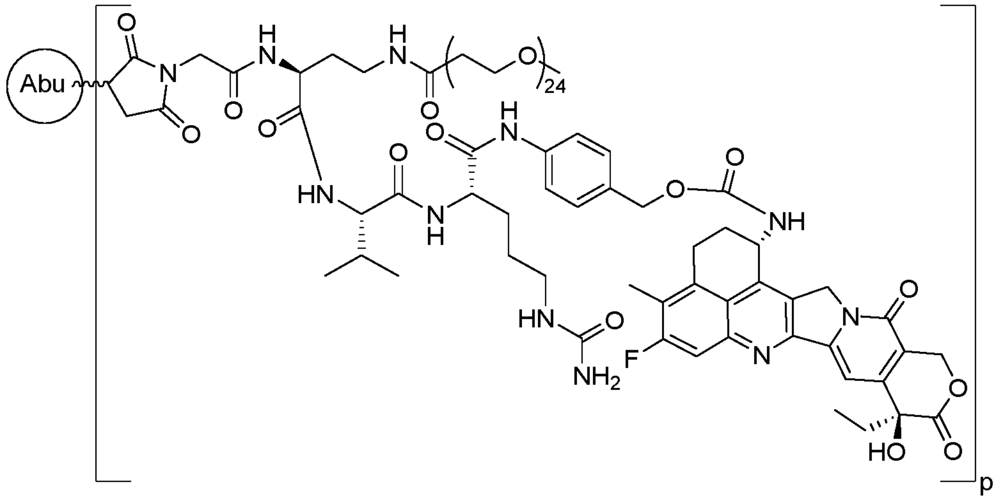

Formula I-A-18

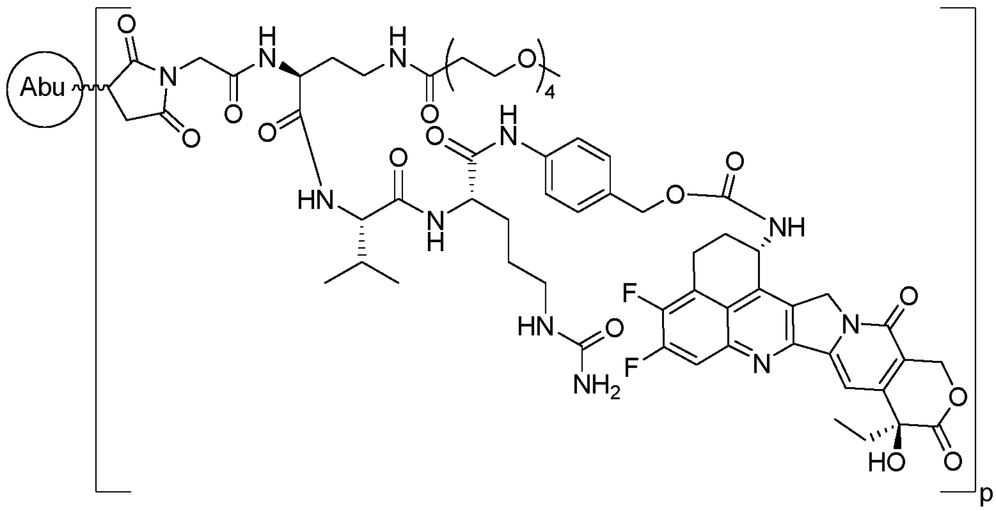

Formula I-A-19

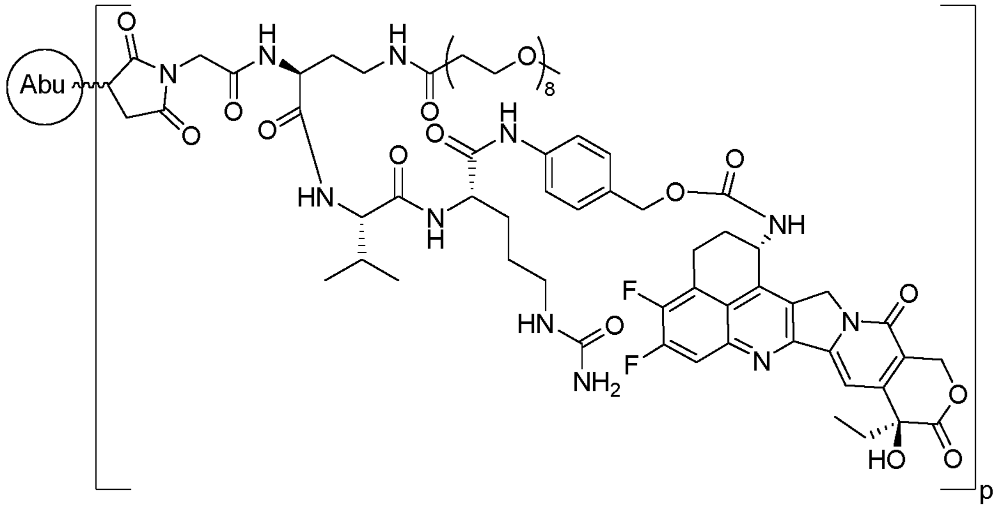

Formula I-A-20

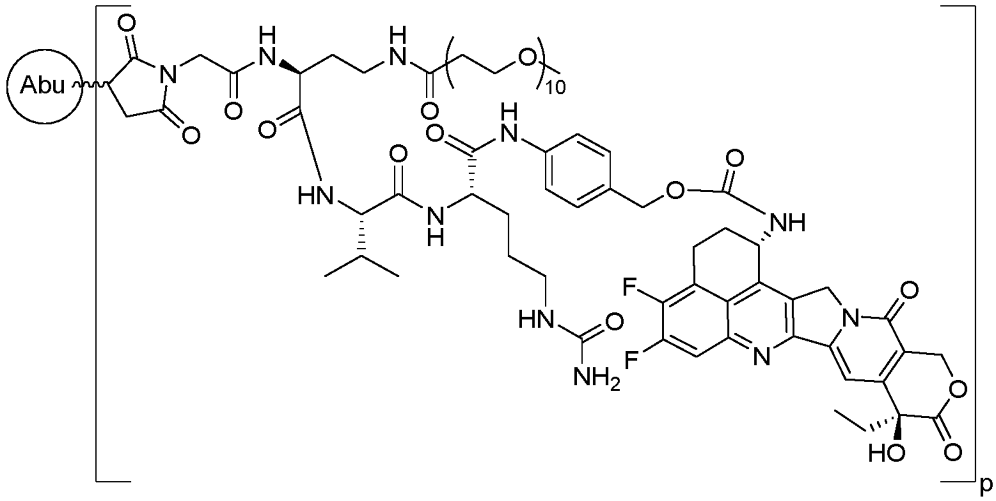

Formula I-A-21

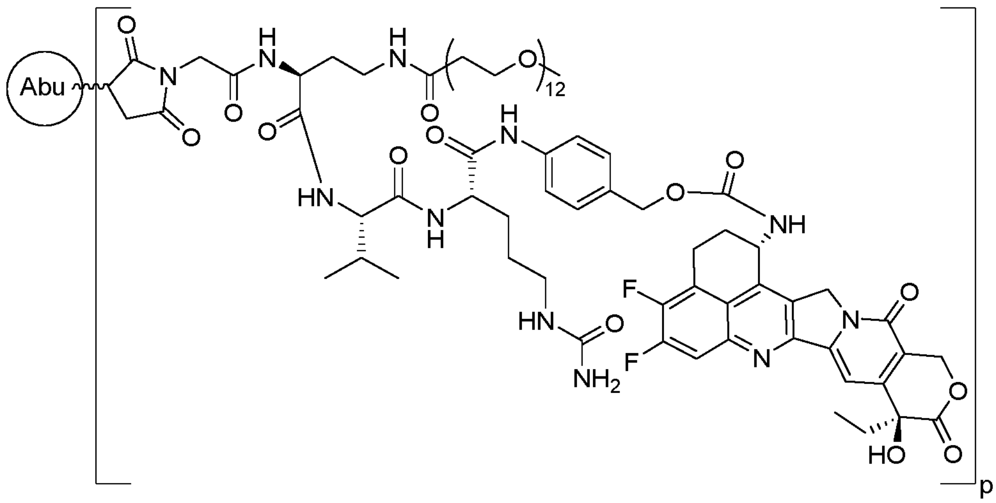

Formula I-A-22

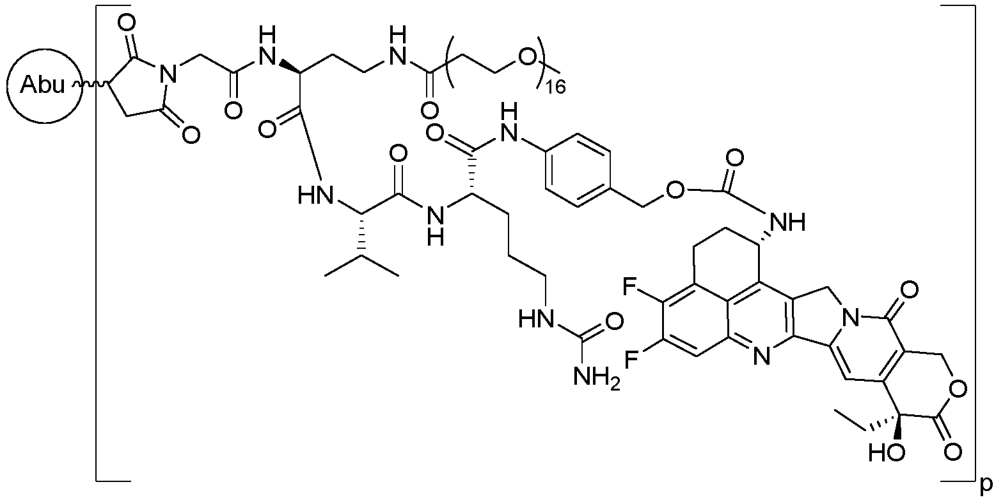

Formula I-A-23

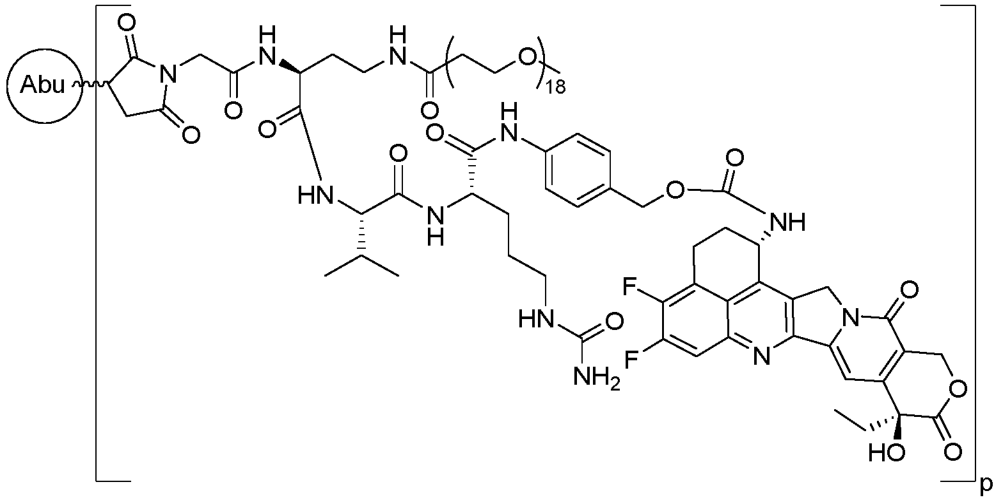

Formula I-A-24

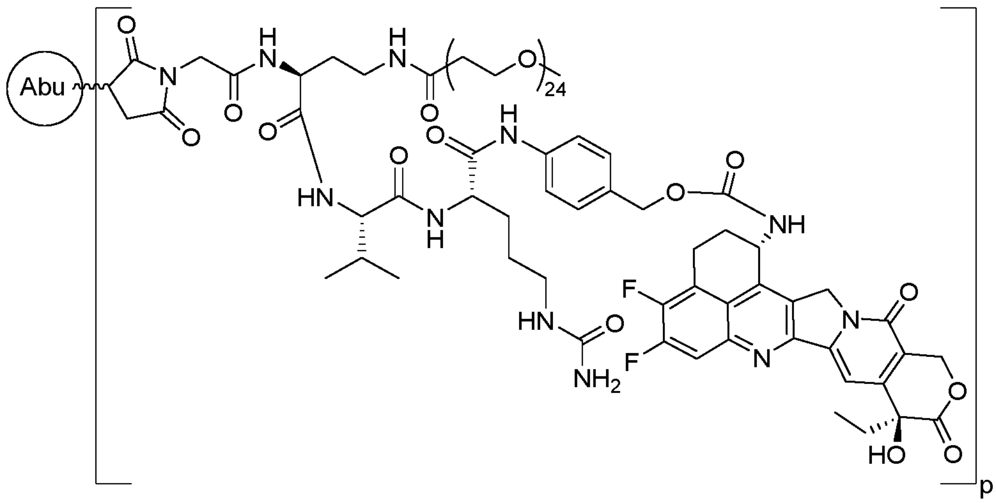

Formula I-A-25

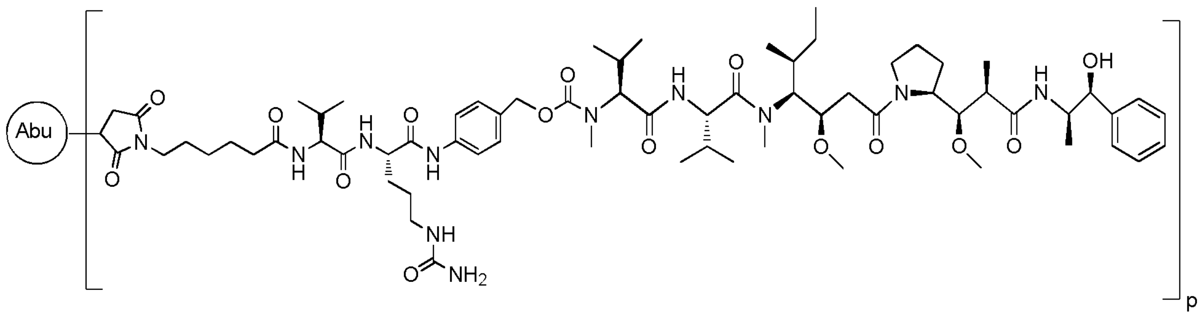

Formula I-B-1

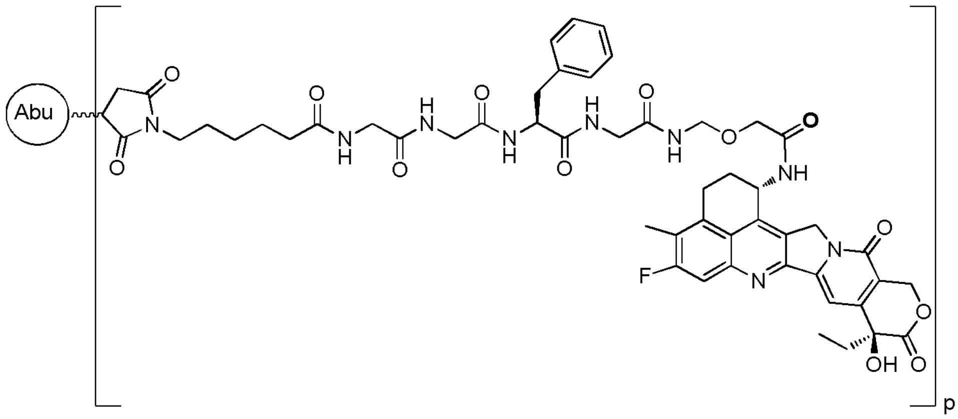

Formula I-C-1

wherein

Abu is the antibody or the antigen-binding unit according to any one of claims 1-9;
p is 1-10.

**30.** The antibody-drug conjugate or the pharmaceutically acceptable salt or solvate thereof according to any one of claims 13-29, wherein p is 2-8; or p is 4-8; or p is 6-8; or p is 7-8.

**31.** A pharmaceutical composition, comprising: the antibody or the antigen-binding unit according to any one of claims 1-9, the biomaterial according to claim 10, or the antibody-drug conjugate or the pharmaceutically acceptable salt or solvate thereof according to any one of claims 12-30; a pharmaceutically acceptable carrier, excipient, and/or adjuvant material; and optionally, an additional anti-cancer drug.

**32.** Use of the antibody or the antigen-binding unit according to any one of claims 1-9, the biomaterial according to claim 10, the antibody-drug conjugate or the pharmaceutically acceptable salt or solvate thereof according to any one of claims 12-30, or the pharmaceutical composition according to claim 31 in treating a disease or in preparing a medicament for treating a disease, wherein alternatively, the use further includes combined use with an additional anti-cancer drug; alternatively, the disease is a disease associated with the abnormal expression of Nectin-4; alternatively, the disease is a tumor expressing or overexpressing Nectin-4; alternatively, the disease is a cancer expressing or overexpressing Nectin-4; alternatively, the disease is a solid tumor or a hematological cancer; alternatively, the disease is selected from breast cancer, pancreatic cancer, bladder cancer, urothelial carcinoma, melanoma, lung cancer, head and neck cancer, cervical cancer, ovarian cancer, choriocarcinoma, skin cancer, esophageal cancer, gastric cancer, uterine cancer, gallbladder cancer, liver cancer, hepatocellular carcinoma, urinary tract cancer, renal pelvic carcinoma, ureteral cancer, colorectal cancer, colon cancer, and prostate cancer.

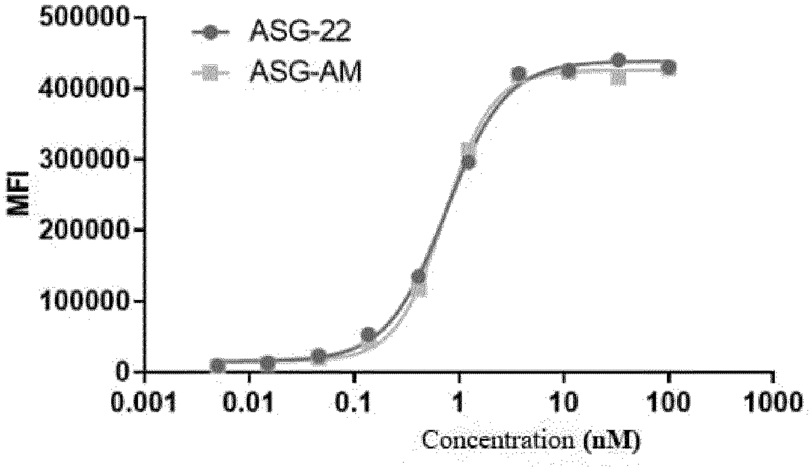


| | ASG-22 | ASG-AM |
|---|---|---|
| EC50 (nM) | 0.7522 | 0.7260 |

FIG. 1

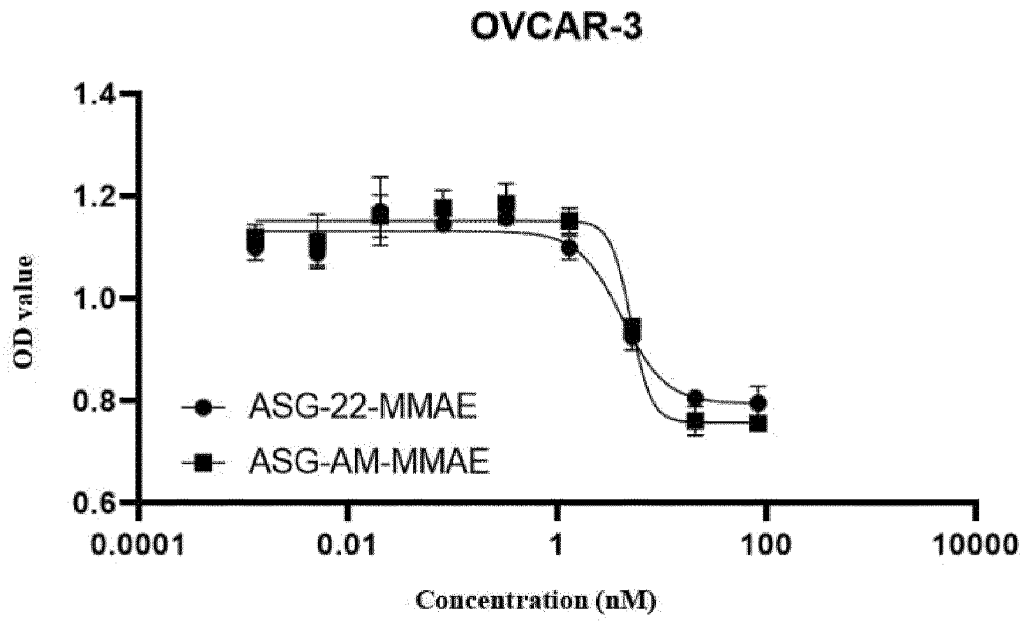


FIG. 2A

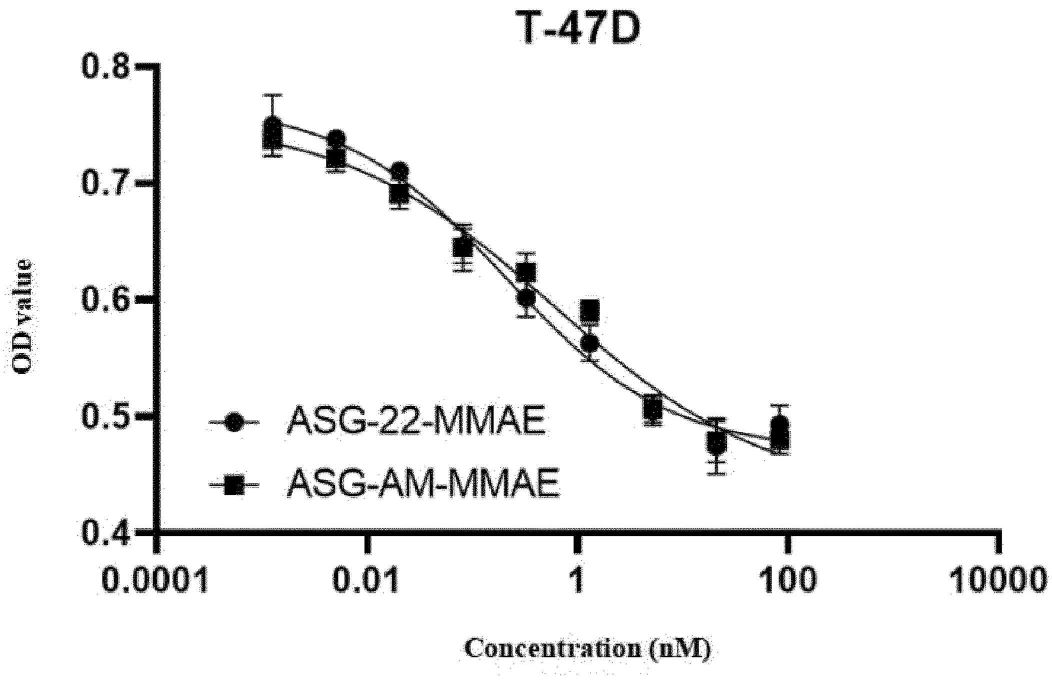
T-47D
0.8
0.7
0.6
0.5
0.4
OD value
ASG-22-MMAE
ASG-AM-MMAE
0.0001
0.01
1
100
10000
Concentration (nM)

FIG. 2B

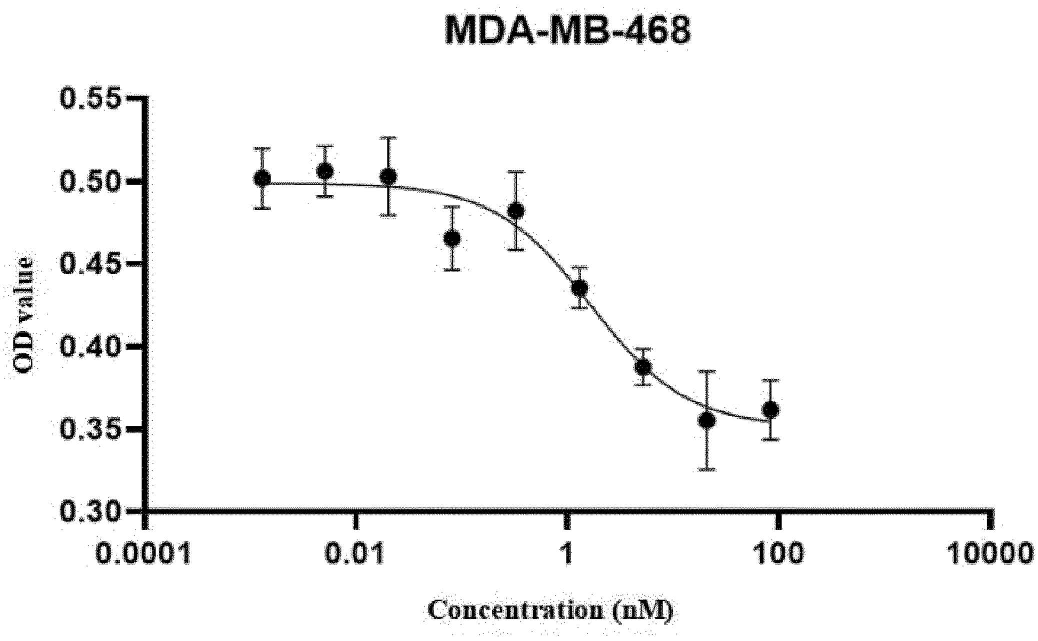
MDA-MB-468
0.55
0.50
0.45
0.40
0.35
0.30
OD value
0.0001
0.01
1
100
10000
Concentration (nM)

FIG. 2C

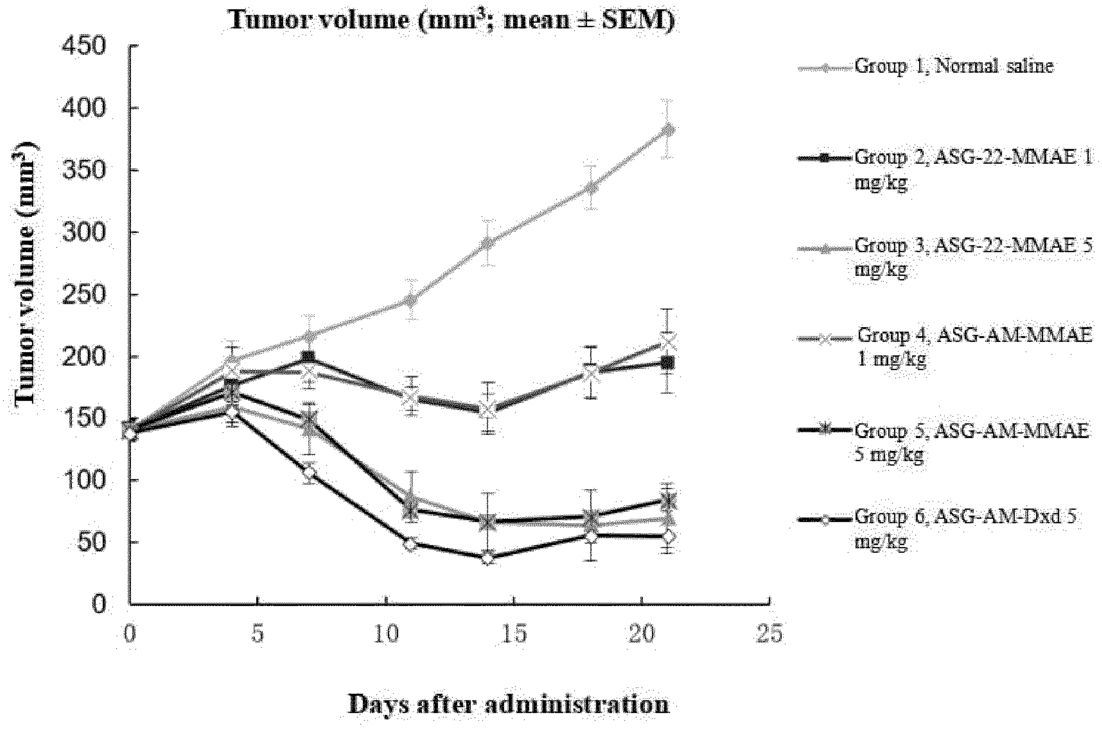
Tumor volume (mm³; mean ± SEM)
Tumor volume (mm³)
Days after administration
Group 1, Normal saline
Group 2, ASG-22-MMAE 1 mg/kg
Group 3, ASG-22-MMAE 5 mg/kg
Group 4, ASG-AM-MMAE 1 mg/kg
Group 5, ASG-AM-MMAE 5 mg/kg
Group 6, ASG-AM-Dxd 5 mg/kg

FIG. 3

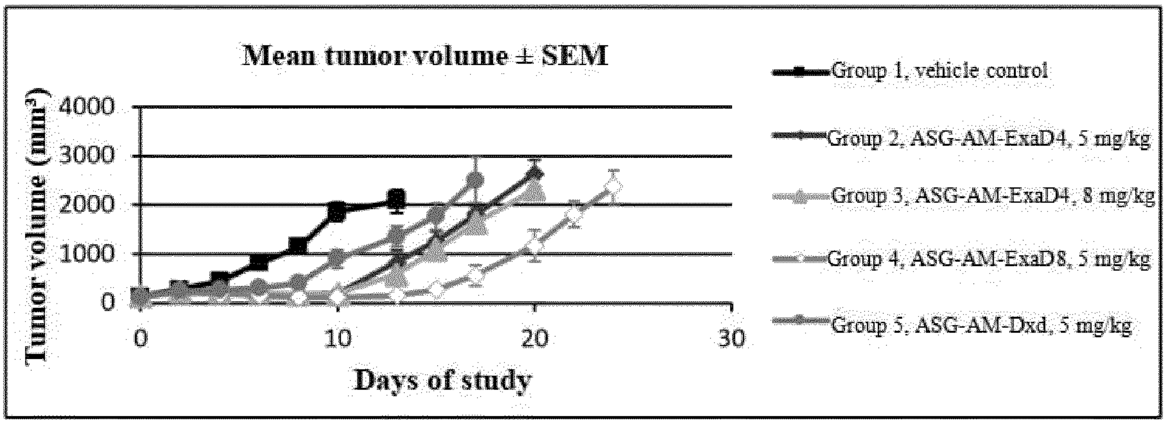
Mean tumor volume ± SEM
Tumor volume (mm³)
Days of study
Group 1, vehicle control
Group 2, ASG-AM-ExaD4, 5 mg/kg
Group 3, ASG-AM-ExaD4, 8 mg/kg
Group 4, ASG-AM-ExaD8, 5 mg/kg
Group 5, ASG-AM-Dxd, 5 mg/kg

FIG. 4

**INTERNATIONAL SEARCH REPORT**

International application No.
**PCT/CN2023/107275**

**A. CLASSIFICATION OF SUBJECT MATTER**

C07K16/28(2006.01)i; C12N15/13(2006.01)i; A61K47/68(2017.01)i; A61K39/395(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07K, A61K, A61P

Documentation searched other than minimum documentation to the extent that such documents are included in the fields searched

Electronic data base consulted during the international search (name of data base and, where practicable, search terms used)

DWPI, VEN, CNABS, CNTXT, CNKI, PubMed, GenBank, EBI, STN, WPABSC, WPABS, ENTXT, ENTXTC, Wanfang Database, ISI web of Knowledge: Nectin-4, PVRL4, PRR4, 191P4D12, antibody, ADC, MMAE, Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, Gly-Phe-Lys, Leu-Ala-Leu, Ile-Ala-Leu, Val-Ala-Val, Ala-Leu-Ala-Leu, Gly-Phe-Leu-Gly, SEQ ID NOs: 1-15, 喜树碱, 药物, 连接子

**C. DOCUMENTS CONSIDERED TO BE RELEVANT**

| Category* | Citation of document, with indication, where appropriate, of the relevant passages | Relevant to claim No. |
|---|---|---|
| X | WO 2021151984 A1 (INNATE PHARMA) 05 August 2021 (2021-08-05) claims 1-36, and description, pages 9-15, 33-35 and 40-71 | 1, 5, 7, 8, 10-32 |
| X | WO 2021108353 A1 (AGENSYS, INC. et al.) 03 June 2021 (2021-06-03) claims 1, 170-195 and 210-219 | 1, 5, 7, 8, 10-32 |
| X | WO 2021030240 A1 (AGENSYS, INC. et al.) 18 February 2021 (2021-02-18) claims 1-56 | 1, 5, 7, 8, 10-32 |
| X | WO 2022076767 A1 (AGENSYS, INC. et al.) 14 April 2022 (2022-04-14) claims 1-43 | 1, 5, 7, 8, 10-32 |
| X | WO 2021168274 A1 (SILVERBACK THERAPEUTICS, INC.) 26 August 2021 (2021-08-26) claims 1-103 | 1, 5, 7, 8, 10-32 |
| X | WO 2021257525 A1 (BIOATLA, INC.) 23 December 2021 (2021-12-23) claims 1-47 | 1, 5, 7, 8, 10-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 August 2023** | **12 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/107275**

**C. DOCUMENTS CONSIDERED TO BE RELEVANT**

| Category* | Citation of document, with indication, where appropriate, of the relevant passages | Relevant to claim No. |
|---|---|---|
| A | HASHIMOTO, H. et al. "Nectin-4: a Novel Therapeutic Target for Skin Cancers." *Curr. Treat. Options in Oncol.,* Vol. vol. 23, 21 March 2022 (2022-03-21), pp. 578-593 | 1-32 |

Form PCT/ISA/210 (second sheet) (July 2022)

**INTERNATIONAL SEARCH REPORT**

International application No.
**PCT/CN2023/107275**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/107275**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **32**
because they relate to subject matter not required to be searched by this Authority, namely:

Claim 32 (in part) relates to the use of the antibody or antigen-binding unit, the biological material, the antibody drug conjugate and a salt and solvate thereof, and the pharmaceutical composition of claims 1-10 and 12-31 in the treatment of diseases, that is, claim 32 (in part) relates to subject matter for which no search is required by the International Searching Authority as defined in PCT Rule 39.1: (4) methods for treatment of the human or animal body by surgery or therapy, as well as diagnostic methods. The international search is made on the basis of the use of the antibody or antigen-binding unit, the biological material, the antibody drug conjugate and a salt and solvate thereof, and the pharmaceutical composition in the preparation of a drug for treating diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/107275**

| Patent document cited in search report | Publication date (day/month/year) | Patent family member(s) | Publication date (day/month/year) |
|---|---|---|---|
| WO 2021151984 A1 | 05 August 2021 | None | |
| WO 2021108353 A1 | 03 June 2021 | JP 2023502517 A | 24 January 2023 |
| | | CA 3162282 A1 | 03 June 2021 |
| | | KR 20220106779 A | 29 July 2022 |
| | | AU 2020391623 A1 | 09 June 2022 |
| | | BR 112022009672 A2 | 16 August 2022 |
| | | US 2023025600 A1 | 26 January 2023 |
| | | TW 202133885 A | 16 September 2021 |
| | | IL 293209 A | 01 July 2022 |
| | | EP 4065173 A1 | 05 October 2022 |
| WO 2021030240 A1 | 18 February 2021 | US 2023001005 A1 | 05 January 2023 |
| | | CA 3150274 A1 | 18 February 2021 |
| | | TW 202120557 A | 01 June 2021 |
| | | AU 2020329761 A1 | 03 March 2022 |
| | | EP 4013795 A1 | 22 June 2022 |
| | | BR 112022002391 A2 | 13 December 2022 |
| | | JP 2022544227 A | 17 October 2022 |
| | | KR 20220054321 A | 02 May 2022 |
| | | IL 290391 A | 01 April 2022 |
| WO 2022076767 A1 | 14 April 2022 | KR 20230106607 A | 13 July 2023 |
| | | IL 302006 A | 01 June 2023 |
| | | TW 202228788 A | 01 August 2022 |
| | | CA 3198359 A1 | 14 April 2022 |
| | | EP 4225379 A1 | 16 August 2023 |
| | | AU 2021356542 A1 | 25 May 2023 |
| WO 2021168274 A1 | 26 August 2021 | EP 4106819 A1 | 28 December 2022 |
| | | TW 202144010 A | 01 December 2021 |
| | | US 2022105196 A1 | 07 April 2022 |
| | | JP 2023514727 A | 07 April 2023 |
| | | US 2021275683 A1 | 09 September 2021 |
| | | US 11179473 B2 | 23 November 2021 |
| WO 2021257525 A1 | 23 December 2021 | TW 202204425 A | 01 February 2022 |
| | | US 2023235054 A1 | 27 July 2023 |
| | | CA 3182395 A1 | 23 December 2021 |
| | | KR 20230038711 A | 21 March 2023 |
| | | AU 2021293183 A1 | 16 February 2023 |
| | | JP 2023531185 A | 21 July 2023 |
| | | IL 298903 A | 01 February 2023 |
| | | EP 4168453 A1 | 26 April 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5892019 A
- US 5168062 A
- US 4510245 A
- US 4968615 A
- US 4399216 A
- US 4634665 A
- US 5179017 A
- US 4816397 A


### Non-patent literature cited in the description


- **KABAT, E. et al.** *U.S. Department of Health and Human Services, Sequences of Proteins of Immunological Interest*, 1983
- **CHOTHIA** ; **LESK**. *J. Mol. Biol.*, 1987, vol. 196, 901-917
- **WINNAKER**. *From Genes to Clones*, 1987
- **KABAT et al.** U.S. Dept. of Health and Human Services. *Sequences of Proteins of Immunological Interest*, 1983
- **CHOTHIA et al.** *J. Mol. Biol.*, 1987, vol. 196, 901-917
- **KABAT et al.** U.S. Dept. of Health and Human Services. *Sequence of Proteins of Immunological Interest*, 1983
- **MALMQVIST, M**. *Nature*, 1993, vol. 361, 186-87
- **DAVIES et al.** *Annual Rev Biochem*, 1990, vol. 59, 439-473
- Immunology-A Synthesis. Sinauer Associates, 1991
- Current Protocols in Molecular Biology. 2007
- Proteins: Structures and Molecular Principles. W. H. Freeman and Company, 1984
- Introduction to Protein Structure. Garland Publishing, 1991
- **THORNTON et al.** *Nature*, 1991, vol. 354, 105
- **OUYANG**. *J. Methods Mol Biol*, 2013, vol. 1045, 275-83
- **WU** ; **WU**. *J. Biol. Chem.*, 1987, vol. 262, 4429-4432
- **CHOTHIA et al.** *J. Mol. Biol.*, 1998, vol. 278, 457-479
- **D. L. HACKER** ; **F. M. WURM**. Recombinant DNA Technology for Production of Protein Therapeutics in Cultured Mammalian Cells. *Reference Module in Life Sciences*, 2017
- **GOEDDEL**. Gene Expression Technology: Methods in Enzymology. Academic Press, 1990, vol. 185
- **TIHOMIR S. DODEV et al.** A tool kit for rapid cloning and expression of recombinant antibodies. *Scientific Reports*, 2014, vol. 4
- Solid Phase Peptide Synthesis. The Pierce Chemical Co., 1984
- **CHU et al.** *Biochemia*, 2001 (2)
- **MURRAY et al.** *Current Opinion in Chemical Biology*, 2013, vol. 17, 420-426
- **D. WILKINSON'S**. The Scientist. Scientist, Inc., 17 April 2000, vol. 14, 25-28
- *CHEMICAL ABSTRACTS*, 143655-49-6